# EUROPEAN PATENT APPLICATION

(11) **EP 4 628 508 A1**
(43) Date of publication of application: **08.10.2025**
(21) Application number: 23900094.6
(22) Date of filing: 08.12.2023
(51) Int. Cl.: C07K 16/28, C12N 1/21, C12N 5/10, C12N 15/13, C12P 21/08, G01N 33/577, A61K 39/395, A61K 47/68, A61P 35/00

(54) **ANTI-ILT4 ANTIBODY AS WELL AS PREPARATION METHOD THEREFOR AND USE THEREOF**

(30) Priority: 09.12.2022 CN 202211584175
(71) Applicant: Biodlink Biopharm Co., Ltd., Suzhou, Jiangsu 215000 (CN)
(72) Inventor: ZENG, Jue, Suzhou, Jiangsu 215000 (CN); HUANG, Jian, Suzhou, Jiangsu 215000 (CN); DUAN, Qing, Suzhou, Jiangsu 215000 (CN); LI, Chenlu, Suzhou, Jiangsu 215000 (CN); ZHANG, Qingqing, Suzhou, Jiangsu 215000 (CN); LIU, Lile, Suzhou, Jiangsu 215000 (CN); FANG, Yunling, Suzhou, Jiangsu 215000 (CN)
(74) Representative: Dilg, Haeusler, Schindelmann Patentanwaltsgesellschaft mbH
(86) International application number: PCT/CN2023/137480
(87) International publication number: WO 2024/120526

(57) **Abstract**

Disclosed in the present invention are an anti-ILT4 antibody as well as a preparation method therefor and the use thereof. The antibody comprises a heavy chain variable region and a light chain variable region, and is specifically described in the specification. The antibody of the present invention has the ability to bind to human ILT4, wherein part of the antibody has the activity of binding to both human ILT2 and human ILT4, and may also bind to crab-eating macaque ILT4. The affinity of the antibody of the present invention binding to the human ILT4 is superior to that of MK-4830 (Merck). The antibody of the present invention promotes the differentiation of macrophages to M1 so as to secrete TNF-α, and the differentiated M1 cells have a better antigen presentation function, thereby improving the killing effect of T cells on tumors. The antibody of the present invention acts on MDSCs and TAMs in tumor microenvironments, and improves the environment of immunosuppression in the tumor microenvironments. The anti-tumor effect of the antibody of the present invention in *in-vivo* pharmacodynamic experiments on mice is obviously superior to that of control antibody MK-4830 (Merck) and that of NGM707 (NGM).

## Description

The present application claims priority to Chinese Patent Application No. 2022115841752 filed on December 9, 2022, which is incorporated herein by reference in its entirety.

### TECHNICAL FIELD

The present disclosure relates to the field of biomedicine and, particularly, to an anti-ILT4 antibody as well as preparation method therefor and use thereof.

### BACKGROUND

Myeloid-derived suppressor cells (MDSCs) are cells that exist in the tumor microenvironment and have a strong immunosuppressive function. They can suppress immune responses mediated by such cells as T cells, natural killer cells (NK cells), dendritic cells (DCs), and macrophages and promote the generation of regulatory T cells (Treg) and tumor-associated macrophages (TAMs). Many studies have reported that both TAM and MDSC can enable tumor cells to evade immune surveillance through their immunosuppressive functions, playing crucial roles in the tumor metastasis process while also reducing patients' responsiveness to tumor immunotherapy.

Immunoglobulin-like transcript (ILT) is also known as the leukocyte immunoglobulin-like receptor (LILR) family or cluster of differentiation 85 (CD85). This receptor family includes inhibitory subtypes (LILRBs) and activating subtypes (LILRAs). The inhibitory receptors include LILRB1/ILT2, LILRB2/ILT4, LILRB3/ILT5, LILRB4/ILT3, and LILRB5/LIR8. The activating receptors include LIRA1/LIR6, LILRA2/ILT1, LILRA3/LIR4, LILRA4/ILT7, LILRA5/ILT11, and LILRA6/ILT8. These receptors are mainly expressed on immune cells and can recognize different ligands, thereby exerting immunosuppressive or immunoactivating effects.

Both ILT2 and ILT4 are inhibitory receptors, with ILT2 being expressed predominantly on monocytes, macrophages, dendritic cells, T cells, and NK cells, and ILT4 being expressed predominantly on the surface of myeloid cells such as monocytes, macrophages, and dendritic cells. Both ILT2 and ILT4 molecules are typical type I transmembrane proteins. The extracellular portion is composed of four Ig-like domains in series, and the intracellular portion contains immunoreceptor tyrosine-based inhibitory motifs (ITIMs), with ILT2 having four ITIM motifs within the cell and ILT4 having three ITIM motifs within the cell. Ligands for ILT2 are mainly human leukocyte antigen class I-G (HLA-G), and ligands for ILT4 are mainly HLA-G, angiopoietin-like protein 2 (ANGPTL2), and semaphorin 4A (SEMA4A). The binding of ILT2/ILT4 to its ligand inhibits signaling by ITIM transduction, inhibiting immune cell activation, antigen presentation, and immune response. Recent studies have shown that ILT4 is also expressed in tumor microenvironment, including tumor cells and tumor mesenchymal cells such as hematopoietic stem cells (HSCs), MDSCs, and TAMs, and it regulates malignant biological behaviors of tumor cells, HSC regeneration, and M2-like polarization of macrophages, inhibits maturation of DCs, reduces antigen presentation ability thereof, and thereby induces dysfunction of effector T cells and differentiation of inhibitory T cells and expands immunosuppressive microenvironment. ILT2 is expressed on the surface of T cells and may also inhibit T cell function. Both ILT2 and ILT4 are therefore very potential targets in the field of tumor immunity.

Currently, there are some antibody drugs targeting ILT2 or ILT4, such as ILT2 blocking antibody BND-22 by Biond Biologics, ILT4 antibody MK-4830 by Merck, and ILT4 antibody JTX-8064 by Jounce. Among these, Merck's ILT4 antibody has progressed the furthest and is currently in phase II clinical trial. The antibody acts on ILT4, and exerts anti-tumor effect by inhibiting MDSCs and TAMs in tumor microenvironment. Moreover, studies have shown that both ILT2 and ILT4 are co-expressed on the surface of MDSCs and TAMs in tumor microenvironment, and ILT2 is expressed on the surface of T cells in tumor microenvironment. The ILT2 or ILT4 molecule on the surface of these cells can exert immunosuppressive functions, and antibodies targeting both ILT2 and ILT4 may exert better anti-tumor effect, such as the NGM707 antibody by NGM.

Therefore, the development of an antibody drug targeting ILT2 or ILT4 or targeting both of them is of great significance in tumor immunotherapy.

### SUMMARY

To solve the technical problems above, the present disclosure provides an anti-ILT4 antibody as well as preparation method therefor and use thereof. The antibodies of the present disclosure have the following properties: 1) the antibodies have an activity of binding to human ILT4, with some of the antibodies having an activity of binding to both human ILT2 and ILT4 and an activity of binding to cynomolgus monkey ILT4; 2) the antibodies of the present disclosure bind to human ILT4 with higher affinity than MK-4830 (Merck); 3) the antibodies of the present disclosure improve the anti-tumor effect through multiple mechanisms of action. Firstly, the antibodies of the present disclosure can promote differentiation of macrophages towards M1 to secrete TNF-α, thus improving the anti-tumor effect; secondly, the differentiated M1 cells exhibit better antigen presenting function, and thus the killing effect of T cells on tumors is improved; thirdly, the antibodies of the present disclosure act on MDSCs and TAMs in the tumor microenvironment to ameliorate the environment for immunosuppression in the tumor microenvironment and promote T cells' killing of tumors; 4) the anti-tumor effect of the antibodies of the present disclosure is significantly better than that of control antibodies MK-4830 (Merck) and NGM707 (NGM) in a mouse *in vivo* efficacy experiment.

In a first aspect, the present disclosure provides an anti-ILT4 antibody comprising a heavy chain variable region and a light chain variable region. The heavy chain variable region comprises HCDR1, HCDR2, and HCDR3, wherein the HCDR1 comprises an amino acid sequence set forth in SEQ ID NO: 1, SEQ ID NO: 12, SEQ ID NO: 18, SEQ ID NO: 23, SEQ ID NO: 32, SEQ ID NO: 36, SEQ ID NO: 44, or SEQ ID NO: 52, the HCDR2 comprises an amino acid sequence set forth in SEQ ID NO: 8, SEQ ID NO: 13, SEQ ID NO: 2, SEQ ID NO: 7, SEQ ID NO: 9, SEQ ID NO: 10, SEQ ID NO: 11, SEQ ID NO: 19, SEQ ID NO: 24, SEQ ID NO: 27, SEQ ID NO: 33, SEQ ID NO: 35, SEQ ID NO: 37, SEQ ID NO: 41, SEQ ID NO: 45, SEQ ID NO: 50, SEQ ID NO: 53, or SEQ ID NO: 57, or the HCDR3 comprises an amino acid sequence set forth in SEQ ID NO: 3, SEQ ID NO: 14, SEQ ID NO: 20, SEQ ID NO: 25, SEQ ID NO: 28, SEQ ID NO: 34, SEQ ID NO: 38, SEQ ID NO: 42, SEQ ID NO: 46, SEQ ID NO: 51, SEQ ID NO: 54, or SEQ ID NO: 58;

the light chain variable region comprises LCDR1, LCDR2, and LCDR3, wherein the LCDR1 comprises an amino acid sequence set forth in SEQ ID NO: 4, SEQ ID NO: 15, SEQ ID NO: 21, SEQ ID NO: 29, SEQ ID NO: 39, or SEQ ID NO: 47, the LCDR2 comprises an amino acid sequence set forth in SEQ ID NO: 5, SEQ ID NO: 16, SEQ ID NO: 30, SEQ ID NO: 48, or SEQ ID NO: 55, and the LCDR3 comprises an amino acid sequence set forth in SEQ ID NO: 6, SEQ ID NO: 17, SEQ ID NO: 22, SEQ ID NO: 26, SEQ ID NO: 31, SEQ ID NO: 40, SEQ ID NO: 43, SEQ ID NO: 49, SEQ ID NO: 56, or SEQ ID NO: 59.

In a certain preferred embodiment, the heavy chain variable region comprises:
HCDR1 having a sequence set forth in SEQ ID NO: 1, HCDR2 having a sequence set forth in SEQ ID NO: 8, and HCDR3 having a sequence set forth in SEQ ID NO: 3;
HCDR1 having a sequence set forth in SEQ ID NO: 12, HCDR2 having a sequence set forth in SEQ ID NO: 13, and HCDR3 having a sequence set forth in SEQ ID NO: 14;
HCDR1 having a sequence set forth in SEQ ID NO: 1, HCDR2 having a sequence set forth in SEQ ID NO: 2, and HCDR3 having a sequence set forth in SEQ ID NO: 3;
HCDR1 having a sequence set forth in SEQ ID NO: 1, HCDR2 having a sequence set forth in SEQ ID NO: 7, and HCDR3 having a sequence set forth in SEQ ID NO: 3;
HCDR1 having a sequence set forth in SEQ ID NO: 1, HCDR2 having a sequence set forth in SEQ ID NO: 9, and HCDR3 having a sequence set forth in SEQ ID NO: 3;
HCDR1 having a sequence set forth in SEQ ID NO: 1, HCDR2 having a sequence set forth in SEQ ID NO: 10, and HCDR3 having a sequence set forth in SEQ ID NO: 3;
HCDR1 having a sequence set forth in SEQ ID NO: 1, HCDR2 having a sequence set forth in SEQ ID NO: 11, and HCDR3 having a sequence set forth in SEQ ID NO: 3;
HCDR1 having a sequence set forth in SEQ ID NO: 18, HCDR2 having a sequence set forth in SEQ ID NO: 19, and HCDR3 having a sequence set forth in SEQ ID NO: 20;
HCDR1 having a sequence set forth in SEQ ID NO: 23, HCDR2 having a sequence set forth in SEQ ID NO: 24, and HCDR3 having a sequence set forth in SEQ ID NO: 25;
HCDR1 having a sequence set forth in SEQ ID NO: 23, HCDR2 having a sequence set forth in SEQ ID NO: 27, and HCDR3 having a sequence set forth in SEQ ID NO: 28;
HCDR1 having a sequence set forth in SEQ ID NO: 32, HCDR2 having a sequence set forth in SEQ ID NO: 33, and HCDR3 having a sequence set forth in SEQ ID NO: 34;
HCDR1 having a sequence set forth in SEQ ID NO: 32, HCDR2 having a sequence set forth in SEQ ID NO: 35, and HCDR3 having a sequence set forth in SEQ ID NO: 34;
HCDR1 having a sequence set forth in SEQ ID NO: 36, HCDR2 having a sequence set forth in SEQ ID NO: 37, and HCDR3 having a sequence set forth in SEQ ID NO: 38;
HCDR1 having a sequence set forth in SEQ ID NO: 23, HCDR2 having a sequence set forth in SEQ ID NO: 41, and HCDR3 having a sequence set forth in SEQ ID NO: 42;
HCDR1 having a sequence set forth in SEQ ID NO: 44, HCDR2 having a sequence set forth in SEQ ID NO: 45, and HCDR3 having a sequence set forth in SEQ ID NO: 46;
HCDR1 having a sequence set forth in SEQ ID NO: 23, HCDR2 having a sequence set forth in SEQ ID NO: 50, and HCDR3 having a sequence set forth in SEQ ID NO: 51;
HCDR1 having a sequence set forth in SEQ ID NO: 52, HCDR2 having a sequence set forth in SEQ ID NO: 53, and HCDR3 having a sequence set forth in SEQ ID NO: 54; or
HCDR1 having a sequence set forth in SEQ ID NO: 32, HCDR2 having a sequence set forth in SEQ ID NO: 57, and HCDR3 having a sequence set forth in SEQ ID NO: 58.

In a certain preferred embodiment, the light chain variable region comprises:
LCDR1 having a sequence set forth in SEQ ID NO: 4, LCDR2 having a sequence set forth in SEQ ID NO: 5, and LCDR3 having a sequence set forth in SEQ ID NO: 6;
LCDR1 having a sequence set forth in SEQ ID NO: 15, LCDR2 having a sequence set forth in SEQ ID NO: 16, and LCDR3 having a sequence set forth in SEQ ID NO: 17;
LCDR1 having a sequence set forth in SEQ ID NO: 21, LCDR2 having a sequence set forth in SEQ ID NO: 5, and LCDR3 having a sequence set forth in SEQ ID NO: 22;
LCDR1 having a sequence set forth in SEQ ID NO: 15, LCDR2 having a sequence set forth in SEQ ID NO: 16, and LCDR3 having a sequence set forth in SEQ ID NO: 26;
LCDR1 having a sequence set forth in SEQ ID NO: 29, LCDR2 having a sequence set forth in SEQ ID NO: 30, and LCDR3 having a sequence set forth in SEQ ID NO: 31;
LCDR1 having a sequence set forth in SEQ ID NO: 39, LCDR2 having a sequence set forth in SEQ ID NO: 30, and LCDR3 having a sequence set forth in SEQ ID NO: 31;
LCDR1 having a sequence set forth in SEQ ID NO: 15, LCDR2 having a sequence set forth in SEQ ID NO: 16, and LCDR3 having a sequence set forth in SEQ ID NO: 40;
LCDR1 having a sequence set forth in SEQ ID NO: 15, LCDR2 having a sequence set forth in SEQ ID NO: 16, and LCDR3 having a sequence set forth in SEQ ID NO: 43;
LCDR1 having a sequence set forth in SEQ ID NO: 47, LCDR2 having a sequence set forth in SEQ ID NO: 48, and LCDR3 having a sequence set forth in SEQ ID NO: 49;
LCDR1 having a sequence set forth in SEQ ID NO: 15, LCDR2 having a sequence set forth in SEQ ID NO: 55, and LCDR3 having a sequence set forth in SEQ ID NO: 56; or
LCDR1 having a sequence set forth in SEQ ID NO: 15, LCDR2 having a sequence set forth in SEQ ID NO: 16, and LCDR3 having a sequence set forth in SEQ ID NO: 59.

In a certain preferred embodiment, the light chain variable region and the heavy chain variable region are selected from any one of the following:
(1) HCDR1 having a sequence set forth in SEQ ID NO: 1, HCDR2 having a sequence set forth in SEQ ID NO: 8, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(2) HCDR1 having a sequence set forth in SEQ ID NO: 12, HCDR2 having a sequence set forth in SEQ ID NO: 13, and HCDR3 having a sequence set forth in SEQ ID NO: 14; and LCDR1 having a sequence set forth in SEQ ID NO: 15, LCDR2 having a sequence set forth in SEQ ID NO: 16, and LCDR3 having a sequence set forth in SEQ ID NO: 17;
(3) HCDR1 having a sequence set forth in SEQ ID NO: 1, HCDR2 having a sequence set forth in SEQ ID NO: 2, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(4) HCDR1 having a sequence set forth in SEQ ID NO: 1, HCDR2 having a sequence set forth in SEQ ID NO: 7, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(5) HCDR1 having a sequence set forth in SEQ ID NO: 1, HCDR2 having a sequence set forth in SEQ ID NO: 9, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(6) HCDR1 having a sequence set forth in SEQ ID NO: 1, HCDR2 having a sequence set forth in SEQ ID NO: 10, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(7) HCDR1 having a sequence set forth in SEQ ID NO: 1, HCDR2 having a sequence set forth in SEQ ID NO: 11, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(8) HCDR1 having a sequence set forth in SEQ ID NO: 18, HCDR2 having a sequence set forth in SEQ ID NO: 19, and HCDR3 having a sequence set forth in SEQ ID NO: 20; and LCDR1 having a sequence set forth in SEQ ID NO: 21, LCDR2 having a sequence set forth in SEQ ID NO: 5, and LCDR3 having a sequence set forth in SEQ ID NO: 22;
(9) HCDR1 having a sequence set forth in SEQ ID NO: 23, HCDR2 having a sequence set forth in SEQ ID NO: 24, and HCDR3 having a sequence set forth in SEQ ID NO: 25; and LCDR1 having a sequence set forth in SEQ ID NO: 15, LCDR2 having a sequence set forth in SEQ ID NO: 16, and LCDR3 having a sequence set forth in SEQ ID NO: 26;
(10) HCDR1 having a sequence set forth in SEQ ID NO: 23, HCDR2 having a sequence set forth in SEQ ID NO: 27, and HCDR3 having a sequence set forth in SEQ ID NO: 28; and LCDR1 having a sequence set forth in SEQ ID NO: 29, LCDR2 having a sequence set forth in SEQ ID NO: 30, and LCDR3 having a sequence set forth in SEQ ID NO: 31;
(11) HCDR1 having a sequence set forth in SEQ ID NO: 23, HCDR2 having a sequence set forth in SEQ ID NO: 27, and HCDR3 having a sequence set forth in SEQ ID NO: 28; and LCDR1 having a sequence set forth in SEQ ID NO: 39, LCDR2 having a sequence set forth in SEQ ID NO: 30, and LCDR3 having a sequence set forth in SEQ ID NO: 31;
(12) HCDR1 having a sequence set forth in SEQ ID NO: 32, HCDR2 having a sequence set forth in SEQ ID NO: 33, and HCDR3 having a sequence set forth in SEQ ID NO: 34; and LCDR1 having a sequence set forth in SEQ ID NO: 15, LCDR2 having a sequence set forth in SEQ ID NO: 16, and LCDR3 having a sequence set forth in SEQ ID NO: 17;
(13) HCDR1 having a sequence set forth in SEQ ID NO: 32, HCDR2 having a sequence set forth in SEQ ID NO: 35, and HCDR3 having a sequence set forth in SEQ ID NO: 34; and LCDR1 having a sequence set forth in SEQ ID NO: 15, LCDR2 having a sequence set forth in SEQ ID NO: 16, and LCDR3 having a sequence set forth in SEQ ID NO: 17;
(14) HCDR1 having a sequence set forth in SEQ ID NO: 36, HCDR2 having a sequence set forth in SEQ ID NO: 37, and HCDR3 having a sequence set forth in SEQ ID NO: 38; and LCDR1 having a sequence set forth in SEQ ID NO: 15, LCDR2 having a sequence set forth in SEQ ID NO: 16, and LCDR3 having a sequence set forth in SEQ ID NO: 40;
(15) HCDR1 having a sequence set forth in SEQ ID NO: 23, HCDR2 having a sequence set forth in SEQ ID NO: 41, and HCDR3 having a sequence set forth in SEQ ID NO: 42; and LCDR1 having a sequence set forth in SEQ ID NO: 15, LCDR2 having a sequence set forth in SEQ ID NO: 16, and LCDR3 having a sequence set forth in SEQ ID NO: 43;
(16) HCDR1 having a sequence set forth in SEQ ID NO: 44, HCDR2 having a sequence set forth in SEQ ID NO: 45, and HCDR3 having a sequence set forth in SEQ ID NO: 46; and LCDR1 having a sequence set forth in SEQ ID NO: 47, LCDR2 having a sequence set forth in SEQ ID NO: 48, and LCDR3 having a sequence set forth in SEQ ID NO: 49;
(17) HCDR1 having a sequence set forth in SEQ ID NO: 23, HCDR2 having a sequence set forth in SEQ ID NO: 50, and HCDR3 having a sequence set forth in SEQ ID NO: 51; and LCDR1 having a sequence set forth in SEQ ID NO: 15, LCDR2 having a sequence set forth in SEQ ID NO: 16, and LCDR3 having a sequence set forth in SEQ ID NO: 17;
(18) HCDR1 having a sequence set forth in SEQ ID NO: 52, HCDR2 having a sequence set forth in SEQ ID NO: 53, and HCDR3 having a sequence set forth in SEQ ID NO: 54; and LCDR1 having a sequence set forth in SEQ ID NO: 15, LCDR2 having a sequence set forth in SEQ ID NO: 55, and LCDR3 having a sequence set forth in SEQ ID NO: 56;
(19) HCDR1 having a sequence set forth in SEQ ID NO: 32, HCDR2 having a sequence set forth in SEQ ID NO: 57, and HCDR3 having a sequence set forth in SEQ ID NO: 58; and LCDR1 having a sequence set forth in SEQ ID NO: 15, LCDR2 having a sequence set forth in SEQ ID NO: 16, and LCDR3 having a sequence set forth in SEQ ID NO: 59.

In a certain preferred embodiment, the framework region of the light chain variable region is a human framework region, and/or the framework region of the heavy chain variable region is a human framework region.

Preferably, the heavy chain variable region comprises an amino acid sequence set forth in SEQ ID NO: 64 or an amino acid sequence having at least 85%, 90%, 91%, 92%, 93%, 94%, 95%, 96%, 97%, 98%, or 99% identity to SEQ ID NO: 64, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 68 or an amino acid sequence having at least 85%, 90%, 91%, 92%, 93%, 94%, 95%, 96%, 97%, 98%, or 99% identity to SEQ ID NO: 68, and the light chain variable region comprises an amino acid sequence set forth in SEQ ID NO: 69 or an amino acid sequence having at least 85%, 90%, 91%, 92%, 93%, 94%, 95%, 96%, 97%, 98%, or 99% identity to SEQ ID NO: 69;
the heavy chain variable region comprises an amino acid sequence set forth in SEQ ID NO: 61 or an amino acid sequence having at least 85%, 90%, 91%, 92%, 93%, 94%, 95%, 96%, 97%, 98%, or 99% identity to SEQ ID NO: 61, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 63 or an amino acid sequence having at least 85%, 90%, 91%, 92%, 93%, 94%, 95%, 96%, 97%, 98%, or 99% identity to SEQ ID NO: 63, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 65 or an amino acid sequence having at least 85%, 90%, 91%, 92%, 93%, 94%, 95%, 96%, 97%, 98%, or 99% identity to SEQ ID NO: 65, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 66 or an amino acid sequence having at least 85%, 90%, 91%, 92%, 93%, 94%, 95%, 96%, 97%, 98%, or 99% identity to SEQ ID NO: 66, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 67 or an amino acid sequence having at least 85%, 90%, 91%, 92%, 93%, 94%, 95%, 96%, 97%, 98%, or 99% identity to SEQ ID NO: 67, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 68 or an amino acid sequence having at least 85%, 90%, 91%, 92%, 93%, 94%, 95%, 96%, 97%, 98%, or 99% identity to SEQ ID NO: 68, and the light chain variable region comprises an amino acid sequence set forth in SEQ ID NO: 69 or an amino acid sequence having at least 85%, 90%, 91%, 92%, 93%, 94%, 95%, 96%, 97%, 98%, or 99% identity to SEQ ID NO: 69;
the heavy chain variable region comprises an amino acid sequence set forth in SEQ ID NO: 70 or an amino acid sequence having at least 85%, 90%, 91%, 92%, 93%, 94%, 95%, 96%, 97%, 98%, or 99% identity to SEQ ID NO: 70, and the light chain variable region comprises an amino acid sequence set forth in SEQ ID NO: 71 or an amino acid sequence having at least 85%, 90%, 91%, 92%, 93%, 94%, 95%, 96%, 97%, 98%, or 99% identity to SEQ ID NO: 71;
the heavy chain variable region comprises an amino acid sequence set forth in SEQ ID NO: 72 or an amino acid sequence having at least 85%, 90%, 91%, 92%, 93%, 94%, 95%, 96%, 97%, 98%, or 99% identity to SEQ ID NO: 72, and the light chain variable region comprises an amino acid sequence set forth in SEQ ID NO: 73 or an amino acid sequence having at least 85%, 90%, 91%, 92%, 93%, 94%, 95%, 96%, 97%, 98%, or 99% identity to SEQ ID NO: 73;
the heavy chain variable region comprises an amino acid sequence set forth in SEQ ID NO: 74 or an amino acid sequence having at least 85%, 90%, 91%, 92%, 93%, 94%, 95%, 96%, 97%, 98%, or 99% identity to SEQ ID NO: 74, and the light chain variable region comprises an amino acid sequence set forth in SEQ ID NO: 75 or an amino acid sequence having at least 85%, 90%, 91%, 92%, 93%, 94%, 95%, 96%, 97%, 98%, or 99% identity to SEQ ID NO: 75;
the heavy chain variable region comprises an amino acid sequence set forth in SEQ ID NO: 74 or an amino acid sequence having at least 85%, 90%, 91%, 92%, 93%, 94%, 95%, 96%, 97%, 98%, or 99% identity to SEQ ID NO: 74, and the light chain variable region comprises an amino acid sequence set forth in SEQ ID NO: 76 or an amino acid sequence having at least 85%, 90%, 91%, 92%, 93%, 94%, 95%, 96%, 97%, 98%, or 99% identity to SEQ ID NO: 76;
the heavy chain variable region comprises an amino acid sequence set forth in SEQ ID NO: 77 or an amino acid sequence having at least 85%, 90%, 91%, 92%, 93%, 94%, 95%, 96%, 97%, 98%, or 99% identity to SEQ ID NO: 77, and the light chain variable region comprises an amino acid sequence set forth in SEQ ID NO: 78 or an amino acid sequence having at least 85%, 90%, 91%, 92%, 93%, 94%, 95%, 96%, 97%, 98%, or 99% identity to SEQ ID NO: 78;
the heavy chain variable region comprises an amino acid sequence set forth in SEQ ID NO: 79 or an amino acid sequence having at least 85%, 90%, 91%, 92%, 93%, 94%, 95%, 96%, 97%, 98%, or 99% identity to SEQ ID NO: 79, and the light chain variable region comprises an amino acid sequence set forth in SEQ ID NO: 78 or an amino acid sequence having at least 85%, 90%, 91%, 92%, 93%, 94%, 95%, 96%, 97%, 98%, or 99% identity to SEQ ID NO: 78;
the heavy chain variable region comprises an amino acid sequence set forth in SEQ ID NO: 80 or an amino acid sequence having at least 85%, 90%, 91%, 92%, 93%, 94%, 95%, 96%, 97%, 98%, or 99% identity to SEQ ID NO: 80, and the light chain variable region comprises an amino acid sequence set forth in SEQ ID NO: 81 or an amino acid sequence having at least 85%, 90%, 91%, 92%, 93%, 94%, 95%, 96%, 97%, 98%, or 99% identity to SEQ ID NO: 81;
the heavy chain variable region comprises an amino acid sequence set forth in SEQ ID NO: 82 or an amino acid sequence having at least 85%, 90%, 91%, 92%, 93%, 94%, 95%, 96%, 97%, 98%, or 99% identity to SEQ ID NO: 82, and the light chain variable region comprises an amino acid sequence set forth in SEQ ID NO: 83 or an amino acid sequence having at least 85%, 90%, 91%, 92%, 93%, 94%, 95%, 96%, 97%, 98%, or 99% identity to SEQ ID NO: 83;
the heavy chain variable region comprises an amino acid sequence set forth in SEQ ID NO: 84 or an amino acid sequence having at least 85%, 90%, 91%, 92%, 93%, 94%, 95%, 96%, 97%, 98%, or 99% identity to SEQ ID NO: 84, and the light chain variable region comprises an amino acid sequence set forth in SEQ ID NO: 85 or an amino acid sequence having at least 85%, 90%, 91%, 92%, 93%, 94%, 95%, 96%, 97%, 98%, or 99% identity to SEQ ID NO: 85;
the heavy chain variable region comprises an amino acid sequence set forth in SEQ ID NO: 86 or an amino acid sequence having at least 85%, 90%, 91%, 92%, 93%, 94%, 95%, 96%, 97%, 98%, or 99% identity to SEQ ID NO: 86, and the light chain variable region comprises an amino acid sequence set forth in SEQ ID NO: 78 or an amino acid sequence having at least 85%, 90%, 91%, 92%, 93%, 94%, 95%, 96%, 97%, 98%, or 99% identity to SEQ ID NO: 78;
the heavy chain variable region comprises an amino acid sequence set forth in SEQ ID NO: 87 or an amino acid sequence having at least 85%, 90%, 91%, 92%, 93%, 94%, 95%, 96%, 97%, 98%, or 99% identity to SEQ ID NO: 87, and the light chain variable region comprises an amino acid sequence set forth in SEQ ID NO: 88 or an amino acid sequence having at least 85%, 90%, 91%, 92%, 93%, 94%, 95%, 96%, 97%, 98%, or 99% identity to SEQ ID NO: 88; or
the heavy chain variable region comprises an amino acid sequence set forth in SEQ ID NO: 60 or an amino acid sequence having at least 85%, 90%, 91%, 92%, 93%, 94%, 95%, 96%, 97%, 98%, or 99% identity to SEQ ID NO: 60, and the light chain variable region comprises an amino acid sequence set forth in SEQ ID NO: 89 or an amino acid sequence having at least 85%, 90%, 91%, 92%, 93%, 94%, 95%, 96%, 97%, 98%, or 99% identity to SEQ ID NO: 89.

In a certain preferred embodiment, the antibody is a full-length antibody, a Fab, a Fab', a F(ab')₂, an Fv, an scFv, a di-scFv, a VHH, or a heavy-chain antibody (HCAb).

In a certain preferred embodiment, the antibody is a monoclonal antibody or a polyclonal antibody prepared based on the antibody described above.

In a certain preferred embodiment, the antibody comprises a heavy chain constant region and/or a light chain constant region, wherein the heavy chain constant region of the antibody is derived from the heavy chain constant region of a human antibody IgG1 or IgG4, and/or the light chain constant region of the antibody is derived from the κ chain of a human antibody.

In a certain preferred embodiment, the heavy chain of the antibody comprises an amino acid sequence set forth in SEQ ID NO: 93 or an amino acid sequence having at least 85%, 90%, 91%, 92%, 93%, 94%, 95%, 96%, 97%, 98%, or 99% identity to SEQ ID NO: 93, and the light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91.

In a certain preferred embodiment, the heavy chain of the antibody comprises an amino acid sequence set forth in SEQ ID NO: 99 or an amino acid sequence having at least 85%, 90%, 91%, 92%, 93%, 94%, 95%, 96%, 97%, 98%, or 99% identity to SEQ ID NO: 99, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 90 or an amino acid sequence having at least 85%, 90%, 91%, 92%, 93%, 94%, 95%, 96%, 97%, 98%, or 99% identity to SEQ ID NO: 90, and the light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91.

In a certain preferred embodiment, the heavy chain of the antibody comprises an amino acid sequence set forth in SEQ ID NO: 92 or an amino acid sequence having at least 85%, 90%, 91%, 92%, 93%, 94%, 95%, 96%, 97%, 98%, or 99% identity to SEQ ID NO: 92, and the light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91.

In a certain preferred embodiment, the heavy chain of the antibody comprises an amino acid sequence set forth in SEQ ID NO: 94 or an amino acid sequence having at least 85%, 90%, 91%, 92%, 93%, 94%, 95%, 96%, 97%, 98%, or 99% identity to SEQ ID NO: 94, and the light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91.

In a certain preferred embodiment, the heavy chain of the antibody comprises an amino acid sequence set forth in SEQ ID NO: 95 or an amino acid sequence having at least 85%, 90%, 91%, 92%, 93%, 94%, 95%, 96%, 97%, 98%, or 99% identity to SEQ ID NO: 95, and the light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91.

In a certain preferred embodiment, the heavy chain of the antibody comprises an amino acid sequence set forth in SEQ ID NO: 96 or an amino acid sequence having at least 85%, 90%, 91%, 92%, 93%, 94%, 95%, 96%, 97%, 98%, or 99% identity to SEQ ID NO: 96, and the light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91.

In a certain preferred embodiment, the heavy chain of the antibody comprises an amino acid sequence set forth in SEQ ID NO: 97 or an amino acid sequence having at least 85%, 90%, 91%, 92%, 93%, 94%, 95%, 96%, 97%, 98%, or 99% identity to SEQ ID NO: 97, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 100 or an amino acid sequence having at least 85%, 90%, 91%, 92%, 93%, 94%, 95%, 96%, 97%, 98%, or 99% identity to SEQ ID NO: 100, and the light chain of the antibody comprises an amino acid sequence set forth in SEQ ID NO: 101 or an amino acid sequence having at least 85%, 90%, 91%, 92%, 93%, 94%, 95%, 96%, 97%, 98%, or 99% identity to SEQ ID NO: 101.

In a certain preferred embodiment, the heavy chain of the antibody comprises an amino acid sequence set forth in SEQ ID NO: 102 or an amino acid sequence having at least 85%, 90%, 91%, 92%, 93%, 94%, 95%, 96%, 97%, 98%, or 99% identity to SEQ ID NO: 102, and the light chain of the antibody comprises an amino acid sequence set forth in SEQ ID NO: 101 or an amino acid sequence having at least 85%, 90%, 91%, 92%, 93%, 94%, 95%, 96%, 97%, 98%, or 99% identity to SEQ ID NO: 101.

In a certain preferred embodiment, the heavy chain of the antibody comprises an amino acid sequence set forth in SEQ ID NO: 103 or an amino acid sequence having at least 85%, 90%, 91%, 92%, 93%, 94%, 95%, 96%, 97%, 98%, or 99% identity to SEQ ID NO: 103, and the light chain of the antibody comprises an amino acid sequence set forth in SEQ ID NO: 104 or an amino acid sequence having at least 85%, 90%, 91%, 92%, 93%, 94%, 95%, 96%, 97%, 98%, or 99% identity to SEQ ID NO: 104.

In a certain preferred embodiment, the heavy chain of the antibody comprises an amino acid sequence set forth in SEQ ID NO: 105 or an amino acid sequence having at least 85%, 90%, 91%, 92%, 93%, 94%, 95%, 96%, 97%, 98%, or 99% identity to SEQ ID NO: 105, and the light chain of the antibody comprises an amino acid sequence set forth in SEQ ID NO: 104 or an amino acid sequence having at least 85%, 90%, 91%, 92%, 93%, 94%, 95%, 96%, 97%, 98%, or 99% identity to SEQ ID NO: 104.

In a certain preferred embodiment, the heavy chain of the antibody comprises an amino acid sequence set forth in SEQ ID NO: 106 or an amino acid sequence having at least 85%, 90%, 91%, 92%, 93%, 94%, 95%, 96%, 97%, 98%, or 99% identity to SEQ ID NO: 106, and the light chain of the antibody comprises an amino acid sequence set forth in SEQ ID NO: 107 or an amino acid sequence having at least 85%, 90%, 91%, 92%, 93%, 94%, 95%, 96%, 97%, 98%, or 99% identity to SEQ ID NO: 107.

In a certain preferred embodiment, the heavy chain of the antibody comprises an amino acid sequence set forth in SEQ ID NO: 108 or an amino acid sequence having at least 85%, 90%, 91%, 92%, 93%, 94%, 95%, 96%, 97%, 98%, or 99% identity to SEQ ID NO: 108, and the light chain of the antibody comprises an amino acid sequence set forth in SEQ ID NO: 109 or an amino acid sequence having at least 85%, 90%, 91%, 92%, 93%, 94%, 95%, 96%, 97%, 98%, or 99% identity to SEQ ID NO: 109.

In a certain preferred embodiment, the heavy chain of the antibody comprises an amino acid sequence set forth in SEQ ID NO: 110 or an amino acid sequence having at least 85%, 90%, 91%, 92%, 93%, 94%, 95%, 96%, 97%, 98%, or 99% identity to SEQ ID NO: 110, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 111 or an amino acid sequence having at least 85%, 90%, 91%, 92%, 93%, 94%, 95%, 96%, 97%, 98%, or 99% identity to SEQ ID NO: 111, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 112 or an amino acid sequence having at least 85%, 90%, 91%, 92%, 93%, 94%, 95%, 96%, 97%, 98%, or 99% identity to SEQ ID NO: 112, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 113 or an amino acid sequence having at least 85%, 90%, 91%, 92%, 93%, 94%, 95%, 96%, 97%, 98%, or 99% identity to SEQ ID NO: 113, and the light chain of the antibody comprises an amino acid sequence set forth in SEQ ID NO: 114 or an amino acid sequence having at least 85%, 90%, 91%, 92%, 93%, 94%, 95%, 96%, 97%, 98%, or 99% identity to SEQ ID NO: 114.

In a certain preferred embodiment, the heavy chain of the antibody comprises an amino acid sequence set forth in SEQ ID NO: 115 or an amino acid sequence having at least 85%, 90%, 91%, 92%, 93%, 94%, 95%, 96%, 97%, 98%, or 99% identity to SEQ ID NO: 115, and the light chain of the antibody comprises an amino acid sequence set forth in SEQ ID NO: 116 or an amino acid sequence having at least 85%, 90%, 91%, 92%, 93%, 94%, 95%, 96%, 97%, 98%, or 99% identity to SEQ ID NO: 116.

In a certain preferred embodiment, the heavy chain of the antibody comprises an amino acid sequence set forth in SEQ ID NO: 117 or an amino acid sequence having at least 85%, 90%, 91%, 92%, 93%, 94%, 95%, 96%, 97%, 98%, or 99% identity to SEQ ID NO: 117, and the light chain of the antibody comprises an amino acid sequence set forth in SEQ ID NO: 118 or an amino acid sequence having at least 85%, 90%, 91%, 92%, 93%, 94%, 95%, 96%, 97%, 98%, or 99% identity to SEQ ID NO: 118.

In a certain preferred embodiment, the heavy chain of the antibody comprises an amino acid sequence set forth in SEQ ID NO: 119 or an amino acid sequence having at least 85%, 90%, 91%, 92%, 93%, 94%, 95%, 96%, 97%, 98%, or 99% identity to SEQ ID NO: 119, and the light chain of the antibody comprises an amino acid sequence set forth in SEQ ID NO: 118 or an amino acid sequence having at least 85%, 90%, 91%, 92%, 93%, 94%, 95%, 96%, 97%, 98%, or 99% identity to SEQ ID NO: 118.

In a certain preferred embodiment, the heavy chain of the antibody comprises an amino acid sequence set forth in SEQ ID NO: 120 or an amino acid sequence having at least 85%, 90%, 91%, 92%, 93%, 94%, 95%, 96%, 97%, 98%, or 99% identity to SEQ ID NO: 120, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 121 or an amino acid sequence having at least 85%, 90%, 91%, 92%, 93%, 94%, 95%, 96%, 97%, 98%, or 99% identity to SEQ ID NO: 121, and the light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98.

In a certain preferred embodiment, the heavy chain of the antibody comprises an amino acid sequence set forth in SEQ ID NO: 122 or an amino acid sequence having at least 85%, 90%, 91%, 92%, 93%, 94%, 95%, 96%, 97%, 98%, or 99% identity to SEQ ID NO: 122, and the light chain of the antibody comprises an amino acid sequence set forth in SEQ ID NO: 123 or an amino acid sequence having at least 85%, 90%, 91%, 92%, 93%, 94%, 95%, 96%, 97%, 98%, or 99% identity to SEQ ID NO: 123.

In a certain preferred embodiment, the heavy chain of the antibody comprises an amino acid sequence set forth in SEQ ID NO: 124 or an amino acid sequence having at least 85%, 90%, 91%, 92%, 93%, 94%, 95%, 96%, 97%, 98%, or 99% identity to SEQ ID NO: 124, and the light chain of the antibody comprises an amino acid sequence set forth in SEQ ID NO: 125 or an amino acid sequence having at least 85%, 90%, 91%, 92%, 93%, 94%, 95%, 96%, 97%, 98%, or 99% identity to SEQ ID NO: 125.

In a certain preferred embodiment, the heavy chain of the antibody comprises an amino acid sequence set forth in SEQ ID NO: 126 or an amino acid sequence having at least 85%, 90%, 91%, 92%, 93%, 94%, 95%, 96%, 97%, 98%, or 99% identity to SEQ ID NO: 126, and the light chain of the antibody comprises an amino acid sequence set forth in SEQ ID NO: 125 or an amino acid sequence having at least 85%, 90%, 91%, 92%, 93%, 94%, 95%, 96%, 97%, 98%, or 99% identity to SEQ ID NO: 125.

In a second aspect, the present disclosure provides an isolated nucleic acid encoding the antibody described in the first aspect of the present disclosure.

In a third aspect, the present disclosure provides a recombinant expression vector, which comprises the isolated nucleic acid described in the second aspect of the present disclosure.

Preferably, the backbone of the recombinant expression vector is a plasmid, a cosmid, a phage, or a viral vector, wherein the viral vector is preferably a retroviral vector, a lentiviral vector, an adenoviral vector, or an adeno-associated viral vector.

In a fourth aspect, the present disclosure provides a transformant, which comprises the recombinant expression vector described in the third aspect of the present disclosure.

Preferably, the host cell of the transformant is a prokaryotic cell or a eukaryotic cell.

More preferably, the eukaryotic cell is a yeast cell or a mammalian cell, such as an HEK293 cell or a CHO cell.

In a fifth aspect, the present disclosure provides a method for preparing an antibody, which comprises culturing the transformant described in the fourth aspect of the present disclosure and obtaining the antibody from the culture.

In a sixth aspect, the present disclosure provides an antigen-binding protein derivative, which comprises the antibody described in the first aspect of the present disclosure and a detectable labeling molecule.

Preferably, the detectable labeling molecule is an enzyme, a radionuclide, a fluorescent dye, a luminescent substance, or biotin.

In a seventh aspect, the present disclosure provides an antibody-drug conjugate, which comprises a cytotoxic agent or a tag and the antibody described in the first aspect of the present disclosure.

In an eighth aspect, the present disclosure provides a chimeric antigen receptor, which comprises the antibody described in the first aspect of the present disclosure.

In a ninth aspect, the present disclosure provides a genetically modified cell, which comprises the chimeric antigen receptor described in the eighth aspect of the present disclosure.

Preferably, the genetically modified cell is a eukaryotic cell, such as an isolated human cell.

In a certain preferred embodiment, the genetically modified cell is an immune cell, such as a T cell or an NK cell.

In a tenth aspect, the present disclosure provides a multispecific antibody, which comprises the antibody described in the first aspect of the present disclosure.

Preferably, the multispecific antibody is a bispecific antibody.

In an eleventh aspect, the present disclosure provides a pharmaceutical composition, which comprises the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, or the multispecific antibody described in the tenth aspect of the present disclosure, and a pharmaceutically acceptable carrier.

In a twelfth aspect, the present disclosure provides a kit, which comprises the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, or the pharmaceutical composition described in the eleventh aspect of the present disclosure.

Preferably, the kit further comprises (i) a device for administering the antibody, the antigen-binding protein derivative, the antibody-drug conjugate, the chimeric antigen receptor, the genetically modified cell, or the pharmaceutical composition, and/or (ii) instructions.

In a thirteenth aspect, the present disclosure provides a kit-of-parts, which comprises a kit A and a kit B.

The kit A comprises the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, or the pharmaceutical composition described in the eleventh aspect of the present disclosure;
the kit B comprises other anti-tumor antibodies or a pharmaceutical composition comprising the other anti-tumor antibodies, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

In a fourteenth aspect, the present disclosure provides use of the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, the pharmaceutical composition described in the eleventh aspect of the present disclosure, the kit described in the twelfth aspect of the present disclosure, or the kit-of-parts described in the thirteenth aspect of the present disclosure in the manufacture of a product for the diagnosis, treatment, and/or prevention of cancer.

Preferably, the cancer is ILT4-positive cancer, ILT2-positive cancer, or ILT4-positive and ILT2-positive cancer.

More preferably, the cancer is solid cancer or hematological cancer.

The hematological cancer is, e.g., B-cell leukemia, lymphoma, acute myeloid leukemia, Burkitt lymphoma, mantle cell lymphoma, acute lymphoblastic leukemia, chronic lymphocytic leukemia, diffuse large B-cell lymphoma, follicular lymphoma, or marginal zone lymphoma.

The solid cancer is, e.g., squamous cell carcinoma of the head and neck, renal cell carcinoma, ovarian cancer, urothelial cancer, diffuse large B-cell carcinoma, non-small cell lung cancer, melanoma, metastatic renal cell carcinoma, metastatic colorectal cancer, small cell lung cancer, metastatic non-small cell lung cancer, breast cancer, lung cancer, head and neck cancer, colorectal cancer, prostate cancer, skin cancer, stomach cancer, intestinal cancer, cervical cancer, uterine cancer, endometrial cancer, bladder cancer, brain cancer, esophageal cancer, liver cancer, renal cancer, testicular cancer, mesothelioma, glioblastoma, cholangiocarcinoma, or pancreatic cancer.

In a certain preferred embodiment, the cancer is pancreatic cancer.

In a fifteenth aspect, the present disclosure provides a method for diagnosing, treating, and/or preventing an ILT4-mediated disease or disorder, which comprises administering to a patient in need thereof a therapeutically effective amount of the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, the pharmaceutical composition described in the eleventh aspect of the present disclosure, the kit described in the twelfth aspect of the present disclosure, or the kit-of-parts described in the thirteenth aspect of the present disclosure.

In a certain preferred embodiment, the disease or disorder is cancer.

Preferably, the cancer is an ILT4-positive and/or ILT2-positive cancer.

More preferably, the cancer is solid cancer or hematological cancer.

The hematological cancer is, e.g., B-cell leukemia, lymphoma, acute myeloid leukemia, Burkitt lymphoma, mantle cell lymphoma, acute lymphoblastic leukemia, chronic lymphocytic leukemia, diffuse large B-cell lymphoma, follicular lymphoma, or marginal zone lymphoma.

The solid cancer is, e.g., squamous cell carcinoma of the head and neck, renal cell carcinoma, ovarian cancer, urothelial cancer, diffuse large B-cell carcinoma, non-small cell lung cancer, melanoma, metastatic renal cell carcinoma, metastatic colorectal cancer, small cell lung cancer, metastatic non-small cell lung cancer, breast cancer, lung cancer, head and neck cancer, colorectal cancer, prostate cancer, skin cancer, stomach cancer, intestinal cancer, cervical cancer, uterine cancer, endometrial cancer, bladder cancer, brain cancer, esophageal cancer, liver cancer, renal cancer, testicular cancer, mesothelioma, glioblastoma, cholangiocarcinoma, or pancreatic cancer.

In a certain preferred embodiment, the cancer is pancreatic cancer.

In a sixteenth aspect, the present disclosure provides a method for immunoassaying or determining ILT4, which comprises using the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, the pharmaceutical composition described in the eleventh aspect of the present disclosure, the kit described in the twelfth aspect of the present disclosure, or the kit-of-parts described in the thirteenth aspect of the present disclosure.

Preferably, the assaying is for non-diagnostic purposes, and the applicable scenario for the non-diagnostic purpose may be immunoassay or determination involved in laboratory development or environment, etc.

In a seventeenth aspect, the present disclosure provides a combination therapy, which comprises administering to a patient in need thereof the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, the pharmaceutical composition described in the eleventh aspect of the present disclosure, the kit described in the twelfth aspect of the present disclosure, or the kit-of-parts described in the thirteenth aspect of the present disclosure, and
a second therapeutic agent, wherein the second therapeutic agent preferably comprises other anti-tumor antibodies or a pharmaceutical composition comprising the other anti-tumor antibodies, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

In an eighteenth aspect, the present disclosure provides an administration device, which comprises the antibody described in the first aspect of the present disclosure, the nucleic acid described in the second aspect of the present disclosure, the recombinant vector described in the third aspect of the present disclosure, the antigen-binding protein derivative described in the sixth aspect of the present disclosure, the antibody-drug conjugate described in the seventh aspect of the present disclosure, the chimeric antigen receptor described in the eighth aspect of the present disclosure, the genetically modified cell described in the ninth aspect of the present disclosure, the multispecific antibody described in the tenth aspect of the present disclosure, the pharmaceutical composition described in the eleventh aspect of the present disclosure, the kit described in the twelfth aspect of the present disclosure, or the kit-of-parts described in the thirteenth aspect of the present disclosure.

Preferably, the administration device further comprises a component, such as a syringe, an implantable administration device, or an infusion device, for containing or administering to a subject the antibody, the multispecific antibody, the antigen-binding protein derivative, the antibody-drug conjugate, the chimeric antigen receptor, the genetically modified cell, the pharmaceutical composition, the kit, or the kit-of-parts.

On the basis of the general knowledge in the art, the preferred conditions described above may be combined arbitrarily to obtain preferred embodiments of the present disclosure.

In the present disclosure, there are antibodies specifically targeting ILT4, which exhibit with higher antibody affinity than the control antibody from Merck. In addition, there are also antibodies targeting both ILT2 and ILT4 molecules.

Among them, PR302934, PR302264-2, and PR302264-3 are capable of targeting both ILT4 and ILT2.

The reagents and starting materials used in the present disclosure are commercially available.

The positive and progressive effects of the present disclosure are as follows:

The present disclosure provides a fully human antibody that can target ILT4. The antibodies of the present disclosure have the following properties: 1) some of the antibodies have an activity of binding to both human ILT2 and ILT4 and an activity of binding to cynomolgus monkey ILT4; 2) the antibodies of the present disclosure bind to human ILT4 with higher affinity than MK-4830 (Merck); 3) the antibodies of the present disclosure improve the anti-tumor effect through multiple mechanisms of action. Firstly, the antibodies of the present disclosure can promote differentiation of macrophages towards M1 to secrete TNF-α, thus improving the anti-tumor effect; secondly, the differentiated M1 cells exhibit better antigen presenting function, and thus the killing effect of T cells on tumors is improved; thirdly, the antibodies of the present disclosure act on MDSCs and TAMs in the tumor microenvironment to ameliorate the environment for immunosuppression in the tumor microenvironment and promote T cells' killing of tumors; 4) the anti-tumor effect of the antibodies of the present disclosure is significantly better than that of control antibodies MK-4830 (Merck) and NGM707 (NGM) in a mouse *in vivo* efficacy experiment.

### BRIEF DESCRIPTION OF THE DRAWINGS

FIG. 1 shows an assay on the binding capacity of ILT4 antibodies for the human/monkey ILT4-ECD protein.
FIG. 2 shows a flow cytometry-based assay on the binding capacity of ILT4 antibodies for CHO-huILT4 cells.
FIG. 3 shows an ELISA-based assay on the binding capacity of ILT-4 antibodies for ILT2-ECD protein.
FIG. 4 shows a flow cytometry-based assay on the capacity of ILT4 antibodies to block the binding of ILT4 to HLA-G.
FIG. 5 shows an ELISA-based assay on the capacity of ILT4 antibodies to block the binding of ILT4 to SEMA4A.
FIG. 6 shows an ELISA-based assay on macrophage activation by ILT4 antibodies.
FIG. 7 shows an ELISA-based assay on activation of peripheral blood mononuclear cells by ILT4 antibodies.
FIG. 8 shows an ELISA-based assay on the activation effect of ILT4 antibodies in mixed lymphocyte reaction.
FIG. 9 shows the pharmacodynamic experiment of ILT4 antibodies in SU8686 mouse tumor model.

### DETAILED DESCRIPTION

### Terms and Definitions

In the present application, the term "antibody" generally refers to a protein comprising an antigen-binding moiety, and optionally a scaffold or framework moiety that allows the antigen-binding moiety to adopt a conformation that facilitates binding of the antibody to the antigen. An antibody may typically comprise an antibody light chain variable region (VL) or an antibody heavy chain variable region (VH), or both. For example, the "heavy-chain antibody" in the present application comprises no VL regions but comprises VH regions only. The VH or VL regions can be further divided into hypervariable regions termed complementarity determining regions (CDRs), which are scattered over more conserved regions termed framework regions (FRs). Each VH or VL may consist of three CDRs and four FRs arranged from the amino-terminus to the carboxyl-terminus in the following order: FR1, CDR1, FR2, CDR2, FR3, CDR3, and FR4. The variable regions of the heavy and light chains comprise binding domains that interact with antigens. Examples of antibodies include, but are not limited to, full-length antibodies, heavy-chain antibodies (HCAbs), antigen-binding fragments (Fab, Fab', F(ab)₂, Fv fragments, F(ab')₂, scFv, di-scFv, and/or dAb), immunoconjugates, multispecific antibodies (e.g., bispecific antibodies), antibody fragments, antibody derivatives, antibody analogs, fusion proteins, etc., provided that they exhibit the desired antigen binding activity.

In the present application, the term "variable" generally refers to the fact that certain portions of the sequences of the variable domains of antibodies vary considerably, resulting in the binding and specificity of various particular antibodies to their particular antigens. However, the variability is not evenly distributed throughout the variable region of the antibody. It is concentrated in three segments in each of the light and heavy chain variable regions called CDRs or hypervariable regions (HVRs). FRs are more highly conserved portions of the variable domains. The variable domains of native heavy and light chains each comprise four FRs largely in a β-sheet configuration. The FRs are connected by three CDRs to form a loop connection, and in some cases to form part of a β-sheet structure. The CDRs in each chain are held in close proximity by the FRs and form, together with the CDRs from the other chain, antigen-binding sites of the antibody. The constant regions are not directly involved in the binding of the antibody to antigens, but they exhibit different effector functions, for example, being involved in antibody-dependent cytotoxicity of the antibody.

In the present application, the term "Fab" generally refers to the portion of a conventional antibody (e.g., IgG) that binds to an antigen, including the heavy chain variable region VH, the light chain variable region VL, the heavy chain constant region domain CH1, and the light chain constant region CL of the antibody. In conventional antibodies, the C-terminus of VH is linked to the N-terminus of CH1 to form a heavy chain Fd fragment, the C-terminus of VL is linked to the N-terminus of CL to form a light chain, and the C-terminus of CH1 is further linked to the hinge region and other constant region domains of the heavy chain to form a heavy chain. In some embodiments, "Fab" also refers to a variant structure of the Fab. For example, in certain embodiments, the C-terminus of VH is linked to the N-terminus of CL to form one polypeptide chain, and the C-terminus of VL is linked to the N-terminus of CH1 to form another polypeptide chain, in which case a Fab (cross VH/VL) structure is formed; in certain embodiments, CH1 of the Fab is not linked to the hinge region, but rather the C-terminus of CL is linked to the hinge region of the heavy chain, in which case a Fab (cross Fd/LC) structure is formed.

In the present application, the term "VH" generally refers to the heavy chain variable region VH domain of an antibody, i.e., the heavy chain variable region VH of a conventional antibody (H2L2 structure) from human or other animals, the heavy chain variable region VHH of a heavy-chain antibody (HCAb structure) from animals such as those of Camelidae species, or the heavy chain variable region VH of a fully human heavy-chain antibody (HCAb structure) produced using a Harbour HCAb transgenic mouse.

As used herein, "a" or "an" is used in the present disclosure to refer to one or more grammatical objects. Unless otherwise specifically stated in the content, the term "or" is used in the present disclosure to refer to, and is interchangeable with, the term "and/or".

The term "heavy-chain antibody" of the present disclosure, also known as HCAb antibody, refers to an antibody lacking an antibody light chain and comprising only a heavy chain, specifically comprising a heavy chain variable domain and an Fc constant domain, relative to a diabody (immunoglobulin).

A "human antibody" has amino acid sequences that correspond to the amino acid sequences generated by a human or a human cell or derived from a non-human source antibody that utilizes human antibody libraries or other human antibody coding sequences. This definition of a human antibody particularly excludes humanized antibodies comprising non-human antigen-binding residues.

The term "polynucleotide", "nucleic acid", or "nucleotide sequence" of the present disclosure refers to an isolated nucleic acid molecule, such as messenger RNA (mRNA), virus-derived RNA, DNA, or plasmid DNA (pDNA). The polynucleotide may comprise conventional phosphodiester bonds or unconventional bonds (e.g., amide bonds, such as those found in peptide nucleic acids (PNAs)). The term "nucleic acid molecule" refers to any one or more of nucleic acid segments, e.g., DNA or RNA fragments, present in a polynucleotide.

The term "isolated" nucleic acid molecule or polynucleotide of the present disclosure refers to a nucleic acid molecule, DNA or RNA, which has been separated from its natural environment. In the present disclosure, a recombinant polynucleotide encoding polypeptides contained in the vector is also isolated. Other examples of the isolated polynucleotide include recombinant polynucleotides in heterologous host cells or polynucleotides purified in solutions. The isolated polynucleotide includes a polynucleotide molecule generally contained in a cell, but the polynucleotide molecule exists extrachromosomally or at a chromosomal location that is different from its natural chromosomal location. The isolated RNA molecule includes *in vivo* or *in vitro* RNA transcripts of the present disclosure, which are in the forward or reverse strand form, or in the single-stranded or double-stranded form. The isolated polynucleotide or nucleic acid of the present disclosure further includes such molecules generated synthetically. In addition, the polynucleotide or nucleic acid may be or may include regulatory elements such as promoters, ribosome binding sites, or transcription terminators.

The terms "vector" and "expression vector" of the present disclosure are used interchangeably and refer to a DNA molecule for introducing a particular gene operably linked thereto into a target cell and directing the expression. The vector includes vectors that serve as self-replicating nucleic acid structures as well as vectors incorporated into the genome of a host cell into which they have been introduced. The expression vector of the present disclosure comprises an expression cassette. The expression vector enables transcriptions of large amounts of stable mRNA. Once the expression vector is within the target cell, a ribonucleic acid molecule or protein encoded by the gene is generated by cellular transcription and/or translation mechanism. In one embodiment, the expression vector of the present disclosure includes an expression cassette comprising a polynucleotide sequence encoding the bispecific antigen-binding molecule or the fragment thereof of the present disclosure.

The terms "host cell", "host cell line", and "host cell culture" are used interchangeably and refer to cells into which exogenous nucleic acids have been introduced, including progenies of such cells. Host cells include "transformants" and "transformed cells", including primary transformed cells and progenies derived therefrom. The nucleic acids of progenies may not be exactly identical to those of parent cells, and may contain mutations. The host cells are any type of cells that can be used to generate the bispecific antigen-binding molecules of the present disclosure. The host cells include cultured cells, for example, cultured mammalian cells such as CHO cells, HEK293 cells, BHK cells, NS0 cells, SP2/0 cells, YO myeloma cells, P3X63 mouse myeloma cells, PER cells, PER.C6 cells, or hybridoma cells, e.g., yeast cells, insect cells, and plant cells, and further include cells contained within transgenic animals, transgenic plants, or cultured plant or animal tissues.

"Affinity" or "binding affinity" refers to the strength of a non-covalent interaction between a single binding site of a molecule (such as an antibody) and its ligand (such as an antigen). The binding affinity may generally be represented by a dissociation constant (Kd), which is the ratio of a dissociation rate constant (k_{off}) to an association rate constant (kₒₙ). Therefore, equivalent affinities may include different rate constants, as long as the ratio of the rate constants remains the same. The affinity can be determined by conventional methods known in the art, such as surface plasmon resonance (SPR).

The term "Harbour HCAb transgenic mouse" herein is a transgenic mouse carrying an immune repertoire of human immunoglobulins. It is capable of producing new "heavy chain"-only antibodies that are only half the size of conventional IgG antibodies. The antibodies produced have only human antibody "heavy chain" variable domains and mouse Fc constant domains. Due to the absence of light chain, the antibody almost solves the problems of light chain mispairing and heterodimerization, enabling the technical platform to develop products that are difficult to realize by conventional antibody platforms.

The term "chimeric antigen receptor" means that a chimeric antigen receptor-T cell is conjugated *in vitro,* via the antigen-binding site of an antibody that recognizes a certain tumor antigen, with the intracellular portion of CD3-δ chain or FcεRIγ to form a chimeric protein, and the T cells of a patient are transfected by a gene transduction method to express the chimeric antigen receptor, so that the T cells of the patient can generate a large number of tumor-specific CAR-T cells after being "recoded". By introducing the recoded chimeric antigen receptor T cells into the patient, such chimeric antigen receptors, like GPS, can specifically track and recognize tumor cells, and direct T cells to kill them. Most chimeric antigen receptors consist of an extracellular antigen-binding region (consisting of a light chain and a heavy chain derived from a monoclonal antibody, connected by a flexible hinge region in the middle to form a single-chain antibody), a transmembrane region, and an intracellular signaling region. The CAR structure is obtained by genetic recombination *in vitro* of scFv that recognizes tumor-associated antigens with an intracellular signaling domain "immunoreceptor tyrosine-based activation motif".

The term "immune cells" include cells that are of hematopoietic origin and play a role in immune response, for example, lymphocytes such as B cells and T cells; natural killer cells; and myeloid cells such as monocytes, macrophages, eosinophils, mast cells, basophils, or granulocytes.

The term "antibody-drug conjugate" refers to an antigen-binding protein moiety and a conjugate moiety. The antigen-binding protein moiety includes "antigen-binding proteins" described above and antigen-binding fragments obtained on the basis of the "antigen-binding proteins". Examples of the antigen-binding fragments include, but are not limited to, a Fab, a Fab', an Fv fragment, a F(ab')₂, an scFv, a di-scFv, and/or a dAb, and the like. The conjugate moiety may comprise at least one payload. The payloads are a class of small molecule compounds, toxins, other drug molecule forms, or the aforementioned labeling molecules with pharmaceutical activity, which can be, but are not limited to, small molecule compounds, toxin molecules, oligonucleotides, proteolysis targeting chimeras (PROTACs), affinity ligands, fluorescent groups, nuclide groups, etc. A variety of payloads are known in the art. For example, small molecule compounds generally refer to a class of substances having strong cytotoxicity. Exemplary small molecule compounds can exert such cytotoxic and cytostatic effects by mechanisms including, but not limited to, tubulin binding, DNA binding, inhibition of RNA polymerase, protein synthesis, or inhibition of topoisomerase. For example, the small molecule compound may be a tubulin inhibitor; exemplary tubulin inhibitors may be maytansines (such as DM1 or DM4), auristatins (such as MMAE or MMAF), and the like. For example, the small molecule compound may be a DNA damaging agent; exemplary DNA damaging agents may be calicheamicins, pyrrolobenzodiazepines (PBDs), and the like. For example, the proteolysis targeting chimeras (PROTACs) are a class of compounds that can cause the degradation of a target protein by inducing polyubiquitination of the target protein; exemplary PROTACs may be BET protein degrading agents. The drug conjugate may further comprise at least one linker. The linker is used to link one or more payloads to the antigen-binding protein. For example, the linker comprises a cleavable linker or a non-cleavable linker. In the present application, the cleavable linker may be a "cleavable" linker favoring the release of drugs. Exemplary cleavable linkers may include, but are not limited to, an acid-sensitive linker, a protease-sensitive linker, a light-sensitive linker, or a disulfide-containing linker. The linker may comprise one or more linker members. A variety of linker members are known in the art, such as maleimidocaproyl (MC), maleimidopropionyl (MP), valine-citrulline (val-cit or vc), or paminobenzyloxycarbonyl (PAB).

The term "pharmaceutical composition" refers to a mixture containing one or more of the antibodies or the antigen-binding fragments thereof disclosed herein and other chemical components, such as physiologically/pharmaceutically acceptable carriers or excipients. The pharmaceutical composition is intended to promote administration to an organism and facilitate the absorption of the active ingredient so that it can exert its biological activity. The "pharmaceutical composition" in the present disclosure may comprise a combination of a first active ingredient and a second active ingredient, both of which are present together in one unit dose or in a single entity, wherein the first active ingredient and the second active ingredient are present in a mixture for administering simultaneously, for example in a formulation.

The term "pharmaceutically acceptable carrier" refers to a component of the pharmaceutical composition that is not toxic to a subject in addition to the active ingredients. The pharmaceutically acceptable excipients include, but are not limited to a buffer, a stabilizer, and/or a preservative.

The "kit-of-parts" in the present disclosure is used in a manner known to those skilled in the art and is defined as a combination in which the first active ingredient (kit A) and the second active ingredient (kit B) are present in more than one unit. One example of the kit-of-parts is a combination in which the first active ingredient and the second active ingredient are present independently. The components of the kit-of-parts may be administered separately, sequentially, simultaneously, concurrently, or in staggered chronological order. The antibodies or the antigen-binding proteins thereof of the present disclosure may be administered as a single agent or in combination with one or more other agents, wherein the combination does not cause unacceptable adverse events.

The term "cancer" refers to or describes a disease in mammals that is characterized by unregulated cell growth. Cancer includes, but is not limited to, tumors, such as lymphoma, blastoma, sarcoma and leukemia or lymphoid malignancy. More specific examples of cancer include, but are not limited to, squamous cell carcinoma (such as epithelial squamous cell carcinoma), lung cancer (including small cell lung cancer, non-small cell lung cancer, adenocarcinoma of lung, and squamous lung carcinoma), peritoneal cancer, hepatocellular carcinoma, stomach cancer (including gastrointestinal cancer and gastrointestinal stromal cancer), bone cancer, pancreatic cancer, cervical cancer, ovarian cancer, liver cancer, bladder cancer, urinary tract cancer, breast cancer, colon cancer, rectal cancer, endometrial cancer or cervical cancer, salivary gland carcinoma, renal cancer or ureteral cancer, prostate cancer, vaginal cancer, vulval cancer, thyroid cancer, anal cancer, penile cancer, melanoma, cholangiocarcinoma, central nervous system (CNS) tumor, spinal axis tumor, brain stem glioma, glioblastoma such as glioblastoma multiforme, astrocytoma, schwannoma, ependymoma, myeloblastoma, meningioma, squamous cell carcinoma, pituitary adenoma and Ewing's sarcoma, superficial spreading melanoma, lentigo maligna melanoma, acral melanoma, nodular melanoma, multiple myeloma and B-cell lymphoma, chronic lymphocytic leukemia (CLL), acute lymphoblastic leukemia (ALL), hairy cell leukemia, chronic myeloblastic leukemia and post-transplant lymphoproliferative disorder (PTLD), and abnormal vascular proliferation associated with phakomatoses, edema (such as those associated with brain tumors) and Meigs syndrome, brain tumor and brain cancer, and head or neck cancer, and related metastatic carcinomas.

"Treatment" refers to administering a therapeutic agent, such as a composition comprising any one of the antibodies or the antigen-binding fragments thereof disclosed herein or a nucleic acid molecule encoding the antibody or the antigen-binding fragment thereof, either internally or externally to a patient with one or more diseases or symptoms on which the therapeutic agent has a therapeutic effect. Typically, the therapeutic agent is administered in an amount effective to alleviate one or more diseases or symptoms to the patient or population being treated to induce regression of such symptoms or to inhibit the progression of such symptoms to any clinically measurable degree.

The term "preventing cancer" refers to delaying, inhibiting, or preventing the onset of cancer in mammals in which the initiation of carcinogenesis or tumorigenesis has not been confirmed, but a susceptibility to cancer has been identified as determined, for example, by genetic screening or other methods. The term also includes treating mammals having pre-cancerous disorders to terminate the progression of the pre-cancerous disorders to malignancies or to cause the regression thereof.

The term "administration device" mainly relates to packaging materials and injection systems. The packaging materials include, but are not limited to, vials, syringes, or tubes, and the injection systems include, but are not limited to, syringes, infusion pumps, injection pens, needleless devices, or subcutaneous patch delivery devices. The composition of the administration device may be conventional in the art and includes a container, a seal, and an injection needle. The container includes, but is not limited to a vial, a syringe, or a tube. The material of the container may be conventional in the art, such as glass or plastic. The seal includes, but is not limited to, a sealing plug or a sealing ring. The material of the seal may be conventional in the art, such as rubber, plastic, or polymer material. The injection needle includes, but is not limited to, a water needle, a single needle, or a microneedle group. The material of the injection needle may be conventional in the art, such as metal, silicon, silica, glass, nickel, titanium, or biodegradable polymers. The water needle includes, but is not limited to, a vial water needle, an ampoule water needle, or a pre-filled injection system. The ampoule water needle may be a glass ampoule or a plastic ampoule. The pre-filled injection system may be conventional in the art, such as a pre-filled syringe.

In the art, the CDRs of an antibody can be defined using a variety of methods, such as the Kabat scheme based on sequence variability (see Kabat et al., Sequences of Proteins of Immunological Interest, Fifth Edition, National Institutes of Health (U.S.), Bethesda, Maryland (1991)) and the Chothia scheme based on the location of the structural loop regions (see A1-Lazikani et al., J Mol Biol 273: 927-948, 1997). In the present application, the Combined scheme comprising the Kabat scheme and the Chothia scheme can also be used to determine the amino acid residues in variable domain sequences and full-length antibody sequences. In the present disclosure, the sequences determined according to the Combined scheme are shown in the table below.

See Table 1 for schemes for defining the CDRs of the antibodies of the present application (see http://bioinf.org.uk/abs/).

**Table 1. Schemes for defining CDRs of antibodies of the present application**

| | Kabat | Chothia | Combined |
|---|---|---|---|
| LCDR1 | L24--L34 | L24--L34 | L24-L34 |
| LCDR2 | L50--L56 | L50--L56 | L50-L56 |
| LCDR3 | L89--L97 | L89--L97 | L89-L97 |
| HCDR1 | H31--H35 | H26--H32 | H26-H35 |
| HCDR2 | H50--H65 | H52--H56 | H50-H65 |
| HCDR3 | H95--H102 | H95--H102 | H95-H102 |

Laa-Lbb may refer to an amino acid sequence from position aa (the Chothia scheme) to position bb (the Chothia scheme) beginning at the N-terminus of the light chain of the antibody; Haa-Hbb may refer to an amino acid sequence from position aa (the Chothia scheme) to position bb (the Chothia scheme) beginning at the N-terminus of the heavy chain of the antibody. For example, L24-L34 may refer to the amino acid sequence from position 24 to position 34 according to the Chothia scheme beginning at the N-terminus of the light chain of the antibody; H26-H32 may refer to the amino acid sequence from position 26 to position 32 according to the Chothia scheme beginning at the N-terminus of the heavy chain of the antibody.

See Table 2 below for the information on CDRs of the antibodies of the present disclosure.

**Table 2**

| Ab I | HCDR1 | HCDR2 | HCDR3 | LCDR1 | LCDR 2 | LCDR3 |
|---|---|---|---|---|---|---|
| **PR302264 (IgG1)** | GGSLSG YYWS | EINHSGGTNYNP SLKS | GQQQLDWY FDL | RASQNIYSHL V | GAST RAT | QQYND WPPIT |
| Sequence No. | SEQ ID NO: 1 | SEQ ID NO: 2 | SEQ ID NO: 3 | SEQ ID NO: 4 | SEQ ID NO: 5 | SEQ ID NO: 6 |
| **PR302264-1 (IgG4)** | GGSLSG YYWS | EIQHSGGTNYNP SLKS | GQQQLDWY FDL | RASQNIYSHL V | GAST RAT | QQYND WPPIT |
| Sequence No. | SEQ ID NO: 1 | SEQ ID NO: 7 | SEQ ID NO: 3 | SEQ ID NO: 4 | SEQ ID NO: 5 | SEQ ID NO: 6 |
| **PR302264-2 (IgG4)** | GGSLSG YYWS | EIAHSGGTNYNP SLKS | GQQQLDWY FDL | RASQNIYSHL V | GAST RAT | QQYND WPPIT |
| Sequence No. | SEQ ID NO: 1 | SEQ ID NO: 8 | SEQ ID NO: 3 | SEQ ID NO: 4 | SEQ ID NO: 5 | SEQ ID NO: 6 |
| **PR302264-3 (IgG4)** | GGSLSG YYWS | EITHSGGTNYNP SLKS | GQQQLDWY FDL | RASQNIYSHL V | GAST RAT | QQYND WPPIT |
| Sequence No. | SEQ ID NO: 1 | SEQ ID NO: 9 | SEQ ID NO: 3 | SEQ ID NO: 4 | SEQ ID NO: 5 | SEQ ID NO: 6 |
| **PR302264-4 (IgG4)** | GGSLSG YYWS | EINHAGGTNYN PSLKS | GQQQLDWY FDL | RASQNIYSHL V | GAST RAT | QQYND WPPIT |
| Sequence No. | SEQ ID NO: 1 | SEQ ID NO: 10 | SEQ ID NO: 3 | SEQ ID NO: 4 | SEQ ID NO: 5 | SEQ ID NO: 6 |
| **PR302264-5 (IgG4)** | GGSLSG YYWS | EINHGGGTNYN PSLKS | GQQQLDWY FDL | RASQNIYSHL V | GAST RAT | QQYND WPPIT |
| Sequence No. | SEQ ID NO: 1 | SEQ ID NO: 11 | SEQ ID NO: 3 | SEQ ID NO: 4 | SEQ ID NO: 5 | SEQ ID NO: 6 |
| **PR302951 (IgG1)** | RFTFSTY AMS | SISGSGFNTYYA DSVKG | DRQWLVSSF DY | QASQDISNYL N | DASN LET | QQYDNL IT |
| Sequence No. | SEQ ID NO: 12 | SEQ ID NO: 13 | SEQ ID NO: 14 | SEQ ID NO: 15 | SEQ ID NO: 16 | SEQ ID NO: 17 |
| **PR302951-1 (IgG4)** | RFTFSTY AMS | SISGSGFNTYYA DSVKG | DRQWLVSSF DY | QASQDISNYL N | DASN LET | QQYDNL IT |
| Sequence No. | SEQ ID NO: 12 | SEQ ID NO: 13 | SEQ ID NO: 14 | SEQ ID NO: 15 | SEQ ID NO: 16 | SEQ ID NO: 17 |
| **PR302239 (IgG1)** | GDSISSY YWS | FIYYSGSTNYNP SLKS | QLWFGESPF DY | RASQSVSSNL A | GAST RAT | QQYNN WPLT |
| Sequence No. | SEQ ID NO: 18 | SEQ ID NO: 19 | SEQ ID NO: 20 | SEQ ID NO: 21 | SEQ ID NO: 5 | SEQ ID NO: 22 |
| **PR302239-1 (IgG4)** | GDSISSY YWS | FIYYSGSTNYNP SLKS | QLWFGESPF DY | RASQSVSSNL A | GAST RAT | QQYNN WPLT |
| Sequence No. | SEQ ID NO: 18 | SEQ ID NO: 19 | SEQ ID NO: 20 | SEQ ID NO: 21 | SEQ ID NO: 5 | SEQ ID NO: 22 |
| **PR302259 (IgG1)** | GFTFSSY AMS | AISDNAGSTYYA DSVKG | ERQWLVLG VMDA | QASQDISNYL N | DASN LET | QQNDNL PFT |
| Sequence No. | SEQ ID NO: 23 | SEQ ID NO: 24 | SEQ ID NO: 25 | SEQ ID NO: 15 | SEQ ID NO: 16 | SEQ ID NO: 26 |
| **PR302259-1 (IgG4)** | GFTFSSY AMS | AISDNAGSTYYA DSVKG | ERQWLVLG VMDA | QASQDISNYL N | DASN LET | QQNDNL PFT |
| Sequence No. | SEQ ID NO: 23 | SEQ ID NO: 24 | SEQ ID NO: 25 | SEQ ID NO: 15 | SEQ ID NO: 16 | SEQ ID NO: 26 |
| **PR302261 (IgG1)** | GFTFSSY AMS | TISGSGGSAYYA DSVKG | LSGDDAFDI | RSSQSLLRGN GYNYLD | LGSN RAS | MQALQS PLT |
| Sequence No. | SEQ ID NO: 23 | SEQ ID NO: 27 | SEQ ID NO: 28 | SEQ ID NO: 29 | SEQ ID NO: 30 | SEQ ID NO: 31 |
| **PR302261-5 (IgG4)** | GFTFSSY AMS | TISGSGGSAYYA DSVKG | LSGDDAFDI | RSSQSLLRGN AYNYLD | LGSN RAS | MQALQS PLT |
| Sequence No. | SEQ ID NO: 23 | SEQ ID NO: 27 | SEQ ID NO: 28 | SEQ ID NO: 39 | SEQ ID NO: 30 | SEQ ID NO: 31 |
| **PR302282 (IgG1)** | GFTFSSY GMH | GMWYDGNYKY YADSVKG | DSPIGVFDY | QASQDISNYL N | DASN LET | QQYDNL IT |
| Sequence No. | SEQ ID NO: 32 | SEQ ID NO: 33 | SEQ ID NO: 34 | SEQ ID NO: 15 | SEQ ID NO: 16 | SEQ ID NO: 17 |
| **PR302282-1 (IgG4)** | GFTFSSY GMH | GMWYDGNYKY YADSVKG | DSPIGVFDY | QASQDISNYL N | DASN LET | QQYDNL IT |
| Sequence No. | SEQ ID NO: 32 | SEQ ID NO: 33 | SEQ ID NO: 34 | SEQ ID NO: 15 | SEQ ID NO: 16 | SEQ ID NO: 17 |
| **PR302282-2 (IgG4)** | GFTFSSY GMH | GMWYEGNYKY YADSVKG | DSPIGVFDY | QASQDISNYL N | DASN LET | QQYDNL IT |
| Sequence No. | SEQ ID NO: 32 | SEQ ID NO: 35 | SEQ ID NO: 34 | SEQ ID NO: 15 | SEQ ID NO: 16 | SEQ ID NO: 17 |
| **PR302702 (IgG1)** | GFTFSTY AMT | SISGSDFNTYYA DSVKG | DQEWLVRS FHH | QASQDISNYL N | DASN LET | QQYENL PT |
| Sequence No. | SEQ ID NO: 36 | SEQ ID NO: 37 | SEQ ID NO: 38 | SEQ ID NO: 15 | SEQ ID NO: 16 | SEQ ID NO: 40 |
| **PR302927 (IgG1)** | GFTFSSY AMS | TINHIGDSTYYA DSVKG | DQWFGVFD Y | QASQDISNYL N | DASN LET | QQYDNL PH |
| Sequence No. | SEQ ID NO: 23 | SEQ ID NO: 41 | SEQ ID NO: 42 | SEQ ID NO: 15 | SEQ ID NO: 16 | SEQ ID NO: 43 |
| **PR302934 (IgG1)** | GGSFSG YYWS | EVSHSGGTNYN PSLKS | SPYYGDYM YVFDI | RASQSVSSSY LA | GASS RAT | QQYGSL FT |
| Sequence No. | SEQ ID NO: 44 | SEQ ID NO: 45 | SEQ ID NO: 46 | SEQ ID NO: 47 | SEQ ID NO: 48 | SEQ ID NO: 49 |
| **PR304341 (IgG4)** | GGSFSG YYWS | EVSHSGGTNYN PSLKS | SPYYGDYM YVFDI | RASQSVSSSY LA | GASS RAT | QQYGSL FT |
| Sequence No. | SEQ ID NO: 44 | SEQ ID NO: 45 | SEQ ID NO: 46 | SEQ ID NO: 47 | SEQ ID NO: 48 | SEQ ID NO: 49 |
| **PR302949 (IgG1)** | GFTFSSY AMS | SISGSDFSTYYA DSVKG | DRQWLVSSF DF | QASQDISNYL N | DASN LET | QQYDNL IT |
| Sequence No. | SEQ ID NO: 23 | SEQ ID NO: 50 | SEQ ID NO: 51 | SEQ ID NO: 15 | SEQ ID NO: 16 | SEQ ID NO: 17 |
| **PR302949-1 (IgG4)** | GFTFSSY AMS | SISGSDFSTYYA DSVKG | DRQWLVSSF DF | QASQDISNYL N | DASN LET | QQYDNL IT |
| Sequence No. | SEQ ID NO: 23 | SEQ ID NO: 50 | SEQ ID NO: 51 | SEQ ID NO: 15 | SEQ ID NO: 16 | SEQ ID NO: 17 |
| **PR302950 (IgG1)** | GGSISSS NWWS | EVYHSGSTNYN PSLKS | VTGNKDYY YYDLDV | QASQDISNYL N | DASN LGT | QQYDNL PRT |
| Sequence No. | SEQ ID NO: 52 | SEQ ID NO: 53 | SEQ ID NO: 54 | SEQ ID NO: 15 | SEQ ID NO: 55 | SEQ ID NO: 56 |
| **PR303413 (IgG1)** | GFTFSSY GMH | GIWYAGSNEYY VDSVKG | DQGWGTMD A | QASQDISNYL N | DASN LET | QQYDNL LT |
| Sequence No. | SEQ ID NO: 32 | SEQ ID NO: 57 | SEQ ID NO: 58 | SEQ ID NO: 15 | SEQ ID NO: 16 | SEQ ID NO: 59 |
| **PR303413-1 (IgG4)** | GFTFSSY GMH | GIWYAGSNEYY VDSVKG | DQGWGTMD A | QASQDISNYL N | DASN LET | QQYDNL LT |
| Sequence No. | SEQ ID NO: 32 | SEQ ID NO: 57 | SEQ ID NO: 58 | SEQ ID NO: 15 | SEQ ID NO: 16 | SEQ ID NO: 59 |

See Table 3 below for the information on VHs and VLs of the antibodies of the present disclosure.

**Table 3**

| Ab ID | VH | SEQ ID NO: | VL | SEQ ID NO: |
|---|---|---|---|---|
| PR302 264 (IgG1) | QVQLQQWGAGLLKPSETLS LTCAVYGGSLSGYYWSWI RQPPGKGLEWIGEINHSGG TNYNPSLKSRVTISVPTSKN QFSLKLTSVTAADTAVYYC ARGQQQLDWYFDLWGRG TLVTVSS | 61 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIR | 62 |
| PR302 264-1 (IgG4) | QVQLQQWGAGLLKPSETLS LTCAVYGGSLSGYYWSWI RQPPGKGLEWIGEIQHSGG TNYNPSLKSRVTISVPTSKN QFSLKLTSVTAADTAVYYC ARGQQQLDWYFDLWGRG TLVTVSS | 63 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIR | 62 |
| PR302 264-2 (IgG4) | QVQLQQWGAGLLKPSETLS LTCAVYGGSLSGYYWSWI RQPPGKGLEWIGEIAHSGG TNYNPSLKSRVTISVPTSKN QFSLKLTSVTAADTAVYYC ARGQQQLDWYFDLWGRG TLVTVSS | 64 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIR | 62 |
| PR302 264-3 (IgG4) | QVQLQQWGAGLLKPSETLS LTCAVYGGSLSGYYWSWI RQPPGKGLEWIGEITHSGGT NYNPSLKSRVTISVPTSKNQ FSLKLTSVTAADTAVYYCA RGQQQLDWYFDLWGRGTL VTVSS | 65 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIR | 62 |
| PR302 264-4 (IgG4) | QVQLQQWGAGLLKPSETLS LTCAVYGGSLSGYYWSWI RQPPGKGLEWIGEINHAGG TNYNPSLKSRVTISVPTSKN QFSLKLTSVTAADTAVYYC ARGQQQLDWYFDLWGRG TLVTVSS | 66 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIR | 62 |
| PR302 264-5 (IgG4) | QVQLQQWGAGLLKPSETLS LTCAVYGGSLSGYYWSWI RQPPGKGLEWIGEINHGGG TNYNPSLKSRVTISVPTSKN QFSLKLTSVTAADTAVYYC ARGQQQLDWYFDLWGRG TLVTVSS | 67 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIR | 62 |
| PR302 951 (IgG1) | EVQLLESGGGLVQPGGSLR LSCAASRFTFSTYAMSWVR QAPGKGLEWVSSISGSGFN TYYADSVKGRFTISSDSSKN TLYLQMNSLRAEDTAVYY CAKDRQWLVSSFDYWGQG TLVTVSS | 68 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 69 |
| PR302 951-1 (IgG4) | EVQLLESGGGLVQPGGSLR LSCAASRFTFSTYAMSWVR QAPGKGLEWVSSISGSGFN TYYADSVKGRFTISSDSSKN TLYLQMNSLRAEDTAVYY CAKDRQWLVSSFDYWGQG TLVTVSS | 68 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 69 |
| PR302 239 (IgG1) | QVQLQESGPGLVKPSETLS LTCTVSGDSISSYYWSWIR QPPGKGLEWIGFIYYSGSTN YNPSLKSRVTISVDTSKNQF SLKLTSVTAADTAVYYCAN QLWFGESPFDYWGQGTLV TVSS | 70 | EIVMTQSPATLSVSPGER ATLSCRASQSVSSNLAW YQQKPGQAPRLLIYGAS TRATGIPARFSGSGSGTE FTLTISSLQSEDFAVYYC QQYNNWPLTFGGGTKV EIK | 71 |
| PR302 239-1 (IgG4) | QVQLQESGPGLVKPSETLS LTCTVSGDSISSYYWSWIR QPPGKGLEWIGFIYYSGSTN YNPSLKSRVTISVDTSKNQF SLKLTSVTAADTAVYYCAN QLWFGESPFDYWGQGTLV TVSS | 70 | EIVMTQSPATLSVSPGER ATLSCRASQSVSSNLAW YQQKPGQAPRLLIYGAS TRATGIPARFSGSGSGTE FTLTISSLQSEDFAVYYC QQYNNWPLTFGGGTKV EIK | 71 |
| PR302 259 (IgG1) | EVQLLESGGGLVQPGGSLR LSCAVSGFTFSSYAMSWVR QAPGKGLEWVSAISDNAGS TYYADSVKGRFTISRDNSK NTLHLQMNSLRAEDTAVY SCAKERQWLVLGVMDAW GQGASVTVSS | 72 | DIQMTQSPSSLSASVGD RVTITCQASQDISNYLN WYQQKPGKARKLLISD ASNLETGVPSRFSGSGS GTDFTFTISSLEPEDIATY HCQQNDNLPFTFGPGTK VEIK | 73 |
| PR302 259-1 (IgG4) | EVQLLESGGGLVQPGGSLR LSCAVSGFTFSSYAMSWVR QAPGKGLEWVSAISDNAGS TYYADSVKGRFTISRDNSK NTLHLQMNSLRAEDTAVY SCAKERQWLVLGVMDAW GQGASVTVSS | 72 | DIQMTQSPSSLSASVGD RVTITCQASQDISNYLN WYQQKPGKARKLLISD ASNLETGVPSRFSGSGS GTDFTFTISSLEPEDIATY HCQQNDNLPFTFGPGTK VEIK | 73 |
| PR302 261 (IgG1) | EVQLLESGGGLVQSGGSLR LSCAASGFTFSSYAMSWVR QAPGKGLGWVSTISGSGGS AYYADSVKGRFTVSRDNS KKTLYLQMNSLRAEDTAV YFCAILSGDDAFDIWGQGT MVTVSS | 74 | DVVMTQSPLSLPVTPGE PASISCRSSQSLLRGNGY NYLDWYLQKPGQSPQL LIYLGSNRASGVPDRFS GSGSGTDFTLKISRVEAE DVGIFYCMQALQSPLTF GGGTKVEIK | 75 |
| PR302 261-5 (IgG4) | EVQLLESGGGLVQSGGSLR LSCAASGFTFSSYAMSWVR QAPGKGLGWVSTISGSGGS AYYADSVKGRFTVSRDNS KKTLYLQMNSLRAEDTAV YFCAILSGDDAFDIWGQGT MVTVSS | 74 | DVVMTQSPLSLPVTPGE PASISCRSSQSLLRGNAY NYLDWYLQKPGQSPQL LIYLGSNRASGVPDRFS GSGSGTDFTLKISRVEAE DVGIFYCMQALQSPLTF GGGTKVEIK | 76 |
| PR302 282 (IgG1) | QVQLVESGGTVVQSGRSLR LSCAASGFTFSSYGMHWVR QAPGKGLEWVAGMWYDG NYKYYADSVKGRFTISRDN SKNTLSLQMNSLRAEDTAV YYCARDSPIGVFDYWGQG TLVTVSS | 77 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 78 |
| PR302 282-1 (IgG4) | QVQLVESGGTVVQSGRSLR LSCAASGFTFSSYGMHWVR QAPGKGLEWVAGMWYDG NYKYYADSVKGRFTISRDN SKNTLSLQMNSLRAEDTAV YYCARDSPIGVFDYWGQG TLVTVSS | 77 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 78 |
| PR302 282-2 (IgG4) | QVQLVESGGTVVQSGRSLR LSCAASGFTFSSYGMHWVR QAPGKGLEWVAGMWYEG NYKYYADSVKGRFTISRDN SKNTLSLQMNSLRAEDTAV YYCARDSPIGVFDYWGQG TLVTVSS | 79 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 78 |
| PR302 702 (IgG1) | EVQLLESGGGLVQPGGSLR LSCAASGFTFSTYAMTWVR QAPGKGLEWVSSISGSDFN TYYADSVKGRFTISRDISKN TLYLQMNSLRAEDTAVYY CAKDQEWLVRSFHHWGQG TLVTVSS | 80 | DIQMTQFPSSLFAFVGD RVTITCQASQDISNYLN WYQQKAGKAPKLLIND ASNLETGVPSRFSGSGF GTDFTFTISSLQPEDFAT YYCQQYENLPTFGGGT KVEIK | 81 |
| PR302 927 (IgG1) | EVQLLESGGGLVQPGGSLR LSCAASGFTFSSYAMSWVR QAPGKGLEWVSTINHIGDS TYYADSVKGRFTISRDNSK NTLYLQMNSLRAEDTAVY YCAKDQWFGVFDYWGQG TLVTVSS | 82 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLPHFGQGT KLEIK | 83 |
| PR302 934 (IgG1) | QVQLQQWGAGLLKPSETLS LTCAVYGGSFSGYYWSWIR QTLGKGLEWIGEVSHSGGT NYNPSLKSRVTISVDTSKN QFSLKLSSVTAADTAVYYC ARSPYYGDYMYVFDIWGQ GTMVTVSS | 84 | EIVLTQSPGTLSLSPGER ATLSCRASQSVSSSYLA WYQQKPGQAPRLLIYG ASSRATGIPDRFSGSGSG TDFTLTISRLEPEDFAVY YCQQYGSLFTFGGGTKV EIK | 85 |
| PR304 341 (IgG4) | QVQLQQWGAGLLKPSETLS LTCAVYGGSFSGYYWSWIR QTLGKGLEWIGEVSHSGGT NYNPSLKSRVTISVDTSKN QFSLKLSSVTAADTAVYYC ARSPYYGDYMYVFDIWGQ GTMVTVSS | 84 | EIVLTQSPGTLSLSPGER ATLSCRASQSVSSSYLA WYQQKPGQAPRLLIYG ASSRATGIPDRFSGSGSG TDFTLTISRLEPEDFAVY YCQQYGSLFTFGGGTKV EIK | 85 |
| PR302 949 (IgG1) | EVQLLESGGGLVQPGGSLR LSCAASGFTFSSYAMSWVR QAPGKGLEWVSSISGSDFST YYADSVKGRFTISRDTSKN TLYLQMNSLRAEDTAVYY CAKDRQWLVSSFDFWGQG ALVTVSS | 86 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 78 |
| PR302 949-1 (IgG4) | EVQLLESGGGLVQPGGSLR LSCAASGFTFSSYAMSWVR QAPGKGLEWVSSISGSDFST YYADSVKGRFTISRDTSKN TLYLQMNSLRAEDTAVYY CAKDRQWLVSSFDFWGQG ALVTVSS | 86 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIK | 78 |
| PR302 950 (IgG1) | QVQLQESGPGLVKPSGTLS LTCAVSGGSISSSNWWSWV RQPPGKGLEWIGEVYHSGS TNYNPSLKSRVTISVDKSQ NQFSLKLTSVTAADTAVYY CARVTGNKDYYYYDLDV WGQGTTVTVSS | 87 | DIQMTQSPSSLSASVGD RVTITCQASQDISNYLN WYQQKPGKAPKLLIYD ASNLGTGVPSRFSGGGS GTDFTFTISSLQPEDIAT YYCQQYDNLPRTFGQG TKVEIK | 88 |
| PR303 413 (IgG1) | QVQLVESGGGVVQPGRSLR LSCAASGFTFSSYGMHWVR QAPGKGLGWVAGIWYAGS NEYYVDSVKGRFIISRDNSK NTLYLQMNSLRAEDTAVY YCARDQGWGTMDAWGQG ASVTVSS | 60 | DIQMTQSPSSLSASVGD RVTITCQASQDISNYLN WYQQKPGKAPKLLIYD ASNLETGVPSRFSGSGS GTDFTFTISSLQPEDIAIY YCQQYDNLLTFGGGTK VEIK | 89 |
| PR303 413-1 (IgG4) | QVQLVESGGGVVQPGRSLR LSCAASGFTFSSYGMHWVR QAPGKGLGWVAGIWYAGS NEYYVDSVKGRFIISRDNSK NTLYLQMNSLRAEDTAVY YCARDQGWGTMDAWGQG ASVTVSS | 60 | DIQMTQSPSSLSASVGD RVTITCQASQDISNYLN WYQQKPGKAPKLLIYD ASNLETGVPSRFSGSGS GTDFTFTISSLQPEDIAIY YCQQYDNLLTFGGGTK VEIK | 89 |

See Table 4 below for the information on light chains and heavy chains of the antibodies of the present disclosure.

**Table 4**

| Ab ID | HC | SEQ ID NO: | LC | SEQ ID NO: |
|---|---|---|---|---|
| PR302264 (IgG1) | QVQLQQWGAGLLK PSETLSLTCAVYGG SLSGYYWSWIRQPP GKGLEWIGEINHSG GTNYNPSLKSRVTIS VPTSKNQFSLKLTS VTAADTAVYYCAR GQQQLDWYFDLW GRGTLVTVSSASTK GPSVFPLAPSSKSTS GGTAALGCLVKDY FPEPVTVSWNSGAL TSGVHTFPAVLQSS GLYSLSSVVTVPSSS LGTQTYICNVNHKP SNTKVDKKVEPKSC DKTHTCPPCPAPEL LGGPSVFLFPPKPK DTLMISRTPEVTCV VVDVSHEDPEVKFN WYVDGVEVHNAKT KPREEQYNSTYRVV SVLTVLHQDWLNG KEYKCKVSNKALP APIEKTISKAKGQPR EPQVYTLPPSREEM TKNQVSLTCLVKGF YPSDIAVEWESNGQ PENNYKTTPPVLDS DGSFFLYSKLTVDK SRWQQGNVFSCSV MHEALHNHYTQKS LSLSPGK | 90 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIRRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 91 |
| PR302264-1 (IgG4) | QVQLQQWGAGLLK PSETLSLTCAVYGG SLSGYYWSWIRQPP | 92 | EIVMTQSPATLSVSPGER ATLSCRASQNIYSHLVW YQQKPGQAPRLLIYGAS | 91 |
| | GKGLEWIGEIQHSG GTNYNPSLKSRVTIS VPTSKNQFSLKLTS VTAADTAVYYCAR GQQQLDWYFDLW GRGTLVTVSSASTK GPSVFPLAPCSRSTS ESTAALGCLVKDYF PEPVTVSWNSGALT SGVHTFPAVLQSSG LYSLSSVVTVPSSSL GTKTYTCNVDHKPS NTKVDKRVESKYG PPCPPCPAPEFLGGP SVFLFPPKPKDTLMI SRTPEVTCVVVDVS QEDPEVQFNWYVD GVEVHNAKTKPRE EQFNSTYRVVSVLT VLHQDWLNGKEYK CKVSNKGLPSSIEKT ISKAKGQPREPQVY TLPPSQEEMTKNQV SLTCLVKGFYPSDIA VEWESNGQPENNY KTTPPVLDSDGSFFL YSRLTVDKSRWQE GNVFSCSVMHEAL HNHYTQKSLSLSLG K | | TRATGIPARFSGSGSGTE FTLTITSLQSEDFTVYYC QQYNDWPPITFGQGTRL EIRRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | |
| PR302264-2 (IgG4) | QVQLQQWGAGLLK PSETLSLTCAVYGG SLSGYYWSWIRQPP GKGLEWIGEIAHSG GTNYNPSLKSRVTIS VPTSKNQFSLKLTS VTAADTAVYYCAR GQQQLDWYFDLW GRGTLVTVSSASTK GPSVFPLAPCSRSTS ESTAALGCLVKDYF PEPVTVSWNSGALT SGVHTFPAVLQSSG LYSLSSVVTVPSSSL GTKTYTCNVDHKPS NTKVDKRVESKYG PPCPPCPAPEFLGGP SVFLFPPKPKDTLMI SRTPEVTCVVVDVS QEDPEVQFNWYVD GVEVHNAKTKPRE EQFNSTYRVVSVLT VLHQDWLNGKEYK CKVSNKGLPSSIEKT ISKAKGQPREPQVY TLPPSQEEMTKNQV SLTCLVKGFYPSDIA | 93 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIRRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 91 |
| | VEWESNGQPENNY KTTPPVLDSDGSFFL YSRLTVDKSRWQE GNVFSCSVMHEAL HNHYTQKSLSLSLG K | | | |
| PR302264-3 (IgG4) | QVQLQQWGAGLLK PSETLSLTCAVYGG SLSGYYWSWIRQPP GKGLEWIGEITHSG GTNYNPSLKSRVTIS VPTSKNQFSLKLTS VTAADTAVYYCAR GQQQLDWYFDLW GRGTLVTVSSASTK GPSVFPLAPCSRSTS ESTAALGCLVKDYF PEPVTVSWNSGALT SGVHTFPAVLQSSG LYSLSSVVTVPSSSL GTKTYTCNVDHKPS NTKVDKRVESKYG PPCPPCPAPEFLGGP SVFLFPPKPKDTLMI SRTPEVTCVVVDVS QEDPEVQFNWYVD GVEVHNAKTKPRE EQFNSTYRVVSVLT VLHQDWLNGKEYK CKVSNKGLPSSIEKT ISKAKGQPREPQVY TLPPSQEEMTKNQV SLTCLVKGFYPSDIA VEWESNGQPENNY KTTPPVLDSDGSFFL YSRLTVDKSRWQE GNVFSCSVMHEAL HNHYTQKSLSLSLG K | 94 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIRRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 91 |
| PR302264-4 (IgG4) | QVQLQQWGAGLLK PSETLSLTCAVYGG SLSGYYWSWIRQPP GKGLEWIGEINHAG GTNYNPSLKSRVTIS VPTSKNQFSLKLTS VTAADTAVYYCAR GQQQLDWYFDLW GRGTLVTVSSASTK GPSVFPLAPCSRSTS ESTAALGCLVKDYF PEPVTVSWNSGALT SGVHTFPAVLQSSG LYSLSSVVTVPSSSL GTKTYTCNVDHKPS NTKVDKRVESKYG PPCPPCPAPEFLGGP | 95 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIRRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 91 |
| | SVFLFPPKPKDTLMI SRTPEVTCVVVDVS QEDPEVQFNWYVD GVEVHNAKTKPRE EQFNSTYRVVSVLT VLHQDWLNGKEYK CKVSNKGLPSSIEKT ISKAKGQPREPQVY TLPPSQEEMTKNQV SLTCLVKGFYPSDIA VEWESNGQPENNY KTTPPVLDSDGSFFL YSRLTVDKSRWQE GNVFSCSVMHEAL HNHYTQKSLSLSLG K | | | |
| PR302264-5 (IgG4) | QVQLQQWGAGLLK PSETLSLTCAVYGG SLSGYYWSWIRQPP GKGLEWIGEINHGG GTNYNPSLKSRVTIS VPTSKNQFSLKLTS VTAADTAVYYCAR GQQQLDWYFDLW GRGTLVTVSSASTK GPSVFPLAPCSRSTS ESTAALGCLVKDYF PEPVTVSWNSGALT SGVHTFPAVLQSSG LYSLSSVVTVPSSSL GTKTYTCNVDHKPS NTKVDKRVESKYG PPCPPCPAPEFLGGP SVFLFPPKPKDTLMI SRTPEVTCVVVDVS QEDPEVQFNWYVD GVEVHNAKTKPRE EQFNSTYRVVSVLT VLHQDWLNGKEYK CKVSNKGLPSSIEKT ISKAKGQPREPQVY TLPPSQEEMTKNQV SLTCLVKGFYPSDIA VEWESNGQPENNY KTTPPVLDSDGSFFL YSRLTVDKSRWQE GNVFSCSVMHEAL HNHYTQKSLSLSLG K | 96 | EIVMTQSPATLSVSPGER ATLSCRASQNIYSHLVW YQQKPGQAPRLLIYGAS TRATGIPARFSGSGSGTE FTLTITSLQSEDFTVYYC QQYNDWPPITFGQGTRL EIRRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 91 |
| PR302951 (IgG1) | EVQLLESGGGLVQP GGSLRLSCAASRFT FSTYAMSWVRQAP GKGLEWVSSISGSG FNTYYADSVKGRFT ISSDSSKNTLYLQM NSLRAEDTAVYYC | 97 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD | 98 |
| | AKDRQWLVSSFDY WGQGTLVTVSSAST KGPSVFPLAPSSKST SGGTAALGCLVKD YFPEPVTVSWNSGA LTSGVHTFPAVLQS SGLYSLSSVVTVPSS SLGTQTYICNVNHK PSNTKVDKKVEPKS CDKTHTCPPCPAPE LLGGPSVFLFPPKPK DTLMISRTPEVTCV VVDVSHEDPEVKFN WYVDGVEVHNAKT KPREEQYNSTYRVV SVLTVLHQDWLNG KEYKCKVSNKALP APIEKTISKAKGQPR EPQVYTLPPSREEM TKNQVSLTCLVKGF YPSDIAVEWESNGQ PENNYKTTPPVLDS DGSFFLYSKLTVDK SRWQQGNVFSCSV MHEALHNHYTQKS LSLSPGK | | EQLKSGTASVVCLLNNF YPREAKVQWKVDNALQ SGNSQESVTEQDSKDST YSLSSTLTLSKADYEKH KVYACEVTHQGLSSPVT KSFNRGEC | |
| PR302951-1 (IgG4) | EVQLLESGGGLVQP GGSLRLSCAASRFT FSTYAMSWVRQAP GKGLEWVSSISGSG FNTYYADSVKGRFT ISSDSSKNTLYLQM NSLRAEDTAVYYC AKDRQWLVSSFDY WGQGTLVTVSSAST KGPSVFPLAPCSRST SESTAALGCLVKDY FPEPVTVSWNSGAL TSGVHTFPAVLQSS GLYSLSSVVTVPSSS LGTKTYTCNVDHK PSNTKVDKRVESKY GPPCPPCPAPEFLGG PSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH | 99 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD EQLKSGTASVVCLLNNF YPREAKVQWKVDNALQ SGNSQESVTEQDSKDST YSLSSTLTLSKADYEKH KVYACEVTHQGLSSPVT KSFNRGEC | 98 |
| | EALHNHYTQKSLSL SLGK | | | |
| PR302239 (IgG1) | QVQLQESGPGLVKP SETLSLTCTVSGDSI SSYYWSWIRQPPGK GLEWIGFIYYSGST NYNPSLKSRVTISV DTSKNQFSLKLTSV TAADTAVYYCANQ LWFGESPFDYWGQ GTLVTVSSASTKGP SVFPLAPSSKSTSGG TAALGCLVKDYFPE PVTVSWNSGALTSG VHTFPAVLQSSGLY SLSSVVTVPSSSLGT QTYICNVNHKPSNT KVDKKVEPKSCDK THTCPPCPAPELLG GPSVFLFPPKPKDTL MISRTPEVTCVVVD VSHEDPEVKFNWY VDGVEVHNAKTKP REEQYNSTYRVVSV LTVLHQDWLNGKE YKCKVSNKALPAPI EKTISKAKGQPREP QVYTLPPSREEMTK NQVSLTCLVKGFYP SDIAVEWESNGQPE NNYKTTPPVLDSDG SFFLYSKLTVDKSR WQQGNVFSCSVMH EALHNHYTQKSLSL SPGK | 100 | EIVMTQSPATLSVSPGER ATLSCRASQSVSSNLAW YQQKPGQAPRLLIYGAS TRATGIPARFSGSGSGTE FTLTISSLQSEDFAVYYC QQYNNWPLTFGGGTKV EIKRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 101 |
| PR302239-1 (IgG4) | QVQLQESGPGLVKP SETLSLTCTVSGDSI SSYYWSWIRQPPGK GLEWIGFIYYSGST NYNPSLKSRVTISV DTSKNQFSLKLTSV TAADTAVYYCANQ LWFGESPFDYWGQ GTLVTVSSASTKGP SVFPLAPCSRSTSES TAALGCLVKDYFPE PVTVSWNSGALTSG VHTFPAVLQSSGLY SLSSVVTVPSSSLGT KTYTCNVDHKPSNT KVDKRVESKYGPPC PPCPAPEFLGGPSVF LFPPKPKDTLMISRT PEVTCVVVDVSQED PEVQFNWYVDGVE VHNAKTKPREEQFN | 102 | EIVMTQSPATLSVSPGER ATLSCRASQSVSSNLAW YQQKPGQAPRLLIYGAS TRATGIPARFSGSGSGTE FTLTISSLQSEDFAVYYC QQYNNWPLTFGGGTKV EIKRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 101 |
| | STYRVVSVLTVLHQ DWLNGKEYKCKVS NKGLPSSIEKTISKA KGQPREPQVYTLPP SQEEMTKNQVSLTC LVKGFYPSDIAVEW ESNGQPENNYKTTP PVLDSDGSFFLYSR LTVDKSRWQEGNV FSCSVMHEALHNH YTQKSLSLSLGK | | | |
| PR302259 (IgG1) | EVQLLESGGGLVQP GGSLRLSCAVSGFT FSSYAMSWVRQAP GKGLEWVSAISDNA GSTYYADSVKGRFT ISRDNSKNTLHLQM NSLRAEDTAVYSCA KERQWLVLGVMDA WGQGASVTVSSAS TKGPSVFPLAPSSKS TSGGTAALGCLVK DYFPEPVTVSWNSG ALTSGVHTFPAVLQ SSGLYSLSSVVTVPS SSLGTQTYICNVNH KPSNTKVDKKVEPK SCDKTHTCPPCPAP ELLGGPSVFLFPPKP KDTLMISRTPEVTC VVVDVSHEDPEVKF NWYVDGVEVHNA KTKPREEQYNSTYR VVSVLTVLHQDWL NGKEYKCKVSNKA LPAPIEKTISKAKGQ PREPQVYTLPPSREE MTKNQVSLTCLVK GFYPSDIAVEWESN GQPENNYKTTPPVL DSDGSFFLYSKLTV DKSRWQQGNVFSC SVMHEALHNHYTQ KSLSLSPGK | 103 | DIQMTQSPSSLSASVGD RVTITCQASQDISNYLN WYQQKPGKARKLLISD ASNLETGVPSRFSGSGS GTDFTFTISSLEPEDIATY HCQQNDNLPFTFGPGTK VEIKRTVAAPSVFIFPPS DEQLKSGTASVVCLLNN FYPREAKVQWKVDNAL QSGNSQESVTEQDSKDS TYSLSSTLTLSKADYEK HKVYACEVTHQGLSSPV TKSFNRGEC | 104 |
| PR302259-1 (IgG4) | EVQLLESGGGLVQP GGSLRLSCAVSGFT FSSYAMSWVRQAP GKGLEWVSAISDNA GSTYYADSVKGRFT ISRDNSKNTLHLQM NSLRAEDTAVYSCA KERQWLVLGVMDA WGQGASVTVSSAS TKGPSVFPLAPCSRS TSESTAALGCLVKD YFPEPVTVSWNSGA | 105 | DIQMTQSPSSLSASVGD RVTITCQASQDISNYLN WYQQKPGKARKLLISD ASNLETGVPSRFSGSGS GTDFTFTISSLEPEDIATY HCQQNDNLPFTFGPGTK VEIKRTVAAPSVFIFPPS DEQLKSGTASVVCLLNN FYPREAKVQWKVDNAL QSGNSQESVTEQDSKDS TYSLSSTLTLSKADYEK | 104 |
| | LTSGVHTFPAVLQS SGLYSLSSVVTVPSS SLGTKTYTCNVDH KPSNTKVDKRVESK YGPPCPPCPAPEFLG GPSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH EALHNHYTQKSLSL SLGK | | HKVYACEVTHQGLSSPV TKSFNRGEC | |
| PR302261 (IgG1) | EVQLLESGGGLVQS GGSLRLSCAASGFT FSSYAMSWVRQAP GKGLGWVSTISGSG GSAYYADSVKGRF TVSRDNSKKTLYLQ MNSLRAEDTAVYF CAILSGDDAFDIWG QGTMVTVSSASTK GPSVFPLAPSSKSTS GGTAALGCLVKDY FPEPVTVSWNSGAL TSGVHTFPAVLQSS GLYSLSSVVTVPSSS LGTQTYICNVNHKP SNTKVDKKVEPKSC DKTHTCPPCPAPEL LGGPSVFLFPPKPK DTLMISRTPEVTCV VVDVSHEDPEVKFN WYVDGVEVHNAKT KPREEQYNSTYRVV SVLTVLHQDWLNG KEYKCKVSNKALP APIEKTISKAKGQPR EPQVYTLPPSREEM TKNQVSLTCLVKGF YPSDIAVEWESNGQ PENNYKTTPPVLDS DGSFFLYSKLTVDK SRWQQGNVFSCSV MHEALHNHYTQKS LSLSPGK | 106 | DVVMTQSPLSLPVTPGE PASISCRSSQSLLRGNGY NYLDWYLQKPGQSPQL LIYLGSNRASGVPDRFS GSGSGTDFTLKISRVEAE DVGIFYCMQALQSPLTF GGGTKVEIKRTVAAPSV FIFPPSDEQLKSGTASVV CLLNNFYPREAKVQWK VDNALQSGNSQESVTEQ DSKDSTYSLSSTLTLSKA DYEKHKVYACEVTHQG LSSPVTKSFNRGEC | 107 |
| PR302261-5 (IgG4) | EVQLLESGGGLVQS GGSLRLSCAASGFT | 108 | DVVMTQSPLSLPVTPGE PASISCRSSQSLLRGNAY | 109 |
| | FSSYAMSWVRQAP GKGLGWVSTISGSG GSAYYADSVKGRF TVSRDNSKKTLYLQ MNSLRAEDTAVYF CAILSGDDAFDIWG QGTMVTVSSASTK GPSVFPLAPCSRSTS ESTAALGCLVKDYF PEPVTVSWNSGALT SGVHTFPAVLQSSG LYSLSSVVTVPSSSL GTKTYTCNVDHKPS NTKVDKRVESKYG PPCPPCPAPEFLGGP SVFLFPPKPKDTLMI SRTPEVTCVVVDVS QEDPEVQFNWYVD GVEVHNAKTKPRE EQFNSTYRVVSVLT VLHQDWLNGKEYK CKVSNKGLPSSIEKT ISKAKGQPREPQVY TLPPSQEEMTKNQV SLTCLVKGFYPSDIA VEWESNGQPENNY KTTPPVLDSDGSFFL YSRLTVDKSRWQE GNVFSCSVMHEAL HNHYTQKSLSLSLG K | | NYLDWYLQKPGQSPQL LIYLGSNRASGVPDRFS GSGSGTDFTLKISRVEAE DVGIFYCMQALQSPLTF GGGTKVEIKRTVAAPSV FIFPPSDEQLKSGTASVV CLLNNFYPREAKVQWK VDNALQSGNSQESVTEQ DSKDSTYSLSSTLTLSKA DYEKHKVYACEVTHQG LSSPVTKSFNRGEC | |
| PR302282 (IgG1) | QVQLVESGGTVVQ SGRSLRLSCAASGF TFSSYGMHWVRQA PGKGLEWVAGMW YDGNYKYYADSVK GRFTISRDNSKNTLS LQMNSLRAEDTAV YYCARDSPIGVFDY WGQGTLVTVSSAST KGPSVFPLAPSSKST SGGTAALGCLVKD YFPEPVTVSWNSGA LTSGVHTFPAVLQS SGLYSLSSVVTVPSS SLGTQTYICNVNHK PSNTKVDKKVEPKS CDKTHTCPPCPAPE LLGGPSVFLFPPKPK DTLMISRTPEVTCV VVDVSHEDPEVKFN WYVDGVEVHNAKT KPREEQYNSTYRVV SVLTVLHQDWLNG KEYKCKVSNKALP APIEKTISKAKGQPR EPQVYTLPPSREEM | 110 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD EQLKSGTASVVCLLNNF YPREAKVQWKVDNALQ SGNSQESVTEQDSKDST YSLSSTLTLSKADYEKH KVYACEVTHQGLSSPVT KSFNRGEC | 98 |
| | TKNQVSLTCLVKGF YPSDIAVEWESNGQ PENNYKTTPPVLDS DGSFFLYSKLTVDK SRWQQGNVFSCSV MHEALHNHYTQKS LSLSPGK | | | |
| PR302282-1 (IgG4) | QVQLVESGGTVVQ SGRSLRLSCAASGF TFSSYGMHWVRQA PGKGLEWVAGMW YDGNYKYYADSVK GRFTISRDNSKNTLS LQMNSLRAEDTAV YYCARDSPIGVFDY WGQGTLVTVSSAST KGPSVFPLAPCSRST SESTAALGCLVKDY FPEPVTVSWNSGAL TSGVHTFPAVLQSS GLYSLSSVVTVPSSS LGTKTYTCNVDHK PSNTKVDKRVESKY GPPCPPCPAPEFLGG PSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH EALHNHYTQKSLSL SLGK | 111 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD EQLKSGTASVVCLLNNF YPREAKVQWKVDNALQ SGNSQESVTEQDSKDST YSLSSTLTLSKADYEKH KVYACEVTHQGLSSPVT KSFNRGEC | 98 |
| PR302282-2 (IgG4) | QVQLVESGGTVVQ SGRSLRLSCAASGF TFSSYGMHWVRQA PGKGLEWVAGMW YEGNYKYYADSVK GRFTISRDNSKNTLS LQMNSLRAEDTAV YYCARDSPIGVFDY WGQGTLVTVSSAST KGPSVFPLAPCSRST SESTAALGCLVKDY FPEPVTVSWNSGAL TSGVHTFPAVLQSS GLYSLSSVVTVPSSS LGTKTYTCNVDHK PSNTKVDKRVESKY | 112 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD EQLKSGTASVVCLLNNF YPREAKVQWKVDNALQ SGNSQESVTEQDSKDST YSLSSTLTLSKADYEKH KVYACEVTHQGLSSPVT KSFNRGEC | 98 |
| | GPPCPPCPAPEFLGG PSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH EALHNHYTQKSLSL SLGK | | | |
| PR302702 (IgG1) | EVQLLESGGGLVQP GGSLRLSCAASGFT FSTYAMTWVRQAP GKGLEWVSSISGSD FNTYYADSVKGRFT ISRDISKNTLYLQM NSLRAEDTAVYYC AKDQEWLVRSFHH WGQGTLVTVSSAST KGPSVFPLAPSSKST SGGTAALGCLVKD YFPEPVTVSWNSGA LTSGVHTFPAVLQS SGLYSLSSVVTVPSS SLGTQTYICNVNHK PSNTKVDKKVEPKS CDKTHTCPPCPAPE LLGGPSVFLFPPKPK DTLMISRTPEVTCV VVDVSHEDPEVKFN WYVDGVEVHNAKT KPREEQYNSTYRVV SVLTVLHQDWLNG KEYKCKVSNKALP APIEKTISKAKGQPR EPQVYTLPPSREEM TKNQVSLTCLVKGF YPSDIAVEWESNGQ PENNYKTTPPVLDS DGSFFLYSKLTVDK SRWQQGNVFSCSV MHEALHNHYTQKS LSLSPGK | 113 | DIQMTQFPSSLFAFVGD RVTITCQASQDISNYLN WYQQKAGKAPKLLIND ASNLETGVPSRFSGSGF GTDFTFTISSLQPEDFAT YYCQQYENLPTFGGGT KVEIKRTVAAPSVFIFPP SDEQLKSGTASVVCLLN NFYPREAKVQWKVDNA LQSGNSQESVTEQDSKD STYSLSSTLTLSKADYEK HKVYACEVTHQGLSSPV TKSFNRGEC | 114 |
| PR302927 (IgG1) | EVQLLESGGGLVQP GGSLRLSCAASGFT FSSYAMSWVRQAP GKGLEWVSTINHIG DSTYYADSVKGRFT ISRDNSKNTLYLQM | 115 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLPHFGQGT | 116 |
| | NSLRAEDTAVYYC AKDQWFGVFDYW GQGTLVTVSSASTK GPSVFPLAPSSKSTS GGTAALGCLVKDY FPEPVTVSWNSGAL TSGVHTFPAVLQSS GLYSLSSVVTVPSSS LGTQTYICNVNHKP SNTKVDKKVEPKSC DKTHTCPPCPAPEL LGGPSVFLFPPKPK DTLMISRTPEVTCV VVDVSHEDPEVKFN WYVDGVEVHNAKT KPREEQYNSTYRVV SVLTVLHQDWLNG KEYKCKVSNKALP APIEKTISKAKGQPR EPQVYTLPPSREEM TKNQVSLTCLVKGF YPSDIAVEWESNGQ PENNYKTTPPVLDS DGSFFLYSKLTVDK SRWQQGNVFSCSV MHEALHNHYTQKS LSLSPGK | | KLEIKRTVAAPSVFIFPPS DEQLKSGTASVVCLLNN FYPREAKVQWKVDNAL QSGNSQESVTEQDSKDS TYSLSSTLTLSKADYEK HKVYACEVTHQGLSSPV TKSFNRGEC | |
| PR302934 (IgG1) | QVQLQQWGAGLLK PSETLSLTCAVYGG SFSGYYWSWIRQTL GKGLEWIGEVSHSG GTNYNPSLKSRVTIS VDTSKNQFSLKLSS VTAADTAVYYCAR SPYYGDYMYVFDI WGQGTMVTVSSAS TKGPSVFPLAPSSKS TSGGTAALGCLVK DYFPEPVTVSWNSG ALTSGVHTFPAVLQ SSGLYSLSSVVTVPS SSLGTQTYICNVNH KPSNTKVDKKVEPK SCDKTHTCPPCPAP ELLGGPSVFLFPPKP KDTLMISRTPEVTC VVVDVSHEDPEVKF NWYVDGVEVHNA KTKPREEQYNSTYR VVSVLTVLHQDWL NGKEYKCKVSNKA LPAPIEKTISKAKGQ PREPQVYTLPPSREE MTKNQVSLTCLVK GFYPSDIAVEWESN GQPENNYKTTPPVL DSDGSFFLYSKLTV | 117 | EIVLTQSPGTLSLSPGER ATLSCRASQSVSSSYLA WYQQKPGQAPRLLIYG ASSRATGIPDRFSGSGSG TDFTLTISRLEPEDFAVY YCQQYGSLFTFGGGTKV EIKRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 118 |
| | DKSRWQQGNVFSC SVMHEALHNHYTQ KSLSLSPGK | | | |
| PR304341 (IgG4) | QVQLQQWGAGLLK PSETLSLTCAVYGG SFSGYYWSWIRQTL GKGLEWIGEVSHSG GTNYNPSLKSRVTIS VDTSKNQFSLKLSS VTAADTAVYYCAR SPYYGDYMYVFDI WGQGTMVTVSSAS TKGPSVFPLAPCSRS TSESTAALGCLVKD YFPEPVTVSWNSGA LTSGVHTFPAVLQS SGLYSLSSVVTVPSS SLGTKTYTCNVDH KPSNTKVDKRVESK YGPPCPPCPAPEFLG GPSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH EALHNHYTQKSLSL SLGK | 119 | EIVLTQSPGTLSLSPGER ATLSCRASQSVSSSYLA WYQQKPGQAPRLLIYG ASSRATGIPDRFSGSGSG TDFTLTISRLEPEDFAVY YCQQYGSLFTFGGGTKV EIKRTVAAPSVFIFPPSDE QLKSGTASVVCLLNNFY PREAKVQWKVDNALQS GNSQESVTEQDSKDSTY SLSSTLTLSKADYEKHK VYACEVTHQGLSSPVTK SFNRGEC | 118 |
| PR302949 (IgG1) | EVQLLESGGGLVQP GGSLRLSCAASGFT FSSYAMSWVRQAP GKGLEWVSSISGSD FSTYYADSVKGRFT ISRDTSKNTLYLQM NSLRAEDTAVYYC AKDRQWLVSSFDF WGQGALVTVSSAS TKGPSVFPLAPSSKS TSGGTAALGCLVK DYFPEPVTVSWNSG ALTSGVHTFPAVLQ SSGLYSLSSVVTVPS SSLGTQTYICNVNH KPSNTKVDKKVEPK SCDKTHTCPPCPAP ELLGGPSVFLFPPKP KDTLMISRTPEVTC VVVDVSHEDPEVKF | 120 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD EQLKSGTASVVCLLNNF YPREAKVQWKVDNALQ SGNSQESVTEQDSKDST YSLSSTLTLSKADYEKH KVYACEVTHQGLSSPVT KSFNRGEC | 98 |
| | NWYVDGVEVHNA KTKPREEQYNSTYR VVSVLTVLHQDWL NGKEYKCKVSNKA LPAPIEKTISKAKGQ PREPQVYTLPPSREE MTKNQVSLTCLVK GFYPSDIAVEWESN GQPENNYKTTPPVL DSDGSFFLYSKLTV DKSRWQQGNVFSC SVMHEALHNHYTQ KSLSLSPGK | | | |
| PR302949-1 (IgG4) | EVQLLESGGGLVQP GGSLRLSCAASGFT FSSYAMSWVRQAP GKGLEWVSSISGSD FSTYYADSVKGRFT ISRDTSKNTLYLQM NSLRAEDTAVYYC AKDRQWLVSSFDF WGQGALVTVSSAS TKGPSVFPLAPCSRS TSESTAALGCLVKD YFPEPVTVSWNSGA LTSGVHTFPAVLQS SGLYSLSSVVTVPSS SLGTKTYTCNVDH KPSNTKVDKRVESK YGPPCPPCPAPEFLG GPSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH EALHNHYTQKSLSL SLGK | 121 | DIQMTQSPSSLSASVGD RVTITCQASQDISNYLN WYQQKPGKAPKLLIYD ASNLETGVPSRFSGSGS GTDFTFTISSLQPEDIAT YYCQQYDNLITFGQGTR LEIKRTVAAPSVFIFPPSD EQLKSGTASVVCLLNNF YPREAKVQWKVDNALQ SGNSQESVTEQDSKDST YSLSSTLTLSKADYEKH KVYACEVTHQGLSSPVT KSFNRGEC | 98 |
| PR302950 (IgG1) | QVQLQESGPGLVKP SGTLSLTCAVSGGSI SSSNWWSWVRQPP GKGLEWIGEVYHS GSTNYNPSLKSRVTI SVDKSQNQFSLKLT SVTAADTAVYYCA RVTGNKDYYYYDL DVWGQGTTVTVSS ASTKGPSVFPLAPSS | 122 | DIQMTQSPSSLSASVGD RVTITCQASQDISNYLN WYQQKPGKAPKLLIYD ASNLGTGVPSRFSGGGS GTDFTFTISSLQPEDIAT YYCQQYDNLPRTFGQG TKVEIKRTVAAPSVFIFP PSDEQLKSGTASVVCLL NNFYPREAKVQWKVDN ALQSGNSQESVTEQDSK | 123 |
| | KSTSGGTAALGCLV KDYFPEPVTVSWNS GALTSGVHTFPAVL QSSGLYSLSSVVTV PSSSLGTQTYICNVN HKPSNTKVDKKVEP KSCDKTHTCPPCPA PELLGGPSVFLFPPK PKDTLMISRTPEVT CVVVDVSHEDPEV KFNWYVDGVEVHN AKTKPREEQYNSTY RVVSVLTVLHQDW LNGKEYKCKVSNK ALPAPIEKTISKAKG QPREPQVYTLPPSR EEMTKNQVSLTCLV KGFYPSDIAVEWES NGQPENNYKTTPPV LDSDGSFFLYSKLT VDKSRWQQGNVFS CSVMHEALHNHYT QKSLSLSPGK | | DSTYSLSSTLTLSKADYE KHKVYACEVTHQGLSSP VTKSFNRGEC | |
| PR303413 (IgG1) | QVQLVESGGGVVQ PGRSLRLSCAASGF TFSSYGMHWVRQA PGKGLGWVAGIWY AGSNEYYVDSVKG RFIISRDNSKNTLYL QMNSLRAEDTAVY YCARDQGWGTMD AWGQGASVTVSSA STKGPSVFPLAPSSK STSGGTAALGCLVK DYFPEPVTVSWNSG ALTSGVHTFPAVLQ SSGLYSLSSVVTVPS SSLGTQTYICNVNH KPSNTKVDKKVEPK SCDKTHTCPPCPAP ELLGGPSVFLFPPKP KDTLMISRTPEVTC VVVDVSHEDPEVKF NWYVDGVEVHNA KTKPREEQYNSTYR VVSVLTVLHQDWL NGKEYKCKVSNKA LPAPIEKTISKAKGQ PREPQVYTLPPSREE MTKNQVSLTCLVK GFYPSDIAVEWESN GQPENNYKTTPPVL DSDGSFFLYSKLTV DKSRWQQGNVFSC SVMHEALHNHYTQ KSLSLSPGK | 124 | DIQMTQSPSSLSASVGD RVTITCQASQDISNYLN WYQQKPGKAPKLLIYD ASNLETGVPSRFSGSGS GTDFTFTISSLQPEDIAIY YCQQYDNLLTFGGGTK VEIKRTVAAPSVFIFPPS DEQLKSGTASVVCLLNN FYPREAKVQWKVDNAL QSGNSQESVTEQDSKDS TYSLSSTLTLSKADYEK HKVYACEVTHQGLSSPV TKSFNRGEC | 125 |
| PR303413-1 (IgG4) | QVQLVESGGGVVQ PGRSLRLSCAASGF TFSSYGMHWVRQA PGKGLGWVAGIWY AGSNEYYVDSVKG RFIISRDNSKNTLYL QMNSLRAEDTAVY YCARDQGWGTMD AWGQGASVTVSSA STKGPSVFPLAPCSR STSESTAALGCLVK DYFPEPVTVSWNSG ALTSGVHTFPAVLQ SSGLYSLSSVVTVPS SSLGTKTYTCNVDH KPSNTKVDKRVESK YGPPCPPCPAPEFLG GPSVFLFPPKPKDTL MISRTPEVTCVVVD VSQEDPEVQFNWY VDGVEVHNAKTKP REEQFNSTYRVVSV LTVLHQDWLNGKE YKCKVSNKGLPSSI EKTISKAKGQPREP QVYTLPPSQEEMTK NQVSLTCLVKGFYP SDIAVEWESNGQPE NNYKTTPPVLDSDG SFFLYSRLTVDKSR WQEGNVFSCSVMH EALHNHYTQKSLSL SLGK | 126 | DIQMTQSPSSLSASVGD RVTITCQASQDISNYLN WYQQKPGKAPKLLIYD ASNLETGVPSRFSGSGS GTDFTFTISSLQPEDIAIY YCQQYDNLLTFGGGTK VEIKRTVAAPSVFIFPPS DEQLKSGTASVVCLLNN FYPREAKVQWKVDNAL QSGNSQESVTEQDSKDS TYSLSSTLTLSKADYEK HKVYACEVTHQGLSSPV TKSFNRGEC | 125 |

See Table 5 below for the information on VHs and VLs of control antibodies involved in the present disclosure.

**Table 5**

| Ab ID | VH | SEQ ID NO: | VL | SEQ ID NO: |
|---|---|---|---|---|
| PR001795 | EVQLQQWGAGLLKPSETLSLTC AVYGGSFSGYYWSWIRQPPGKG LEWIGEINHAGSTNYNPSLKSRV TISVDTSKNQFSLKLSSVTAADT AVYYCARLPTRWVTTRYFDLW GRGTLVTVSS | 133 | ESVLTQPPSVSGAPGQRVTIS CTGSSSNIGAGYDVHWYQQL PGTAPKLLIYGDSNRPSGVPD RFSVSKSGASASLAITGLQAE DEADYYCQSFDNSLSAYVFG GGTQLTVL | 134 |
| PR301366 | EVQLLESGGGLVQPGGSLRLSCA ASGFTFSNYAMNWVRQAPGKG LEWVSVISVSGGSTYYADSVKG RFTISRDNSKNTLYLQMNSLRAE DTAVYYCAKDYYYDSGSYYDSF FDYWGQGTLVTVSS | 135 | EIVLTQSPGTLSLSPGERATLS CRASQSVSSSYLAWYQQKPG QAPRLLIYGASSRATGIPDRFS GSGSGTDFTLTISRLEPEDFAV YYCQQYGSSPLTFGGGTKVEI K | 136 |
| PR304683 | QVQLVQSGAEVKKPGASVKVSC KASGYTFTDYYINWVRQAPGQG LEWMGNVNPNDGGTTYNQKFK GRVTMTTDTSTSTAYMELRSLRS | 137 | DIQLTQSPSFLSASVGDRVTIT CRASESVDYYGNSFMYWYQ QKPGKAPKLLIYFASNLESGV PSRFSGSGSGTEFTLTISSLQP | 138 |
| | DDTAVYYCARREIYFYGTIYYY AMDYWGQGTLVTVSS | | EDFATYYCQQNNEDPWTFGG GTKVEIK | |

The present disclosure is further illustrated by the following examples; however, these examples should not be construed as limiting the present disclosure. Experimental methods without specific conditions indicated in the following examples were performed in accordance with conventional methods and conditions or in accordance with product instructions.

### Example 1. Immunization of Harbour Transgenic Mice

1. Immunogen: the extracellular region (positions 1-460) of the recombinant human ILT4 protein amino acid sequence (UniprotKB NO. Q8N423) was cloned into an Fc tag-containing pcDNA3.1 vector to prepare a recombinant expression plasmid. HEK293 cells were subjected to transient transfection and expanded, the cell culture solution was collected after 4 days, and the culture supernatant was purified to obtain the high-purity huILT4-ECD-Fc protein (> 90%) having bioactivity, which was aliquoted and then cryopreserved at -80 °C.
2. Immunization of Harbour H2L2 mice: the huILT4-ECD-Fc protein was used to immunize 6-8 week-old Harbour H2L2 transgenic mice (Harbour Antibodies BV; the mice are transgenic mice carrying an immune repertoire of human immunoglobulins and produce antibodies with intact human antibody variable domains and rat constant domains). All transgenic mice were bred in an SPF-grade environment. In the primary immunization, 50 µg of huILT4-ECD-Fc protein was emulsified with Freund's complete adjuvant (Sigma, F5881) and then intraperitoneally injected into mice. For subsequent booster immunizations, 25 µg of huILT4-ECD-Fc protein was well mixed with RIBI adjuvant (Sigma, S6322) and then intraperitoneally injected into mice. The interval between immunizations was 2 weeks. The blood was taken and determined for the serum antibody titers by ELISA 7 days after the immunization, and the mice with high serum titers were selected for subsequent single B cell screening experiments.

### Example 2. Single B Cell Screening based on Beacon^{®} Optofluidic System

The Beacon^{®} Optofluidic system uses opto-electronic positioning (OEP^{™}) technology for the movement of single cells. The Beacon optoelectronic system is an automatic biological instrument that allows various operations such as biological function tests, experimental analysis, positive clone selection, and the like to be simultaneously performed under the cell culture conditions. The Beacon platform can perform these tasks in a massively parallel and automatic manner for thousands of cells.

In the present application, a plasma cell discovery workflow was used. Up to 14k single plasma cells can be screened per experiment for selecting antibody-secreted antigen-specific plasma cells. Those plasma cells secreting specific antibodies were then individually introduced into a 96-well plate containing a cell lysis buffer for subsequent single B cell sequencing to identify the heavy and light chain sequences of the antibodies produced by the single B cells (monoclones).

### Example 3. Single B Cell Sequencing

In the present disclosure, a single B cell sequencing method was used to recover antibody heavy and light chain sequences from a single plasma cell. Single B cell sequencing has become a powerful tool for obtaining antibody sequences. The general procedure comprises RNA purification from single plasma cell lysate, reverse transcription synthesis of cDNA, amplification and purification of cDNA, amplification of heavy and light chains, cloning and transfection, and Sanger sequencing.

The sequences of the heavy and light chain complementarity determining regions (CDRs), the heavy and light chain variable regions (V), and the heavy and light chains of the human ILT4 antibodies obtained in the present disclosure are shown in Table 6 below.

**Table 6. ILT4 antibody sequences and antibodies**

| | Heavy chain | Light chain | VH | VL | HCD R1 | HCD R2 | HCD R3 | LCD R1 | LCD R2 | LCD R3 | IgG Subtype |
|---|---|---|---|---|---|---|---|---|---|---|---|
| PR302239 | 100 | 101 | 70 | 71 | 18 | 19 | 20 | 21 | 5 | 22 | IgG1 |
| PR302259 | 103 | 104 | 72 | 73 | 23 | 24 | 25 | 15 | 16 | 26 | IgG1 |
| PR302261 | 106 | 107 | 74 | 75 | 23 | 27 | 28 | 29 | 30 | 31 | IgG1 |
| PR302264 | 90 | 91 | 61 | 62 | 1 | 2 | 3 | 4 | 5 | 6 | IgG1 |
| PR302282 | 110 | 98 | 77 | 78 | 32 | 33 | 34 | 15 | 16 | 17 | IgG1 |
| PR302702 | 113 | 114 | 80 | 81 | 36 | 37 | 38 | 15 | 16 | 40 | IgG1 |
| PR302927 | 115 | 116 | 82 | 83 | 23 | 41 | 42 | 15 | 16 | 43 | IgG1 |
| PR302949 | 120 | 98 | 86 | 78 | 23 | 50 | 51 | 15 | 16 | 17 | IgG1 |
| PR302950 | 122 | 123 | 87 | 88 | 52 | 53 | 54 | 15 | 55 | 56 | IgG1 |
| PR302951 | 97 | 98 | 68 | 69 | 12 | 13 | 14 | 15 | 16 | 17 | IgG1 |
| PR303413 | 124 | 125 | 60 | 89 | 32 | 57 | 58 | 15 | 16 | 59 | IgG1 |
| PR302934 | 117 | 118 | 84 | 85 | 44 | 45 | 46 | 47 | 48 | 49 | IgG1 |

### Example 4. Expression and Purification of Recombinant Antibodies

The resulting sequences were subjected to uniqueness and clustering analysis, and then paired heavy and light chain DNA sequences were subjected to plasmid synthesis. CHO cells were used for expression of antibodies. The cell density was adjusted to 1 × 10e6 cells/mL. Plasmids of heavy and light chain antibodies were added to a tube in a ratio of 1:1.5, and then the transfection reagent PEI was added, with the ratio of PEI to DNA being 4:1. The mixture was well mixed and incubated for 15 min at room temperature. The mixture above was added to the cells, and the transfected cells were incubated in an incubator at 37 °C and 5% CO₂ with shaking at 125 rpm. The OPM-CHO PFF05 nutrient (OPM, Cat # FB1279-001) was added 24 h after transfection to a final concentration of 3%. After 5 days of cell culture, the supernatant was collected when the cell viability decreased to 70% or less. The transfected supernatant was subjected to protein purification using a protein G column.

### Example 5. Mutation of Antibody Sequences

### 5.1 Conversion of IgG subtypes

The Fc of the antibody was engineered to convert the antibody type from IgG1 to IgG4, the antibody was expressed and purified as described in Example 4, and the corresponding antibody sequence information is shown in Table 7.

**Table 7. Sequences of IgG4-type ILT4 antibodies**

| Antibody | Parent antibody | Heavy chain | Light chain | VH | VL | HCD R1 | HCD R2 | HCD R3 | LCD R1 | LCD R2 | LCD R3 | IgG Subtype |
|---|---|---|---|---|---|---|---|---|---|---|---|---|
| PR302239-1 | PR302239 | 102 | 101 | 70 | 71 | 18 | 19 | 20 | 21 | 5 | 22 | IgG4 |
| PR302259-1 | PR302259 | 105 | 104 | 72 | 73 | 23 | 24 | 25 | 15 | 16 | 26 | IgG4 |
| PR302949-1 | PR302949 | 121 | 98 | 86 | 78 | 23 | 50 | 51 | 15 | 16 | 17 | IgG4 |
| PR302951-1 | PR302951 | 99 | 98 | 68 | 69 | 12 | 13 | 14 | 15 | 16 | 17 | IgG4 |
| PR303413-1 | PR303413 | 126 | 125 | 60 | 89 | 32 | 57 | 58 | 15 | 16 | 59 | IgG4 |
| PR304341 | PR302934 | 119 | 118 | 84 | 85 | 44 | 45 | 46 | 47 | 48 | 49 | IgG4 |

### 5.2 Sequence analysis and optimization of antibodies

Amino acid mutations were introduced into potential post-translational modification (PTM) sites in antibody sequences to obtain novel antibody molecules (referred to as PTM variants). The amino acid sequences of the light and heavy chain variable domains, light chain, heavy chain (human IgG4), and CDRs of PTM variants based on the Combined scheme in this example are listed in Table 8. All designed PTM variants were purified by the method described in Example 4 to obtain purified recombinant antibodies and further validated in subsequent functional experiments.

**Table 8. Sequences of PTM variants**

| Initial antibody | PTM variant | Heavy chain | Light chain | VH | VL | HCDR1 | HCDR2 | HCDR3 | LCDR1 | LCDR2 | LCDR3 | IgG subtype |
|---|---|---|---|---|---|---|---|---|---|---|---|---|
| PR302261 | PR302261-5 | 108 | 109 | 74 | 76 | 23 | 27 | 28 | 39 | 30 | 31 | IgG4 |
| PR302264 | PR302264-1 | 92 | 91 | 63 | 62 | 1 | 7 | 3 | 4 | 5 | 6 | IgG4 |
| | PR302264-2 | 93 | 91 | 64 | 62 | 1 | 8 | 3 | 4 | 5 | 6 | IgG4 |
| | PR302264-3 | 94 | 91 | 65 | 62 | 1 | 9 | 3 | 4 | 5 | 6 | IgG4 |
| | PR302264-4 | 95 | 91 | 66 | 62 | 1 | 10 | 3 | 4 | 5 | 6 | IgG4 |
| | PR302264-5 | 96 | 91 | 67 | 62 | 1 | 11 | 3 | 4 | 5 | 6 | IgG4 |
| PR302282 | PR302282-1 | 111 | 98 | 77 | 78 | 32 | 33 | 34 | 15 | 16 | 17 | IgG4 |
| | PR302282-2 | 112 | 98 | 79 | 78 | 32 | 35 | 34 | 15 | 16 | 17 | IgG4 |

### Example 6. Antibody Affinity Assay

The binding kinetics of the multiple antibodies prepared according to the methods of Examples 1-5 of the present disclosure to human ILT4-ECD protein were assayed by means of bio-layer interferometry (BLI) (Fortebio octet RED 96e).

The test antibody was diluted to the final concentration of 6 µg/mL and directly immobilized on AHC biosensor for kinetic assay, and the antigen protein (HuILT4, UniprotKB NO. Q8N423) was diluted to 3 concentrations of 100 nM, 50 nM, and 25 nM with 0.02% PBST20 and loaded for 70 s, with binding for 300 s, dissociation for 300 s, and regeneration for 15 s with 10 mM glycine-HCl (pH 1.5). The association rate (kon) and dissociation rate (kdis) were calculated using a simple one-to-one Languir binding model (Octet Red 96 data analysis software), with the equilibrium dissociation constant (kD) calculated as the ratio of kdis/kon. The results of the antibody affinity assays are shown in Table 9 below.

**Table 9. Assay results for antibody affinity**

| Antibody | KD (M) | Kon (1/Ms) | Kdis (1/Ms) |
|---|---|---|---|
| PR302259 | < 1.0E-12 | 7.73E+05 | < 1.0E-07 |
| PR302934 | 4.58E-10 | 6.94E+05 | 3.18E-04 |
| PR302951 | 2.56E-10 | 9.15E+05 | 2.34E-04 |
| PR302261 | 9.84E-10 | 5.20E+05 | 5.12E-04 |
| PR302949 | 4.54E-10 | 9.85E+05 | 4.47E-04 |
| PR302927 | 8.84E-10 | 9.80E+05 | 8.66E-04 |
| PR302282 | 1.24E-10 | 6.48E+05 | 8.02E-05 |
| PR302264 | 2.422E-10 | 4.62E+05 | 1.12E-04 |
| PR302264-2 | 7.674E-11 | 4.72E+05 | 3.62E-05 |
| PR302264-3 | 3.515E-10 | 4.54E+05 | 1.60E-04 |
| PR001795 | 3.521E-09 | 4.31E+05 | 1.52E-03 |

As can be seen from the results in the above table, the affinity of antibodies PR302259, PR302934, PR302951, PR302261, PR302949, PR302927, PR302282, PR302264, PR302264-2, and PR302264-3 was better than that of the control antibody PR001795 (Merck: US20180298096).

### Example 7. Assay on Binding Capacity of Antibodies for ILT4-ECD at Protein Level

The binding capacity of the multiple antibodies prepared in Examples 1-5 of the present disclosure for ILT4-ECD protein was assayed based on ELISA. The binding capacity of the different antibodies was determined by comparing their binding curves for the ILT4-ECD protein. The specific experimental process was as follows: human ILT4-ECD and cynomolgus monkey ILT4-ECD proteins were diluted sequentially to a concentration of 1 µg/mL, added to a 96-well plate at 100 µL per well, and left to stand overnight at 4 °C. After the 96-well plate was washed three times with PBST solution, PBS containing 2% BSA was added, and the mixture was left to stand at 37 °C for 1 h. The test antibodies were diluted in a gradient (100 nM, 10-fold-dilution) and added to the 96-well plate, and the mixture was incubated at 37 °C for 1 h. After the plate was washed three times with PBST solution, an anti-human IgG Fc-HRP secondary antibody (5000× dilution) was added, and the mixture was incubated at 37 °C for 30-60 min. After the plate was washed three times with PBST solution, the TMB chromogenic solution was added for 5-15 min of color development, and then a stop buffer was added to stop the color development.

**Table 10. Results of assay on binding capacity of antibodies for ILT4-ECD at protein level**

| | | | | | | |
|---|---|---|---|---|---|---|
| Human ILT4-ECD | Antibody | PR302702 | PR302927 | PR302934 | PR302949 | PR302950 |
| | EC₅₀ | 0.029 | 0.025 | 0.012 | 0.023 | 0.056 |
| | Antibody | PR302261 | PR302239-1 | PR302282 | PR302264 | PR303413 |
| | EC₅₀ | 0.032 | 0.036 | 0.015 | 0.026 | 0.021 |
| | Antibody | PR302951 | PR302259-1 | PR301366 | Isotype control | |
| | EC₅₀ | 0.015 | 0.012 | 0.014 | / | |
| Monkey ILT4-ECD | Antibody | PR302934 | PR302264 | PR302261 | PR301366 | Isotype control |
| | EC₅₀ | 0.047 | 0.935 | 0.008 | 0.401 | / |

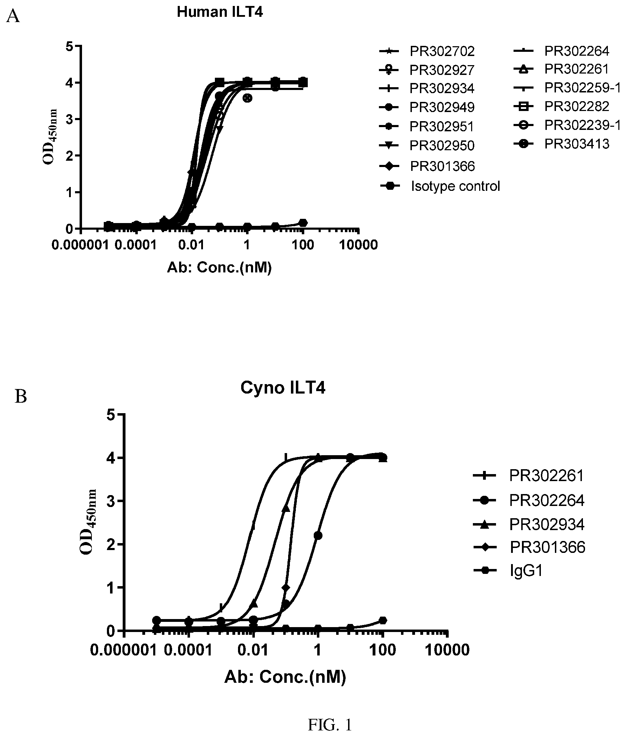

As can be seen from the assay results in Table 10 and FIG. 1, antibodies PR302702, PR302927, PR302934, PR302949, PR302950, PR302951, PR302264, PR302261, PR302282, PR302239-1, PR302259-1, and PR303413 were all able to bind to human ILT4-ECD protein and were comparable to the control antibody PR301366 (Five Prime: WO2020014132). Among these antibodies, antibodies PR302934, PR302261, and PR302264 were also able to bind to monkey ILT4-ECD protein.

### Example 8. Assay on Binding Capacity of Antibodies for ILT4 at Cellular Level

The binding capacity of multiple antibodies prepared in Examples 1-5 of the present disclosure for human ILT4 molecule expressed on the surface of 293T cells was assayed based on flow cytometry. The binding capacity of the different antibodies was determined by comparing their binding curves for human ILT4 molecule expressed on the surface of CHO cells. The specific experimental process was as follows. (1) CHO cells were infected with the lentivirus lenti-huILT4, and infected cells were screened with puromycin to obtain stable cell pool, CHO-huILT4. (2) The stable cell pool was subjected to subclone screening to obtain a monoclonal cell line with high expression of target gene. (3) Test antibodies at different concentrations were each incubated with the monoclonal cell line at 4 °C for 30 min, the mixture was then washed three times with PBS, incubated with an FITC-labeled goat anti-human secondary antibody at 4 °C for 30 min, and then the resulting mixture was washed three times with PBS again and the cells were resuspended with 100 µL of FACS buffer. (4) The median fluorescence intensity in one of the channels was determined using a flow cytometer. The EC₅₀ values were compared by using the base-10 logs of the antibody concentrations as the abscissa and the median fluorescence intensity in one channel as the ordinate.

**Table 11. Results of assay on binding capacity of antibodies for ILT4 at cellular level**

| | | | | | |
|---|---|---|---|---|---|
| CHO-ILT4 | Antibody | PR302702 | PR302927 | PR302934 | PR302949 |
| | EC₅₀ (nM) | 1.580 | 0.805 | 1.130 | 0.822 |
| | Antibody | PR302951 | PR302950 | PR301366 | Isotype control |
| | EC₅₀ (nM) | 1.049 | 1.093 | 1.067 | / |
| CHO-ILT4 | Antibody | PR302239-1 | PR302282 | PR303413 | |
| | EC₅₀ | 0.931 | 1.881 | 0.345 | |
| | Antibody | PR302259-1 | PR301366 | Isotype control | |
| | EC₅₀ | 0.262 | 0.362 | / | |

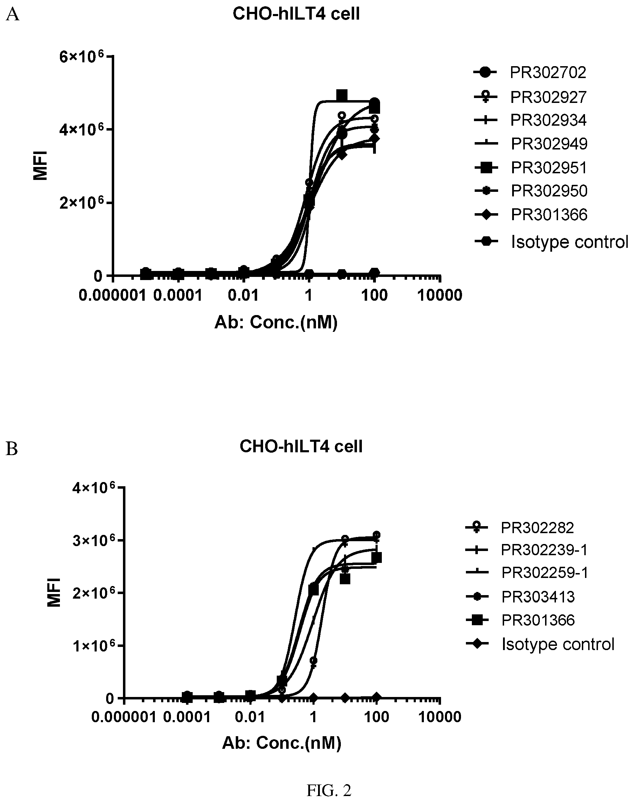

As can be seen from the assay results in Table 11 and FIG. 2, antibodies PR302702, PR302927, PR302934, PR302949, PR302951, PR302950, PR302239-1, PR302282, PR303413, and PR302259-1 all showed strong binding capacity for CHO-huILT4 cells.

### Example 9. Assay on Binding Capacity of Antibodies for ILT2-ECD at Protein Level

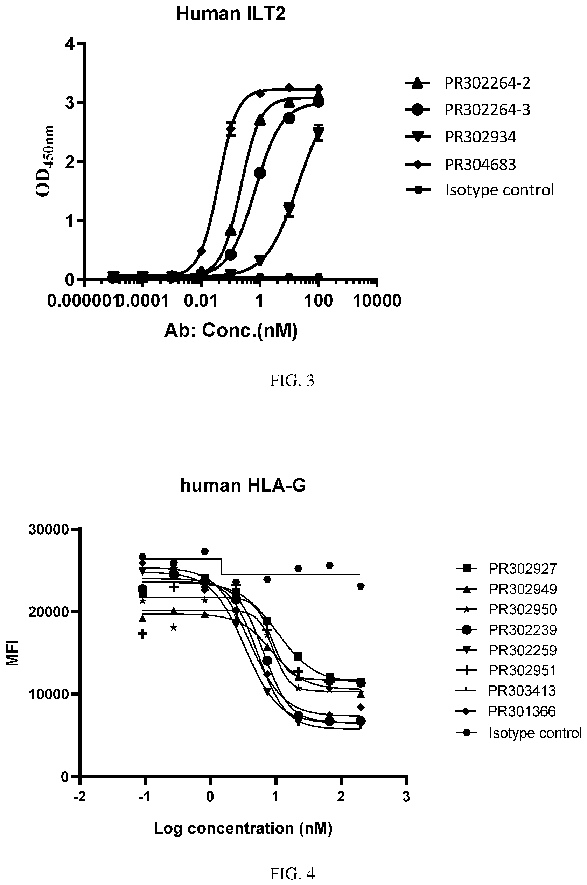

The binding capacity of the multiple antibodies prepared in Examples 1-5 of the present disclosure for ILT2-ECD protein (UniprotKB NO. Q8NHL6) was assayed based on ELISA. The binding capacity of the different antibodies was determined by comparing their binding curves for the ILT2-ECD protein. The specific experimental process was as follows: human ILT2-ECD protein (UniprotKB NO. Q8NHL6) was diluted sequentially to a concentration of 1 µg/mL, added to a 96-well plate at 100 µL per well, and left to stand overnight at 4 °C. After the 96-well plate was washed three times with PBST solution, PBS containing 2% BSA was added, and the mixture was left to stand at 37 °C for 1 h. The test antibodies were diluted in a gradient (100 nM, 10-fold-dilution) and added to the 96-well plate, and the mixture was incubated at 37 °C for 1 h. After the plate was washed three times with PBST solution, an anti-human IgG Fc-HRP secondary antibody (5000× dilution) was added, and the mixture was incubated at 37 °C for 30-60 min. After the plate was washed three times with PBST solution, the TMB chromogenic solution was added for 5-15 min of color development, and then a stop buffer was added to stop the color development. As can be seen from the assay results in FIG. 3, antibodies PR302264-2, PR302264-3, and PR302934 had strong binding capacity for Human ILT2.

### Example 10. Assay on Capacity of Antibodies to Block Binding of ILT4 to HLA-G

The capacity of multiple antibodies prepared in Examples 1-5 of the present disclosure to block the binding of huILT4 protein to human HLA-G-β2M (HLA-G: UniprotKB NO. P17693; β2M: UniprotKB NO. P61769) expressed on the surface of CHO cells was assayed based on flow cytometry. The blocking capacity of the different antibodies was determined by comparing their curves in blocking the binding of huILT4 protein to CHO-HLA-G-β2M cells. The specific experimental process was as follows. (1) Firstly, HLA-G-β2M was constructed into PEE6.4 plasmid, the plasmid was then transfected into CHO cells, and the infected cells were screened with puromycin to obtain a stable cell pool, CHO-HLA-G-β2M. (2) The stable cell pool was subjected to subclone screening to obtain a monoclonal cell line with high expression of target gene. (3) The selected cell line CHO-HLA-G-β2M was transfected with OS8-containing PEE6.4 plasmid and screened with hygromycin B to obtain the CHO-HLA-G-β2M-OS8 cell pool. (4) The cell pool was subjected to subclone screening to obtain a monoclonal cell line with high expression of target gene. (5) The EC₈₀ concentration for the binding of huILT4 protein to CHO-HLA-G-β2M cells was determined. The huILT4 protein was subjected to biotin labeling to obtain huILT4-biotin protein. (6) The test antibodies at different concentrations and huILT4 protein (final concentration was the value of EC₈₀) were incubated with CHO-HLA-G-β2M cells at 4 °C for 1 h, the mixture was then washed three times with PBS, incubated with APC-labeled Strepavidin secondary antibody at 4 °C for 30 min, and then the resulting mixture was washed three times with PBS again and the cells were resuspended with 100 µL of FACS buffer. (7) The median fluorescence intensity of four channels was determined using flow cytometry. The IC₅₀ values were compared by plotting the base-10 logs of the antibody concentrations on the abscissa and the median fluorescence intensity on four channels as the ordinate.

**Table 12. Results of assay on capacity of antibodies to block binding of ILT4 to HLA-G**

| | | | | | |
|---|---|---|---|---|---|
| Antibody | PR302927 | PR302949 | PR302950 | PR302951 | |
| IC₅₀ (nM) | 10.640 | 8.863 | 9.337 | 8.604 | |
| Antibody | PR302239 | PR302259 | PR303413 | PR301366 | Isotype control |
| IC₅₀ (nM) | 6.493 | 3.397 | 5.329 | 3.992 | / |

As can be seen from the assay results in Table 12 and FIG. 4, compared with the negative control Isotype control, antibodies PR302927, PR302949, PR302950, PR302951, PR302239, PR302259, and PR303413 all had the function of significantly blocking the binding of huILT4 to CHO-HLA-G-β2M.

### Example 11. Assay on Capacity of Antibodies to Block Binding of ILT4 to SEMA4A

The capacity of multiple antibodies prepared in Examples 1-5 of the present disclosure to block the binding of huILT4 protein to human SEMA4A (UniprotKB NO. Q9H3S1) protein was assayed based on ELISA. The blocking capacity of the different antibodies was determined by comparing their curves in blocking the binding of huILT4 to human SEMA4A protein. The specific experimental process was as follows: (1) Firstly, the EC₈₀ concentration for the binding of huILT4 protein to human SEMA4A protein was determined. (2) Human SEMA4A protein was diluted to a concentration of 10 µg/mL, added to a 96-well plate at 100 µL per well, and then left to stand at 4 °C overnight. After the 96-well plate was washed three times with PBST solution, PBS containing 2% BSA was added, and the mixture was left to stand at 37 °C for 1 h. The test antibodies were diluted in a gradient (2000 nM, 3-fold-dilution), and then 50 µL of each of the diluted antibodies and 50 µL of huILT4-biotin protein at a concentration of 2× EC₈₀ were added to the 96-well plate and well mixed. The resulting mixture was incubated at 37 °C for 1 h. After the plate was washed three times with PBST solution, a Strepavidin-HRP secondary antibody (5000× diluted) was added, and the mixture was incubated at 37 °C for 30-60 min. After the plate was washed three times with PBST solution, the TMB chromogenic solution was added for 5-15 min of color development, and then a stop buffer was added to stop the color development.

**Table 13. Results of assay on capacity of antibodies to block binding of ILT4 to SEMA4A**

| Antibody | PR302259 | PR303413 | PR302949 | PR302951 | |
|---|---|---|---|---|---|
| IC₅₀ (nM) | 34.09 | 40.87 | 47.88 | 52.92 | |
| Antibody | PR302282 | PR302950 | PR302927 | PR301366 | Isotype control |
| IC₅₀ (nM) | 48.15 | 65.03 | 61.45 | 34.77 | / |

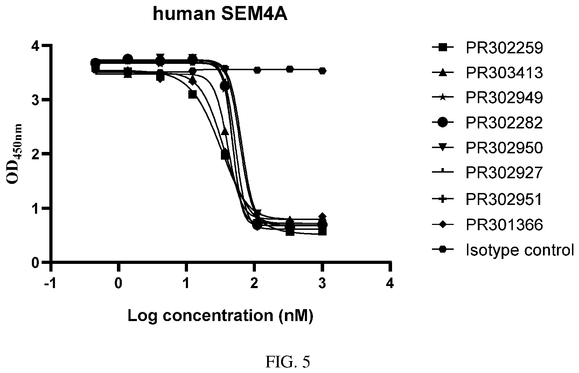

As can be seen from the assay results in Table 13 and FIG. 5, compared with the negative control Isotype control, antibodies PR302259, PR303413, PR302949, PR302951, PR302282, PR302950, and PR302927 all had the function of significantly blocking the binding of huILT4 to SEMA4A.

### Example 12. Cross-Reactivity of Antibodies with Other Family Proteins of ILT4

100 µL of each of human LILRA1 (R&D System, Cat: 9226-T4-050), LILRA2 (R&D System, Cat: 9040-T4-050), LILRA3 (R&D System, Cat: 9517-T4-100), LILRA4 (R&D System, Cat: 8914-T4-050), LILRA5 (R&D System, Cat: 8956-T4-100), LILRA6 (R&D System, Cat:9088-T4-050), LILRB3 (R&D System, Cat: 9159-T5-050), LILRB4 (R&D System, Cat: 8488-T4-025), and LILRB5 (R&D System, Cat: 8478-T4-025) proteins at a concentration of 1 µg/mL was used for ELISA coating overnight at 4 °C. The plate was washed 4 times with PBST and blocked with 200 µL of a solution containing PBST (PBS + 0.05% tween 20) + 2% BSA at 37 °C for 1 h. The plate was washed 4 times with PBST. The ILT4 antibody and positive control antibodies for each protein (LILRA1 Antibody: R&D System, Cat: MAB30851; LILRA2 Antibody: R&D System, Cat: MAB6364; LILRA3 Antibody: R&D System, Cat: MAB2574; LILRA4 Antibody: R&D System, Cat: MAB6287; LILRA5 Antibody: R&D System, Cat: MAB6754; LILRA6 Antibody: R&D System, Cat: MAB8656; LILRB3 Antibody: R&D System, Cat: MAB1806-100; LILRB4 Antibody: R&D System, Cat: MAB24251; LILRB5 Antibody: R&D System, Cat: MAB3065) at a concentration of 100 nM were added to the ELISA plate at 100 µL per well. The plate was incubated at 37 °C for 1 h. The plate was washed 4 times with PBST. 100 µL of Goat Anti-Human IgG, (Fab)-HRP (1:5000, Jackson, Cat: 109-035-098) was added, and the mixture was incubated at 37 °C for 1 h. The plate was washed 4 times with PBST. 100 µL of TMB substrate (Biopanda, Cat: TMB-S-003) was added, and the mixture was incubated at room temperature for 5 min, and then 50 µL of stop buffer (Solarbio, Cat#: C1058) was added to stop the reaction. OD_{450 nm} readings were recorded using a microplate reader (Molecular Devices, Spectra max 384 plus). Control antibody PR304683 (NGM: WO2021127200)

**Table 14. Results of cross-reactivity of antibodies with other family proteins of ILT4**

| Abs | LILRA 1 (LIR6) | LILRA 2 (ILT1) | LILRA 3 (LIR4) | LILRA 4 (ILT7) | LILRA 5 (ILT11 ) | LILRA 6 (ILT8) | LILRB 1 (ILT2) | LILRB 2 (ILT4) | LILRB 3 (ILT5) | LILRB 4 (ILT3) | LILRB 5 (LIR8) |
|---|---|---|---|---|---|---|---|---|---|---|---|
| PR302934 | + | - | - | + | - | + | + | + | + | - | - |
| PR302264-2 | + | - | - | - | - | - | + | + | - | - | - |
| PR302259 | - | - | - | - | - | - | - | + | - | - | - |
| PR302951 | - | - | - | - | - | - | - | + | - | - | - |
| PR302282 | - | - | | - | - | - | - | + | - | - | - |
| PR302927 | - | - | - | - | - | - | - | + | - | - | - |
| PR302949 | - | - | - | - | - | - | - | + | - | - | - |
| PR302239 | - | - | - | - | - | - | - | + | - | - | - |
| PR001795 | - | - | - | - | - | - | - | + | - | - | - |
| PR301366 | - | - | - | - | - | - | - | + | - | - | - |
| PR304683 | + | - | + | - | - | - | + | + | - | - | - |
| Positive control | + | + | + | + | + | + | + | + | + | + | + |
| Negative control | - | - | - | - | - | - | - | - | - | - | - |

As can be seen from the results in Table 14, in addition to binding to ILT4, antibody PR302934 has cross-binding to other family members of LILR, such as LIR6, ILT7, ILT8, ILT2, and ILT5, PR302264-2 had cross-binding capacity for LIR6 and ILT2, and antibodies PR302259, PR302951, PR302282, PR302927, PR302949, and PR302939 specifically bound to ILT4 only.

### Example 13. Assay on Activation of Macrophages by Antibodies-Secretion of Cytokine TNF-α

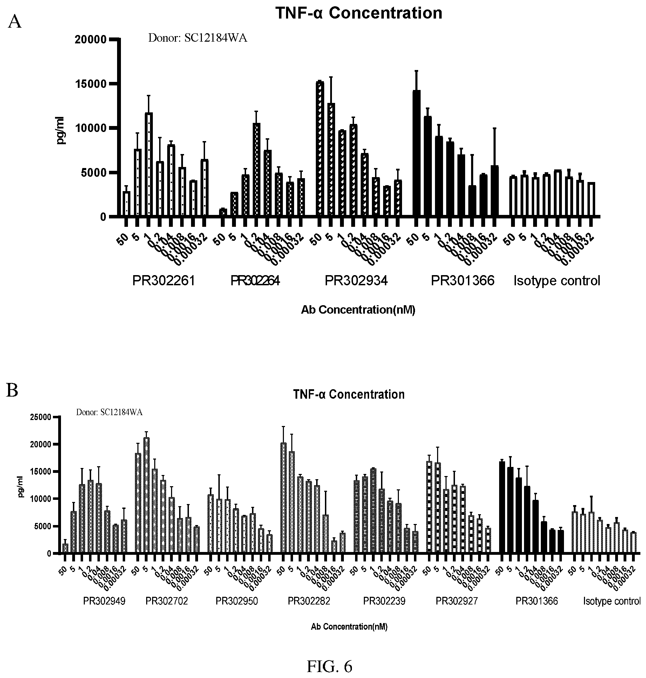

CD14-positive cells were isolated from human PBMCs (Shanghai Saily Biotechnology Co., Ltd., XFB-HP050A) using a human CD14 cell sorting kit (Miltenyi, 130-050-021). The isolated CD14-positive cells were resuspended and diluted to 8 × 10⁵ cells/mL using the 1640 complete medium, and the cell suspension was added to a 96-well cell culture plate at 100 µL per well; 50 µL of the antibody diluted in a gradient (5-fold dilution, start from a final concentration of 50 nM) was added to the 96-well cell plate; MCSF (R&D System, 216-MC-025/CF) was diluted to 400 ng/mL and added at 50 µL per well. The cells were incubated in an incubator at 37 °C and CO₂, and the medium was changed on the third day while keeping the concentrations of the antibody and MCSF constant. On the sixth day, the medium was changed again while keeping the concentrations of the antibody and MCSF constant, LPS at a final concentration of 50 ng/mL was added, and after 24 h, the cell supernatant was collected and the content of TNF-α was assayed using an ELISA kit (Ebioscience, 88-7346-88). As can be seen from the results in FIG. 6, antibodies PR302261, PR302264, PR302934, PR302949, PR302702, PR302950, PR302282, PR302239, and PR302927 all had a strong activation effect on macrophages.

### Example 14. Assay on Effect of Antibodies on Peripheral Blood Mononuclear Cells-Secretion of Cytokine TNF-α

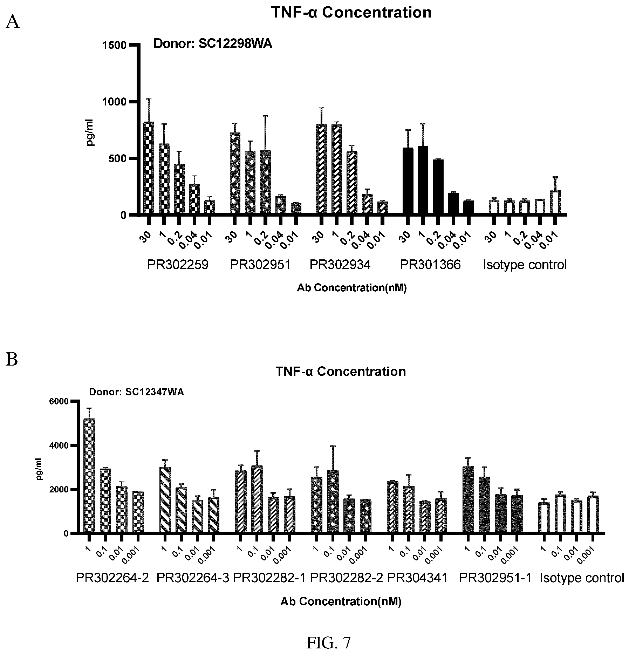

Healthy human PBMCs were resuspended and diluted to 2 × 10⁶ cells/mL using the 1640 complete medium, and the cell suspension was added to a 96-well cell culture plate at 100 µL per well; 50 µL of the antibody diluted in a gradient (diluted from the highest concentration of 30 nM to 1 nM, followed by 5-fold dilution) was added to the 96-well cell plate; LPS was diluted to 400 ng/mL and added at 50 µL per well. After the cells were incubated in an incubator at 37 °C and CO₂ for three days, the cell supernatant was collected and the content of TNF-α was assayed using an ELISA kit. As can be seen from the results in FIG. 7, antibodies PR302259, PR302951, PR302934, PR302264-2, PR302264-3, PR302282-1, PR302282-2, PR304341, and PR302951-1 all had a strong activation effect on PBMCs.

### Example 15. Assay on Effect of Antibodies in Mixed Lymphocyte Reaction-Secretion of Cytokine IFN-y

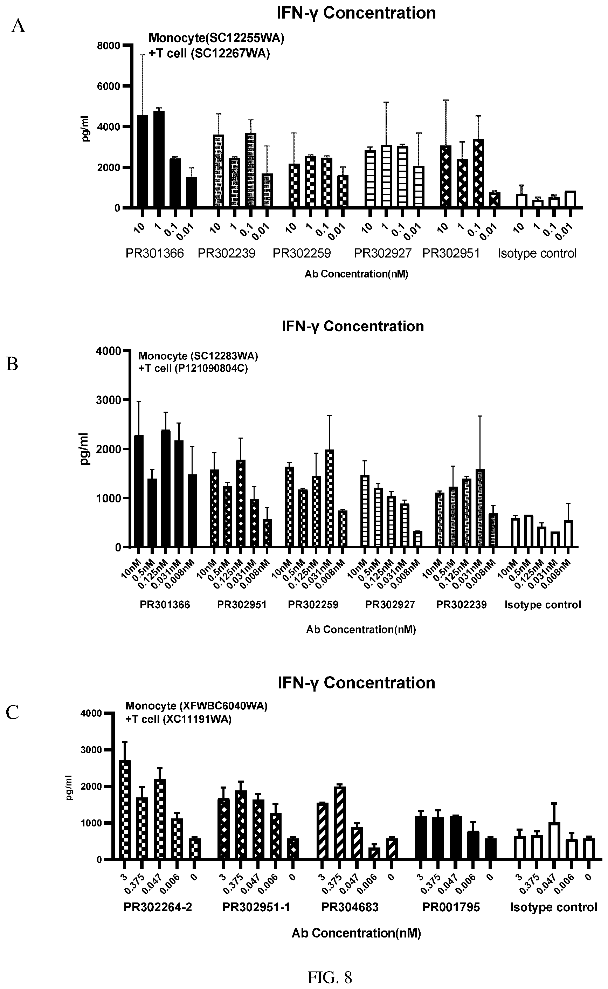

CD14-positive cells were isolated from human PBMCs using a human CD14 cell sorting kit (Miltenyi, 130-050-021). The isolated CD14-positive cells were resuspended and diluted to 1.5 × 10⁵ cells/mL using the 1640 complete medium, and the cell suspension was added to a 96-well cell culture plate at 100 µL per well; 50 µL of the antibody diluted in a gradient (5-fold dilution, start from a final concentration of 50 nM) was added to the 96-well cell plate; MSCF (R&D System, 216-MC-025/CF) was diluted to 400 ng/mL and added at 50 µL per well. The cells were incubated in an incubator at 37 °C and CO₂, and the medium was changed on the third day while keeping the concentrations of the antibody and MCSF constant. On the sixth day, the medium was changed again while keeping the concentrations of the antibody and MCSF constant, and LPS at a final concentration of 50 ng/mL was added. On the seventh day, CD3-positive cells were sorted out from PBMCs from another person using a human CD3 sorting kit (Miltenyi, 130-045-501), and the isolated CD3-positive cells were resuspended and diluted to 1.5 × 10⁶ cells/mL using the 1640 complete medium. The supernatant was removed from the 96-well cell culture plate, then 100 µL of the CD3-positive cell suspension and 50 µL of diluted antibody were added, and then medium was added to allow the volume to be 200 µL. After co-incubation for 72 h, the cell supernatant was collected, and the content of IFN-γ was assayed using an ELISA kit. As can be seen from the results in FIG. 8, antibodies PR302239, PR302259, PR302927, PR302951, PR302951-1, and PR302264-2 exhibited excellent activation effect in the mixed lymphocyte reaction experiment.

### Example 16. Pharmacodynamic Experiment of Antibodies In Vivo

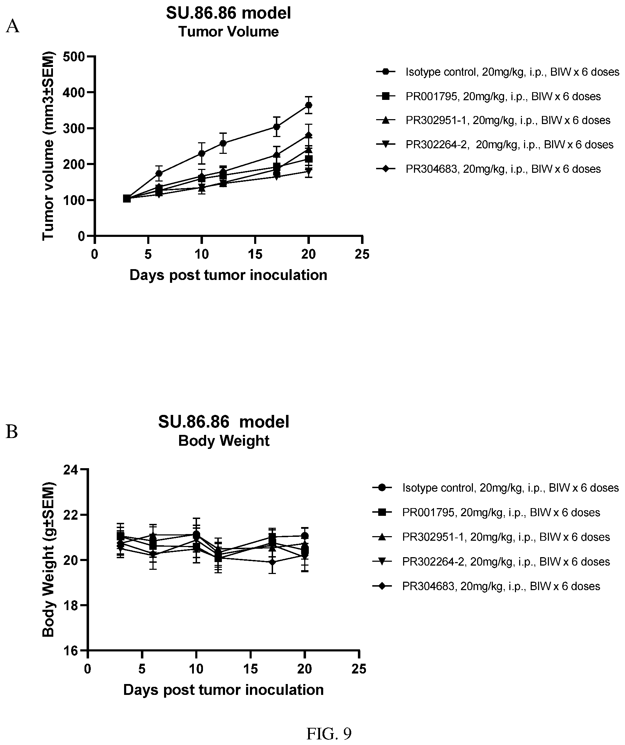

In this experiment, the anti-tumor activity of the anti-human ILT4 antibodies was studied using an SU8686 mouse tumor model. The pharmacodynamics of antibodies PR302951-1 and PR302264-2 administered alone (20 mg/kg) in the SU8686 mouse model was studied. Human pancreatic cancer SU8686 cells (ATCC) were subcutaneously inoculated into female hHSC-NOG-EXL humanized mice (Vital River). The treatment for the antibody groups was as follows: after the tumor cell inoculation, when the tumor volume reached 100 mm³, the first administration was performed, and on the following days 3, 7, 10, 14, and 17, mice were intraperitoneally injected with the corresponding antibodies and the tumor volume was measured at the same time, after which the mice were euthanized. As can be seen from the results in FIG. 9, compared with negative control, the anti-human ILT4 antibodies PR302951-1 and PR302264-2 of the present application exhibited a significant effect in inhibiting tumor growth, wherein the tumor inhibiting effect of antibody PR302264-2 was better than that of the control antibodies PR001795 (Merck: US20180298096) and PR304683 (NGM: WO2021127200).

Other sequences involved in the examples are shown in Table 15 below:

**Table 15**

| Name | Sequence |
|---|---|
| HumanILT4 (UniprotKB NO. Q8N423) | **(SEQ ID NO: 127)** |
| | (SEQ ID NO: 127) MTPIVTVLICLGLSLGPRTHVQTGTIPKPTLWAEPDSVITQGSPVTLSCQGSLEAQ EYRLYREKKSASWITRIRPELVKNGQFHIPSITWEHTGRYGCQYYSRARWSELS DPLVLVMTGAYPKPTLSAQPSPVVTSGGRVTLQCESQVAFGGFILCKEGEEEHP QCLNSQPHARGSSRAIFSVGPVSPNRRWSHRCYGYDLNSPYVWSSPSDLLELLV PGVSKKPSLSVQPGPVVAPGESLTLQCVSDVGYDRFVLYKEGERDLRQLPGRQP QAGLSQANFTLGPVSRSYGGQYRCYGAHNLSSECSAPSDPLDILITGQIRGTPFIS VQPGPTVASGENVTLLCQSWRQFHTFLLTKAGAADAPLRLRSIHEYPKYQAEFP MSPVTSAHAGTYRCYGSLNSDPYLLSHPSEPLELVVSGPSMGSSPPPTGPISTPAG PEDQPLTPTGSDPQSGLGRHLGVVIGILVAVVLLLLLLLLLFLILRHRRQGKHWT STQRKADFQHPAGAVGPEPTDRGLQWRSSPAADAQEENLYAAVKDTQPEDGV EMDTRAAASEAPQDVTYAQLHSLTLRRKATEPPPSQEREPPAEPSIYATLAIH |
| CynoILT4 | **(SEQ ID NO: 128)** |
| | MTPILTILICLGLSLGSRTHVQAGTLPKPTLWAEPGSMISEGSPVTLRCQGNLQV QDYRLQREKKPASWVRQIRQELVKKGYFAIASITWEHTGRYRCQYYSHSCWSE PSDPLELVVIGAYSKPTLSALPSPVVASGGNVTLQCDSQVAFDGFILCKEGEDEH PQRLNSQSHVHGWSWAVFSVGPVSPSHRWSYRCYGYISSAPYVWSLPSDLLEL LVPGVSKKPSLSVQPGPVVARGDKLTLQCGSGAGYDRFALYKEGERDFLQRPG QQLQAGLSQANFLLGPVRGSHGGQYRCYGAHNLSPEWSAPSDPLDILISRQIRG TPFLSVQPGPKVVSGENVTLLCQSSWQFHAFLLTQAGAADAHLHLRSMHKYPK YQAEFPISPVTSAHAGTYRCYGSRSSNPYLLSVPSDPLELVVSGPSGGPSSPTTGP TSPTTGPTSTCAGPEDQPLTPTGSDSQSGLGRHLGVVTGILVAFVLLLFLLLLLFL VLRHRRQGKRWTSAQRKADFQHPAGAVEPEPRDRGLQRRSSPAANTQEENLY AAVKDTQPEDGVELDSRAAASEDPQDVTYAQLQSLTLRRETTEPPPSQERAPPV ESSIYA TLTIH |
| HumanILT2 (UniprotKB NO. Q8NHL6) | **(SEQ ID NO: 129)** |
| | MTPILTVLICLGLSLGPRTHVQAGHLPKPTLWAEPGSVITQGSPVTLRCQGGQET QEYRLYREKKTALWITRIPQELVKKGQFPIPSITWEHAGRYRCYYGSDTAGRSES SDPLELVVTGAYIKPTLSAQPSPVVNSGGNVILQCDSQVAFDGFSLCKEGEDEHP QCLNSQPHARGSSRAIFSVGPVSPSRRWWYRCYAYDSNSPYEWSLPSDLLELLV LGVSKKPSLSVQPGPIVAPEETLTLQCGSDAGYNRFVLYKDGERDFLQLAGAQP QAGLSQANFTLGPVSRSYGGQYRCYGAHNLSSEWSAPSDPLDILIAGQFYDRVS LSVQPGPTVASGENVTLLCQSQGWMQTFLLTKEGAADDPWRLRSTYQSQKYQ AEFPMGPVTSAHAGTYRCYGSQSSKPYLLTHPSDPLELVVSGPSGGPSSPTTGPT STSGPEDQPLTPTGSDPQSGLGRHLGVVIGILVAVILLLLLLLLLFLILRHRRQGK HWTSTQRKADFQHPAGAVGPEPTDRGLQWRSSPAADAQEENLYAAVKHTQPE DGVEMDTRSPHDEDPQAVTYAEVKHSRPRREMASPPSPLSGEFLDTKDRQAEE DRQMDTEAAASEAPQDVTYAQLHSLTLRREATEPPPSQEGPSPAVPSIYATLAIH |
| HumanSEMA 4A ( UniprotKB NO. Q9H3S1 ) | **(SEQ ID NO: 130)** |
| | MALPALGLDPWSLLGLFLFQLLQLLLPTTTAGGGGQGPMPRVRYYAGDERRAL SFFHQKGLQDFDTLLLSGDGNTLYVGAREAILALDIQDPGVPRLKNMIPWPASD RKKSECAFKKKSNETQCFNFIRVLVSYNVTHLYTCGTFAFSPACTFIELQDSYLL PISEDKVMEGKGQSPFDPAHKHTAVLVDGMLYSGTMNNFLGSEPILMRTLGSQP VLKTDNFLRWLHHDASFVAAIPSTQVVYFFFEETASEFDFFERLHTSRVARVCK NDVGGEKLLQKKWTTFLKAQLLCTQPGQLPFNVIRHAVLLPADSPTAPHIYAVF TSQWQVGGTRSSAVCAFSLLDIERVFKGKYKELNKETSRWTTYRGPETNPRPGS CSVGPSSDKALTFMKDHFLMDEQVVGTPLLVKSGVEYTRLAVETAQGLDGHSH LVMYLGTTTGSLHKAVVSGDSSAHLVEEIQLFPDPEPVRNLQLAPTQGAVFVGF SGGVWRVPRANCSVYESCVDCVLARDPHCAWDPESRTCCLLSAPNLNSWKQD MERGNPEWACASGPMSRSLRPQSRPQIIKEVLAVPNSILELPCPHLSALASYYWS HGPAAVPEASSTVYNGSLLLIVQDGVGGLYQCWATENGFSYPVISYWVDSQDQ TLALDPELAGIPREHVKVPLTRVSGGAALAAQQSYWPHFVTVTVLFALVLSGAL IILVASPLRALRARGKVQGCETLRPGEKAPLSREQHLQSPKECRTSASDVDADN NCLGTEVA |
| HLA-G (UniprotKB NO. P17693) | **(SEQ ID NO: 131)** |
| | MVVMAPRTLFLLLSGALTLTETWAGSHSMRYFSAAVSRPGRGEPRFIAMGYVD DTQFVRFDSDSACPRMEPRAPWVEQEGPEYWEEETRNTKAHAQTDRMNLQTL RGYYNQSEASSHTLQWMIGCDLGSDGRLLRGYEQYAYDGKDYLALNEDLRSW TAADTAAQISKRKCEAANVAEQRRAYLEGTCVEWLHRYLENGKEMLQRADPP KTHVTHHPVFDYEATLRCWALGFYPAEIILTWQRDGEDQTQDVELVETRPAGD GTFQKWAAVVVPSGEEQRYTCHVQHEGLPEPLMLRWKQSSLPTIPIMGIVAGLV VLAAVVTGAAVAAVLWRKKSSD |
| β2m ( UniprotKB NO. P61769) | **(SEQ ID NO: 132)** |
| | MSRSVALAVLALLSLSGLEAIQRTPKIQVYSRHPAENGKSNFLNCYVSGFHPSDI EVDLLKNGERIEKVEHSDLSFSKDWSFYLLYYTEFTPTEKDEYACRVNHVTLSQ PKIVKWDRDM |

## Claims

1. An anti-ILT4 antibody, comprising a heavy chain variable region and a light chain variable region, wherein the heavy chain variable region comprises HCDR1, HCDR2, and HCDR3, wherein the HCDR1 comprises an amino acid sequence set forth in SEQ ID NO: 1, SEQ ID NO: 12, SEQ ID NO: 18, SEQ ID NO: 23, SEQ ID NO: 32, SEQ ID NO: 36, SEQ ID NO: 44, or SEQ ID NO: 52, the HCDR2 comprises an amino acid sequence set forth in SEQ ID NO: 8, SEQ ID NO: 13, SEQ ID NO: 2, SEQ ID NO: 7, SEQ ID NO: 9, SEQ ID NO: 10, SEQ ID NO: 11, SEQ ID NO: 19, SEQ ID NO: 24, SEQ ID NO: 27, SEQ ID NO: 33, SEQ ID NO: 35, SEQ ID NO: 37, SEQ ID NO: 41, SEQ ID NO: 45, SEQ ID NO: 50, SEQ ID NO: 53, or SEQ ID NO: 57, or the HCDR3 comprises an amino acid sequence set forth in SEQ ID NO: 3, SEQ ID NO: 14, SEQ ID NO: 20, SEQ ID NO: 25, SEQ ID NO: 28, SEQ ID NO: 34, SEQ ID NO: 38, SEQ ID NO: 42, SEQ ID NO: 46, SEQ ID NO: 51, SEQ ID NO: 54, or SEQ ID NO: 58;
the light chain variable region comprises LCDR1, LCDR2, and LCDR3, wherein the LCDR1 comprises an amino acid sequence set forth in SEQ ID NO: 4, SEQ ID NO: 15, SEQ ID NO: 21, SEQ ID NO: 29, SEQ ID NO: 39, or SEQ ID NO: 47, the LCDR2 comprises an amino acid sequence set forth in SEQ ID NO: 5, SEQ ID NO: 16, SEQ ID NO: 30, SEQ ID NO: 48, or SEQ ID NO: 55, and the LCDR3 comprises an amino acid sequence set forth in SEQ ID NO: 6, SEQ ID NO: 17, SEQ ID NO: 22, SEQ ID NO: 26, SEQ ID NO: 31, SEQ ID NO: 40, SEQ ID NO: 43, SEQ ID NO: 49, SEQ ID NO: 56, or SEQ ID NO: 59.

2. The antibody according to claim 1, wherein the heavy chain variable region comprises:
HCDR1 having a sequence set forth in SEQ ID NO: 1, HCDR2 having a sequence set forth in SEQ ID NO: 8, and HCDR3 having a sequence set forth in SEQ ID NO: 3;
HCDR1 having a sequence set forth in SEQ ID NO: 12, HCDR2 having a sequence set forth in SEQ ID NO: 13, and HCDR3 having a sequence set forth in SEQ ID NO: 14;
HCDR1 having a sequence set forth in SEQ ID NO: 1, HCDR2 having a sequence set forth in SEQ ID NO: 2, and HCDR3 having a sequence set forth in SEQ ID NO: 3;
HCDR1 having a sequence set forth in SEQ ID NO: 1, HCDR2 having a sequence set forth in SEQ ID NO: 7, and HCDR3 having a sequence set forth in SEQ ID NO: 3;
HCDR1 having a sequence set forth in SEQ ID NO: 1, HCDR2 having a sequence set forth in SEQ ID NO: 9, and HCDR3 having a sequence set forth in SEQ ID NO: 3;
HCDR1 having a sequence set forth in SEQ ID NO: 1, HCDR2 having a sequence set forth in SEQ ID NO: 10, and HCDR3 having a sequence set forth in SEQ ID NO: 3;
HCDR1 having a sequence set forth in SEQ ID NO: 1, HCDR2 having a sequence set forth in SEQ ID NO: 11, and HCDR3 having a sequence set forth in SEQ ID NO: 3;
HCDR1 having a sequence set forth in SEQ ID NO: 18, HCDR2 having a sequence set forth in SEQ ID NO: 19, and HCDR3 having a sequence set forth in SEQ ID NO: 20;
HCDR1 having a sequence set forth in SEQ ID NO: 23, HCDR2 having a sequence set forth in SEQ ID NO: 24, and HCDR3 having a sequence set forth in SEQ ID NO: 25;
HCDR1 having a sequence set forth in SEQ ID NO: 23, HCDR2 having a sequence set forth in SEQ ID NO: 27, and HCDR3 having a sequence set forth in SEQ ID NO: 28;
HCDR1 having a sequence set forth in SEQ ID NO: 32, HCDR2 having a sequence set forth in SEQ ID NO: 33, and HCDR3 having a sequence set forth in SEQ ID NO: 34;
HCDR1 having a sequence set forth in SEQ ID NO: 32, HCDR2 having a sequence set forth in SEQ ID NO: 35, and HCDR3 having a sequence set forth in SEQ ID NO: 34;
HCDR1 having a sequence set forth in SEQ ID NO: 36, HCDR2 having a sequence set forth in SEQ ID NO: 37, and HCDR3 having a sequence set forth in SEQ ID NO: 38;
HCDR1 having a sequence set forth in SEQ ID NO: 23, HCDR2 having a sequence set forth in SEQ ID NO: 41, and HCDR3 having a sequence set forth in SEQ ID NO: 42;
HCDR1 having a sequence set forth in SEQ ID NO: 44, HCDR2 having a sequence set forth in SEQ ID NO: 45, and HCDR3 having a sequence set forth in SEQ ID NO: 46;
HCDR1 having a sequence set forth in SEQ ID NO: 23, HCDR2 having a sequence set forth in SEQ ID NO: 50, and HCDR3 having a sequence set forth in SEQ ID NO: 51;
HCDR1 having a sequence set forth in SEQ ID NO: 52, HCDR2 having a sequence set forth in SEQ ID NO: 53, and HCDR3 having a sequence set forth in SEQ ID NO: 54; or
HCDR1 having a sequence set forth in SEQ ID NO: 32, HCDR2 having a sequence set forth in SEQ ID NO: 57, and HCDR3 having a sequence set forth in SEQ ID NO: 58;
and/or
the light chain variable region comprises: LCDR1 having a sequence set forth in SEQ ID NO: 4, LCDR2 having a sequence set forth in SEQ ID NO: 5, and LCDR3 having a sequence set forth in SEQ ID NO: 6;
LCDR1 having a sequence set forth in SEQ ID NO: 15, LCDR2 having a sequence set forth in SEQ ID NO: 16, and LCDR3 having a sequence set forth in SEQ ID NO: 17;
LCDR1 having a sequence set forth in SEQ ID NO: 21, LCDR2 having a sequence set forth in SEQ ID NO: 5, and LCDR3 having a sequence set forth in SEQ ID NO: 22;
LCDR1 having a sequence set forth in SEQ ID NO: 15, LCDR2 having a sequence set forth in SEQ ID NO: 16, and LCDR3 having a sequence set forth in SEQ ID NO: 26;
LCDR1 having a sequence set forth in SEQ ID NO: 29, LCDR2 having a sequence set forth in SEQ ID NO: 30, and LCDR3 having a sequence set forth in SEQ ID NO: 31;
LCDR1 having a sequence set forth in SEQ ID NO: 39, LCDR2 having a sequence set forth in SEQ ID NO: 30, and LCDR3 having a sequence set forth in SEQ ID NO: 31;
LCDR1 having a sequence set forth in SEQ ID NO: 15, LCDR2 having a sequence set forth in SEQ ID NO: 16, and LCDR3 having a sequence set forth in SEQ ID NO: 40;
LCDR1 having a sequence set forth in SEQ ID NO: 15, LCDR2 having a sequence set forth in SEQ ID NO: 16, and LCDR3 having a sequence set forth in SEQ ID NO: 43;
LCDR1 having a sequence set forth in SEQ ID NO: 47, LCDR2 having a sequence set forth in SEQ ID NO: 48, and LCDR3 having a sequence set forth in SEQ ID NO: 49;
LCDR1 having a sequence set forth in SEQ ID NO: 15, LCDR2 having a sequence set forth in SEQ ID NO: 55, and LCDR3 having a sequence set forth in SEQ ID NO: 56; or
LCDR1 having a sequence set forth in SEQ ID NO: 15, LCDR2 having a sequence set forth in SEQ ID NO: 16, and LCDR3 having a sequence set forth in SEQ ID NO: 59;
preferably, the light chain variable region and the heavy chain variable region are selected from any one of the following:
(1) HCDR1 having a sequence set forth in SEQ ID NO: 1, HCDR2 having a sequence set forth in SEQ ID NO: 8, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(2) HCDR1 having a sequence set forth in SEQ ID NO: 12, HCDR2 having a sequence set forth in SEQ ID NO: 13, and HCDR3 having a sequence set forth in SEQ ID NO: 14; and LCDR1 having a sequence set forth in SEQ ID NO: 15, LCDR2 having a sequence set forth in SEQ ID NO: 16, and LCDR3 having a sequence set forth in SEQ ID NO: 17;
(3) HCDR1 having a sequence set forth in SEQ ID NO: 1, HCDR2 having a sequence set forth in SEQ ID NO: 2, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(4) HCDR1 having a sequence set forth in SEQ ID NO: 1, HCDR2 having a sequence set forth in SEQ ID NO: 7, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(5) HCDR1 having a sequence set forth in SEQ ID NO: 1, HCDR2 having a sequence set forth in SEQ ID NO: 9, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(6) HCDR1 having a sequence set forth in SEQ ID NO: 1, HCDR2 having a sequence set forth in SEQ ID NO: 10, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(7) HCDR1 having a sequence set forth in SEQ ID NO: 1, HCDR2 having a sequence set forth in SEQ ID NO: 11, and HCDR3 having a sequence set forth in SEQ ID NO: 3; and LCDR1 having a sequence set forth in SEQ ID NO: 4, LCDR2 having a sequence set forth in SEQ ID NO: 5, and LCDR3 having a sequence set forth in SEQ ID NO: 6;
(8) HCDR1 having a sequence set forth in SEQ ID NO: 18, HCDR2 having a sequence set forth in SEQ ID NO: 19, and HCDR3 having a sequence set forth in SEQ ID NO: 20; and LCDR1 having a sequence set forth in SEQ ID NO: 21, LCDR2 having a sequence set forth in SEQ ID NO: 5, and LCDR3 having a sequence set forth in SEQ ID NO: 22;
(9) HCDR1 having a sequence set forth in SEQ ID NO: 23, HCDR2 having a sequence set forth in SEQ ID NO: 24, and HCDR3 having a sequence set forth in SEQ ID NO: 25; and LCDR1 having a sequence set forth in SEQ ID NO: 15, LCDR2 having a sequence set forth in SEQ ID NO: 16, and LCDR3 having a sequence set forth in SEQ ID NO: 26;
(10) HCDR1 having a sequence set forth in SEQ ID NO: 23, HCDR2 having a sequence set forth in SEQ ID NO: 27, and HCDR3 having a sequence set forth in SEQ ID NO: 28; and LCDR1 having a sequence set forth in SEQ ID NO: 29, LCDR2 having a sequence set forth in SEQ ID NO: 30, and LCDR3 having a sequence set forth in SEQ ID NO: 31;
(11) HCDR1 having a sequence set forth in SEQ ID NO: 23, HCDR2 having a sequence set forth in SEQ ID NO: 27, and HCDR3 having a sequence set forth in SEQ ID NO: 28; and LCDR1 having a sequence set forth in SEQ ID NO: 39, LCDR2 having a sequence set forth in SEQ ID NO: 30, and LCDR3 having a sequence set forth in SEQ ID NO: 31;
(12) HCDR1 having a sequence set forth in SEQ ID NO: 32, HCDR2 having a sequence set forth in SEQ ID NO: 33, and HCDR3 having a sequence set forth in SEQ ID NO: 34; and LCDR1 having a sequence set forth in SEQ ID NO: 15, LCDR2 having a sequence set forth in SEQ ID NO: 16, and LCDR3 having a sequence set forth in SEQ ID NO: 17;
(13) HCDR1 having a sequence set forth in SEQ ID NO: 32, HCDR2 having a sequence set forth in SEQ ID NO: 35, and HCDR3 having a sequence set forth in SEQ ID NO: 34; and LCDR1 having a sequence set forth in SEQ ID NO: 15, LCDR2 having a sequence set forth in SEQ ID NO: 16, and LCDR3 having a sequence set forth in SEQ ID NO: 17;
(14) HCDR1 having a sequence set forth in SEQ ID NO: 36, HCDR2 having a sequence set forth in SEQ ID NO: 37, and HCDR3 having a sequence set forth in SEQ ID NO: 38; and LCDR1 having a sequence set forth in SEQ ID NO: 15, LCDR2 having a sequence set forth in SEQ ID NO: 16, and LCDR3 having a sequence set forth in SEQ ID NO: 40;
(15) HCDR1 having a sequence set forth in SEQ ID NO: 23, HCDR2 having a sequence set forth in SEQ ID NO: 41, and HCDR3 having a sequence set forth in SEQ ID NO: 42; and LCDR1 having a sequence set forth in SEQ ID NO: 15, LCDR2 having a sequence set forth in SEQ ID NO: 16, and LCDR3 having a sequence set forth in SEQ ID NO: 43;
(16) HCDR1 having a sequence set forth in SEQ ID NO: 44, HCDR2 having a sequence set forth in SEQ ID NO: 45, and HCDR3 having a sequence set forth in SEQ ID NO: 46; and LCDR1 having a sequence set forth in SEQ ID NO: 47, LCDR2 having a sequence set forth in SEQ ID NO: 48, and LCDR3 having a sequence set forth in SEQ ID NO: 49;
(17) HCDR1 having a sequence set forth in SEQ ID NO: 23, HCDR2 having a sequence set forth in SEQ ID NO: 50, and HCDR3 having a sequence set forth in SEQ ID NO: 51; and LCDR1 having a sequence set forth in SEQ ID NO: 15, LCDR2 having a sequence set forth in SEQ ID NO: 16, and LCDR3 having a sequence set forth in SEQ ID NO: 17;
(18) HCDR1 having a sequence set forth in SEQ ID NO: 52, HCDR2 having a sequence set forth in SEQ ID NO: 53, and HCDR3 having a sequence set forth in SEQ ID NO: 54; and LCDR1 having a sequence set forth in SEQ ID NO: 15, LCDR2 having a sequence set forth in SEQ ID NO: 55, and LCDR3 having a sequence set forth in SEQ ID NO: 56;
(19) HCDR1 having a sequence set forth in SEQ ID NO: 32, HCDR2 having a sequence set forth in SEQ ID NO: 57, and HCDR3 having a sequence set forth in SEQ ID NO: 58; and LCDR1 having a sequence set forth in SEQ ID NO: 15, LCDR2 having a sequence set forth in SEQ ID NO: 16, and LCDR3 having a sequence set forth in SEQ ID NO: 59.

3. The antibody according to claim 1 or 2, wherein a framework region of the light chain variable region is a human framework region, and/or a framework region of the heavy chain variable region is a human framework region;
preferably, the heavy chain variable region comprises an amino acid sequence set forth in SEQ ID NO: 64 or an amino acid sequence having at least 85%, 90%, 91%, 92%, 93%, 94%, 95%, 96%, 97%, 98%, or 99% identity to SEQ ID NO: 64, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62;
the heavy chain variable region comprises an amino acid sequence set forth in SEQ ID NO: 68 or an amino acid sequence having at least 85%, 90%, 91%, 92%, 93%, 94%, 95%, 96%, 97%, 98%, or 99% identity to SEQ ID NO: 68, and the light chain variable region comprises an amino acid sequence set forth in SEQ ID NO: 69 or an amino acid sequence having at least 85%, 90%, 91%, 92%, 93%, 94%, 95%, 96%, 97%, 98%, or 99% identity to SEQ ID NO: 69; the heavy chain variable region comprises an amino acid sequence set forth in SEQ ID NO: 61 or an amino acid sequence having at least 85%, 90%, 91%, 92%, 93%, 94%, 95%, 96%, 97%, 98%, or 99% identity to SEQ ID NO: 61, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62; the heavy chain variable region comprises an amino acid sequence set forth in SEQ ID NO: 63 or an amino acid sequence having at least 85%, 90%, 91%, 92%, 93%, 94%, 95%, 96%, 97%, 98%, or 99% identity to SEQ ID NO: 63, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62; the heavy chain variable region comprises an amino acid sequence set forth in SEQ ID NO: 65 or an amino acid sequence having at least 85%, 90%, 91%, 92%, 93%, 94%, 95%, 96%, 97%, 98%, or 99% identity to SEQ ID NO: 65, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62; the heavy chain variable region comprises an amino acid sequence set forth in SEQ ID NO: 66 or an amino acid sequence having at least 85%, 90%, 91%, 92%, 93%, 94%, 95%, 96%, 97%, 98%, or 99% identity to SEQ ID NO: 66, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62; the heavy chain variable region comprises an amino acid sequence set forth in SEQ ID NO: 67 or an amino acid sequence having at least 85%, 90%, 91%, 92%, 93%, 94%, 95%, 96%, 97%, 98%, or 99% identity to SEQ ID NO: 67, and the light chain variable region comprises an amino acid sequence set forth in SEQ ID NO: 62 or an amino acid sequence having at least 85%, 90%, 91%, 92%, 93%, 94%, 95%, 96%, 97%, 98%, or 99% identity to SEQ ID NO: 62; the heavy chain variable region comprises an amino acid sequence set forth in SEQ ID NO: 68 or an amino acid sequence having at least 85%, 90%, 91%, 92%, 93%, 94%, 95%, 96%, 97%, 98%, or 99% identity to SEQ ID NO: 68, and the light chain variable region comprises an amino acid sequence set forth in SEQ ID NO: 69 or an amino acid sequence having at least 85%, 90%, 91%, 92%, 93%, 94%, 95%, 96%, 97%, 98%, or 99% identity to SEQ ID NO: 69; the heavy chain variable region comprises an amino acid sequence set forth in SEQ ID NO: 70 or an amino acid sequence having at least 85%, 90%, 91%, 92%, 93%, 94%, 95%, 96%, 97%, 98%, or 99% identity to SEQ ID NO: 70, and the light chain variable region comprises an amino acid sequence set forth in SEQ ID NO: 71 or an amino acid sequence having at least 85%, 90%, 91%, 92%, 93%, 94%, 95%, 96%, 97%, 98%, or 99% identity to SEQ ID NO: 71; the heavy chain variable region comprises an amino acid sequence set forth in SEQ ID NO: 72 or an amino acid sequence having at least 85%, 90%, 91%, 92%, 93%, 94%, 95%, 96%, 97%, 98%, or 99% identity to SEQ ID NO: 72, and the light chain variable region comprises an amino acid sequence set forth in SEQ ID NO: 73 or an amino acid sequence having at least 85%, 90%, 91%, 92%, 93%, 94%, 95%, 96%, 97%, 98%, or 99% identity to SEQ ID NO: 73; the heavy chain variable region comprises an amino acid sequence set forth in SEQ ID NO: 74 or an amino acid sequence having at least 85%, 90%, 91%, 92%, 93%, 94%, 95%, 96%, 97%, 98%, or 99% identity to SEQ ID NO: 74, and the light chain variable region comprises an amino acid sequence set forth in SEQ ID NO: 75 or an amino acid sequence having at least 85%, 90%, 91%, 92%, 93%, 94%, 95%, 96%, 97%, 98%, or 99% identity to SEQ ID NO: 75; the heavy chain variable region comprises an amino acid sequence set forth in SEQ ID NO: 74 or an amino acid sequence having at least 85%, 90%, 91%, 92%, 93%, 94%, 95%, 96%, 97%, 98%, or 99% identity to SEQ ID NO: 74, and the light chain variable region comprises an amino acid sequence set forth in SEQ ID NO: 76 or an amino acid sequence having at least 85%, 90%, 91%, 92%, 93%, 94%, 95%, 96%, 97%, 98%, or 99% identity to SEQ ID NO: 76; the heavy chain variable region comprises an amino acid sequence set forth in SEQ ID NO: 77 or an amino acid sequence having at least 85%, 90%, 91%, 92%, 93%, 94%, 95%, 96%, 97%, 98%, or 99% identity to SEQ ID NO: 77, and the light chain variable region comprises an amino acid sequence set forth in SEQ ID NO: 78 or an amino acid sequence having at least 85%, 90%, 91%, 92%, 93%, 94%, 95%, 96%, 97%, 98%, or 99% identity to SEQ ID NO: 78; the heavy chain variable region comprises an amino acid sequence set forth in SEQ ID NO: 79 or an amino acid sequence having at least 85%, 90%, 91%, 92%, 93%, 94%, 95%, 96%, 97%, 98%, or 99% identity to SEQ ID NO: 79, and the light chain variable region comprises an amino acid sequence set forth in SEQ ID NO: 78 or an amino acid sequence having at least 85%, 90%, 91%, 92%, 93%, 94%, 95%, 96%, 97%, 98%, or 99% identity to SEQ ID NO: 78; the heavy chain variable region comprises an amino acid sequence set forth in SEQ ID NO: 80 or an amino acid sequence having at least 85%, 90%, 91%, 92%, 93%, 94%, 95%, 96%, 97%, 98%, or 99% identity to SEQ ID NO: 80, and the light chain variable region comprises an amino acid sequence set forth in SEQ ID NO: 81 or an amino acid sequence having at least 85%, 90%, 91%, 92%, 93%, 94%, 95%, 96%, 97%, 98%, or 99% identity to SEQ ID NO: 81; the heavy chain variable region comprises an amino acid sequence set forth in SEQ ID NO: 82 or an amino acid sequence having at least 85%, 90%, 91%, 92%, 93%, 94%, 95%, 96%, 97%, 98%, or 99% identity to SEQ ID NO: 82, and the light chain variable region comprises an amino acid sequence set forth in SEQ ID NO: 83 or an amino acid sequence having at least 85%, 90%, 91%, 92%, 93%, 94%, 95%, 96%, 97%, 98%, or 99% identity to SEQ ID NO: 83; the heavy chain variable region comprises an amino acid sequence set forth in SEQ ID NO: 84 or an amino acid sequence having at least 85%, 90%, 91%, 92%, 93%, 94%, 95%, 96%, 97%, 98%, or 99% identity to SEQ ID NO: 84, and the light chain variable region comprises an amino acid sequence set forth in SEQ ID NO: 85 or an amino acid sequence having at least 85%, 90%, 91%, 92%, 93%, 94%, 95%, 96%, 97%, 98%, or 99% identity to SEQ ID NO: 85; the heavy chain variable region comprises an amino acid sequence set forth in SEQ ID NO: 86 or an amino acid sequence having at least 85%, 90%, 91%, 92%, 93%, 94%, 95%, 96%, 97%, 98%, or 99% identity to SEQ ID NO: 86, and the light chain variable region comprises an amino acid sequence set forth in SEQ ID NO: 78 or an amino acid sequence having at least 85%, 90%, 91%, 92%, 93%, 94%, 95%, 96%, 97%, 98%, or 99% identity to SEQ ID NO: 78; the heavy chain variable region comprises an amino acid sequence set forth in SEQ ID NO: 87 or an amino acid sequence having at least 85%, 90%, 91%, 92%, 93%, 94%, 95%, 96%, 97%, 98%, or 99% identity to SEQ ID NO: 87, and the light chain variable region comprises an amino acid sequence set forth in SEQ ID NO: 88 or an amino acid sequence having at least 85%, 90%, 91%, 92%, 93%, 94%, 95%, 96%, 97%, 98%, or 99% identity to SEQ ID NO: 88; or
the heavy chain variable region comprises an amino acid sequence set forth in SEQ ID NO: 60 or an amino acid sequence having at least 85%, 90%, 91%, 92%, 93%, 94%, 95%, 96%, 97%, 98%, or 99% identity to SEQ ID NO: 60, and the light chain variable region comprises an amino acid sequence set forth in SEQ ID NO: 89 or an amino acid sequence having at least 85%, 90%, 91%, 92%, 93%, 94%, 95%, 96%, 97%, 98%, or 99% identity to SEQ ID NO: 89.

4. The antibody according to any one of claims 1-3, wherein the antibody satisfies one or both of:
(1) the antibody is a full-length antibody, a Fab, a Fab', a F(ab')₂, an Fv, an scFv, a di-scFv, a VHH, or a heavy-chain antibody (HCAb);
(2) the antibody is a monoclonal antibody or a polyclonal antibody prepared based on the antibody described above.

5. The antibody according to claim 4, wherein the antibody comprises a heavy chain constant region and/or a light chain constant region;
the heavy chain constant region of the antibody is derived from a heavy chain constant region of a human antibody IgG1 or IgG4, and/or the light chain constant region of the antibody is derived from a κ chain of a human antibody;
more preferably, a heavy chain of the antibody comprises an amino acid sequence set forth in SEQ ID NO: 93 or an amino acid sequence having at least 85%, 90%, 91%, 92%, 93%, 94%, 95%, 96%, 97%, 98%, or 99% identity to SEQ ID NO: 93, and a light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91;
a heavy chain of the antibody comprises an amino acid sequence set forth in SEQ ID NO: 99 or an amino acid sequence having at least 85%, 90%, 91%, 92%, 93%, 94%, 95%, 96%, 97%, 98%, or 99% identity to SEQ ID NO: 99, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 90 or an amino acid sequence having at least 85%, 90%, 91%, 92%, 93%, 94%, 95%, 96%, 97%, 98%, or 99% identity to SEQ ID NO: 90, and a light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91;
a heavy chain of the antibody comprises an amino acid sequence set forth in SEQ ID NO: 92 or an amino acid sequence having at least 85%, 90%, 91%, 92%, 93%, 94%, 95%, 96%, 97%, 98%, or 99% identity to SEQ ID NO: 92, and a light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91;
a heavy chain of the antibody comprises an amino acid sequence set forth in SEQ ID NO: 94 or an amino acid sequence having at least 85%, 90%, 91%, 92%, 93%, 94%, 95%, 96%, 97%, 98%, or 99% identity to SEQ ID NO: 94, and a light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91;
a heavy chain of the antibody comprises an amino acid sequence set forth in SEQ ID NO: 95 or an amino acid sequence having at least 85%, 90%, 91%, 92%, 93%, 94%, 95%, 96%, 97%, 98%, or 99% identity to SEQ ID NO: 95, and a light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91;
a heavy chain of the antibody comprises an amino acid sequence set forth in SEQ ID NO: 96 or an amino acid sequence having at least 85%, 90%, 91%, 92%, 93%, 94%, 95%, 96%, 97%, 98%, or 99% identity to SEQ ID NO: 96, and a light chain of the antibody comprises an amino acid sequence set forth in SEQ ID NO: 91 or an amino acid sequence having at least 85%, 90%, 91%, 92%, 93%, 94%, 95%, 96%, 97%, 98%, or 99% identity to SEQ ID NO: 91;
a heavy chain of the antibody comprises an amino acid sequence set forth in SEQ ID NO: 97 or an amino acid sequence having at least 85%, 90%, 91%, 92%, 93%, 94%, 95%, 96%, 97%, 98%, or 99% identity to SEQ ID NO: 97, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 100 or an amino acid sequence having at least 85%, 90%, 91%, 92%, 93%, 94%, 95%, 96%, 97%, 98%, or 99% identity to SEQ ID NO: 100, and a light chain of the antibody comprises an amino acid sequence set forth in SEQ ID NO: 101 or an amino acid sequence having at least 85%, 90%, 91%, 92%, 93%, 94%, 95%, 96%, 97%, 98%, or 99% identity to SEQ ID NO: 101;
a heavy chain of the antibody comprises an amino acid sequence set forth in SEQ ID NO: 102 or an amino acid sequence having at least 85%, 90%, 91%, 92%, 93%, 94%, 95%, 96%, 97%, 98%, or 99% identity to SEQ ID NO: 102, and a light chain of the antibody comprises an amino acid sequence set forth in SEQ ID NO: 101 or an amino acid sequence having at least 85%, 90%, 91%, 92%, 93%, 94%, 95%, 96%, 97%, 98%, or 99% identity to SEQ ID NO: 101;
a heavy chain of the antibody comprises an amino acid sequence set forth in SEQ ID NO: 103 or an amino acid sequence having at least 85%, 90%, 91%, 92%, 93%, 94%, 95%, 96%, 97%, 98%, or 99% identity to SEQ ID NO: 103, and a light chain of the antibody comprises an amino acid sequence set forth in SEQ ID NO: 104 or an amino acid sequence having at least 85%, 90%, 91%, 92%, 93%, 94%, 95%, 96%, 97%, 98%, or 99% identity to SEQ ID NO: 104;
a heavy chain of the antibody comprises an amino acid sequence set forth in SEQ ID NO: 105 or an amino acid sequence having at least 85%, 90%, 91%, 92%, 93%, 94%, 95%, 96%, 97%, 98%, or 99% identity to SEQ ID NO: 105, and a light chain of the antibody comprises an amino acid sequence set forth in SEQ ID NO: 104 or an amino acid sequence having at least 85%, 90%, 91%, 92%, 93%, 94%, 95%, 96%, 97%, 98%, or 99% identity to SEQ ID NO: 104;
a heavy chain of the antibody comprises an amino acid sequence set forth in SEQ ID NO: 106 or an amino acid sequence having at least 85%, 90%, 91%, 92%, 93%, 94%, 95%, 96%, 97%, 98%, or 99% identity to SEQ ID NO: 106, and a light chain of the antibody comprises an amino acid sequence set forth in SEQ ID NO: 107 or an amino acid sequence having at least 85%, 90%, 91%, 92%, 93%, 94%, 95%, 96%, 97%, 98%, or 99% identity to SEQ ID NO: 107;
a heavy chain of the antibody comprises an amino acid sequence set forth in SEQ ID NO: 108 or an amino acid sequence having at least 85%, 90%, 91%, 92%, 93%, 94%, 95%, 96%, 97%, 98%, or 99% identity to SEQ ID NO: 108, and a light chain of the antibody comprises an amino acid sequence set forth in SEQ ID NO: 109 or an amino acid sequence having at least 85%, 90%, 91%, 92%, 93%, 94%, 95%, 96%, 97%, 98%, or 99% identity to SEQ ID NO: 109;
a heavy chain of the antibody comprises an amino acid sequence set forth in SEQ ID NO: 110 or an amino acid sequence having at least 85%, 90%, 91%, 92%, 93%, 94%, 95%, 96%, 97%, 98%, or 99% identity to SEQ ID NO: 110, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 111 or an amino acid sequence having at least 85%, 90%, 91%, 92%, 93%, 94%, 95%, 96%, 97%, 98%, or 99% identity to SEQ ID NO: 111, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 112 or an amino acid sequence having at least 85%, 90%, 91%, 92%, 93%, 94%, 95%, 96%, 97%, 98%, or 99% identity to SEQ ID NO: 112, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 113 or an amino acid sequence having at least 85%, 90%, 91%, 92%, 93%, 94%, 95%, 96%, 97%, 98%, or 99% identity to SEQ ID NO: 113, and a light chain of the antibody comprises an amino acid sequence set forth in SEQ ID NO: 114 or an amino acid sequence having at least 85%, 90%, 91%, 92%, 93%, 94%, 95%, 96%, 97%, 98%, or 99% identity to SEQ ID NO: 114;
a heavy chain of the antibody comprises an amino acid sequence set forth in SEQ ID NO: 115 or an amino acid sequence having at least 85%, 90%, 91%, 92%, 93%, 94%, 95%, 96%, 97%, 98%, or 99% identity to SEQ ID NO: 115, and a light chain of the antibody comprises an amino acid sequence set forth in SEQ ID NO: 116 or an amino acid sequence having at least 85%, 90%, 91%, 92%, 93%, 94%, 95%, 96%, 97%, 98%, or 99% identity to SEQ ID NO: 116;
a heavy chain of the antibody comprises an amino acid sequence set forth in SEQ ID NO: 117 or an amino acid sequence having at least 85%, 90%, 91%, 92%, 93%, 94%, 95%, 96%, 97%, 98%, or 99% identity to SEQ ID NO: 117, and a light chain of the antibody comprises an amino acid sequence set forth in SEQ ID NO: 118 or an amino acid sequence having at least 85%, 90%, 91%, 92%, 93%, 94%, 95%, 96%, 97%, 98%, or 99% identity to SEQ ID NO: 118;
a heavy chain of the antibody comprises an amino acid sequence set forth in SEQ ID NO: 119 or an amino acid sequence having at least 85%, 90%, 91%, 92%, 93%, 94%, 95%, 96%, 97%, 98%, or 99% identity to SEQ ID NO: 119, and a light chain of the antibody comprises an amino acid sequence set forth in SEQ ID NO: 118 or an amino acid sequence having at least 85%, 90%, 91%, 92%, 93%, 94%, 95%, 96%, 97%, 98%, or 99% identity to SEQ ID NO: 118;
a heavy chain of the antibody comprises an amino acid sequence set forth in SEQ ID NO: 120 or an amino acid sequence having at least 85%, 90%, 91%, 92%, 93%, 94%, 95%, 96%, 97%, 98%, or 99% identity to SEQ ID NO: 120, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 121 or an amino acid sequence having at least 85%, 90%, 91%, 92%, 93%, 94%, 95%, 96%, 97%, 98%, or 99% identity to SEQ ID NO: 121, and a light chain of the antibody comprises an amino acid sequence set forth in SEQ ID NO: 98 or an amino acid sequence having at least 85%, 90%, 91%, 92%, 93%, 94%, 95%, 96%, 97%, 98%, or 99% identity to SEQ ID NO: 98;
a heavy chain of the antibody comprises an amino acid sequence set forth in SEQ ID NO: 122 or an amino acid sequence having at least 85%, 90%, 91%, 92%, 93%, 94%, 95%, 96%, 97%, 98%, or 99% identity to SEQ ID NO: 122, and a light chain of the antibody comprises an amino acid sequence set forth in SEQ ID NO: 123 or an amino acid sequence having at least 85%, 90%, 91%, 92%, 93%, 94%, 95%, 96%, 97%, 98%, or 99% identity to SEQ ID NO: 123;
a heavy chain of the antibody comprises an amino acid sequence set forth in SEQ ID NO: 124 or an amino acid sequence having at least 85%, 90%, 91%, 92%, 93%, 94%, 95%, 96%, 97%, 98%, or 99% identity to SEQ ID NO: 124, and a light chain of the antibody comprises an amino acid sequence set forth in SEQ ID NO: 125 or an amino acid sequence having at least 85%, 90%, 91%, 92%, 93%, 94%, 95%, 96%, 97%, 98%, or 99% identity to SEQ ID NO: 125; or
a heavy chain of the antibody comprises an amino acid sequence set forth in SEQ ID NO: 126 or an amino acid sequence having at least 85%, 90%, 91%, 92%, 93%, 94%, 95%, 96%, 97%, 98%, or 99% identity to SEQ ID NO: 126, and a light chain of the antibody comprises an amino acid sequence set forth in SEQ ID NO: 125 or an amino acid sequence having at least 85%, 90%, 91%, 92%, 93%, 94%, 95%, 96%, 97%, 98%, or 99% identity to SEQ ID NO: 125.

6. An isolated nucleic acid encoding the antibody according to any one of claims 1-5.

7. A recombinant expression vector, comprising the isolated nucleic acid according to claim 6, wherein preferably, a backbone of the recombinant expression vector is a plasmid, a cosmid, a phage, or a viral vector, wherein the viral vector is preferably a retroviral vector, a lentiviral vector, an adenoviral vector, or an adeno-associated viral vector.

8. A transformant, comprising the recombinant expression vector according to claim 7, wherein preferably, a host cell of the transformant is a prokaryotic cell or a eukaryotic cell, and more preferably, the eukaryotic cell is a yeast cell or a mammalian cell, wherein the mammalian cell is, e.g., an HEK293 cell or a CHO cell.

9. A method for preparing an antibody, comprising culturing the transformant according to claim 8 and obtaining the antibody from a culture.

10. An antigen-binding protein derivative, comprising the antibody according to any one of claims 1-5 and a detectable labeling molecule, wherein preferably, the detectable labeling molecule is an enzyme, a radionuclide, a fluorescent dye, a luminescent substance, or biotin.

11. An antibody-drug conjugate, comprising a cytotoxic agent or a tag, and the antibody according to any one of claims 1-5.

12. A chimeric antigen receptor, comprising the antibody according to any one of claims 1-5.

13. A genetically modified cell, comprising the chimeric antigen receptor according to claim 12, wherein the genetically modified cell is preferably a eukaryotic cell, more preferably an isolated human cell, and even more preferably an immune cell such as a T cell or an NK cell.

14. A multispecific antibody, comprising the antibody according to any one of claims 1-5, wherein preferably, the multispecific antibody is a bispecific antibody.

15. A pharmaceutical composition, comprising the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, or the multispecific antibody according to claim 14, and a pharmaceutically acceptable carrier.

16. A kit, comprising the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, or the pharmaceutical composition according to claim 15, wherein
preferably, the kit further comprises (i) a device for administering the antibody, the antigen-binding protein derivative, the antibody-drug conjugate, the chimeric antigen receptor, the genetically modified cell, or the pharmaceutical composition, and/or (ii) instructions.

17. A kit-of-parts, comprising a kit A and a kit B, wherein
the kit A comprises the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, or the pharmaceutical composition according to claim 15;
the kit B comprises other anti-tumor antibodies or a pharmaceutical composition comprising the other anti-tumor antibodies, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

18. Use of the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, the pharmaceutical composition according to claim 15, the kit according to claim 16, or the kit-of-parts according to claim 17 in the manufacture of a product for the diagnosis, treatment, and/or prevention of cancer, wherein preferably, the cancer is ILT4-positive and/or ILT2-positive cancer; more preferably, the cancer is solid cancer or hematological cancer;
the hematological cancer is, e.g., B-cell leukemia, lymphoma, acute myeloid leukemia, Burkitt lymphoma, mantle cell lymphoma, acute lymphoblastic leukemia, chronic lymphocytic leukemia, diffuse large B-cell lymphoma, follicular lymphoma, or marginal zone lymphoma;
the solid cancer is, e.g., squamous cell carcinoma of the head and neck, renal cell carcinoma, ovarian cancer, urothelial cancer, diffuse large B-cell carcinoma, non-small cell lung cancer, melanoma, metastatic renal cell carcinoma, metastatic colorectal cancer, small cell lung cancer, metastatic non-small cell lung cancer, breast cancer, lung cancer, head and neck cancer, colorectal cancer, prostate cancer, skin cancer, stomach cancer, intestinal cancer, cervical cancer, uterine cancer, endometrial cancer, bladder cancer, brain cancer, esophageal cancer, liver cancer, renal cancer, testicular cancer, mesothelioma, glioblastoma, cholangiocarcinoma, or pancreatic cancer.

19. A method for diagnosing, treating, and/or preventing an ILT4-mediated disease or disorder, comprising administering to a patient in need thereof a therapeutically effective amount of the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, the pharmaceutical composition according to claim 15, the kit according to claim 16, or the kit-of-parts according to claim 17.

20. The method according to claim 19, wherein the disease or disorder is cancer; preferably, the cancer is ILT4-positive and/or ILT2-positive cancer; more preferably, the cancer is solid cancer or hematological cancer;
the hematological cancer is, e.g., B-cell leukemia, lymphoma, acute myeloid leukemia, Burkitt lymphoma, mantle cell lymphoma, acute lymphoblastic leukemia, chronic lymphocytic leukemia, diffuse large B-cell lymphoma, follicular lymphoma, or marginal zone lymphoma;
the solid cancer is, e.g., squamous cell carcinoma of the head and neck, renal cell carcinoma, ovarian cancer, urothelial cancer, diffuse large B-cell carcinoma, non-small cell lung cancer, melanoma, metastatic renal cell carcinoma, metastatic colorectal cancer, small cell lung cancer, metastatic non-small cell lung cancer, breast cancer, lung cancer, head and neck cancer, colorectal cancer, prostate cancer, skin cancer, stomach cancer, intestinal cancer, cervical cancer, uterine cancer, endometrial cancer, bladder cancer, brain cancer, esophageal cancer, liver cancer, renal cancer, testicular cancer, mesothelioma, glioblastoma, cholangiocarcinoma, or pancreatic cancer.

21. A method for immunoassaying or determining ILT4, comprising using the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, the pharmaceutical composition according to claim 15, the kit according to claim 16, or the kit-of-parts according to claim 17, wherein preferably, the assaying is for non-diagnostic purposes.

22. A combination therapy, comprising administering to a patient in need thereof the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, the pharmaceutical composition according to claim 15, the kit according to claim 16, or the kit-of-parts according to claim 17, and
a second therapeutic agent, wherein the second therapeutic agent preferably comprises other anti-tumor antibodies or a pharmaceutical composition comprising the other anti-tumor antibodies, and/or one or more of the group consisting of a hormonal agent, a small molecule-targeted agent, a proteasome inhibitor, an imaging agent, a diagnostic agent, a chemotherapeutic agent, an oncolytic drug, a cytotoxic agent, a cytokine, an activator of a co-stimulatory molecule, an inhibitor of an inhibitory molecule, and a vaccine.

23. An administration device, comprising the antibody according to any one of claims 1-5, the nucleic acid according to claim 6, the recombinant vector according to claim 7, the antigen-binding protein derivative according to claim 10, the antibody-drug conjugate according to claim 11, the chimeric antigen receptor according to claim 12, the genetically modified cell according to claim 13, the multispecific antibody according to claim 14, the pharmaceutical composition according to claim 15, the kit according to claim 16, or the kit-of-parts according to claim 17, wherein
preferably, the administration device further comprises a component, such as a syringe, an implantable administration device, or an infusion device, for containing or administering to a subject the antibody, the multispecific antibody, the antigen-binding protein derivative, the antibody-drug conjugate, the chimeric antigen receptor, the genetically modified cell, the pharmaceutical composition, the kit, or the kit-of-parts.
